(19) Europäisches Patentamt
European Patent Office
Office européen des brevets

(11) Numéro de publication : **0 286 516 B1**

(12) # FASCICULE DE BREVET EUROPEEN

(45) Date de publication du fascicule du brevet : **18.03.92 Bulletin 92/12**

(51) Int. Cl.$^5$ : **C07D 307/92,** C07D 409/12, C07D 405/04, A61K 31/34

(21) Numéro de dépôt : **88400780.8**

(22) Date de dépôt : **31.03.88**

(54) **Nouveaux dérivés de l'amino tétrahydro-5,6,7,8 naphto [2,3b] furanne, leurs procédés de préparation et les compositions pharmaceutiques qui les contiennent.**

Le dossier contient des informations techniques présentées postérieurement au dépôt de la demande et ne figurant pas dans le présent fascicule.

(30) Priorité : **01.04.87 FR 8704551**

(43) Date de publication de la demande : **12.10.88 Bulletin 88/41**

(45) Mention de la délivrance du brevet : **18.03.92 Bulletin 92/12**

(84) Etats contractants désignés : **AT BE CH DE FR GB IT LI LU NL SE**

(56) Documents cités : **GB-A- 1 084 439** **US-A- 4 470 990**

(56) Documents cités : **CHIMIE THERAPEUTIQUE, vol. 6, no. 3, mai-juin 1971, pages 196-202, Paris, FR; R. VIOLLAND et al.: "VIII. Psychotropes potentiels. Synthèse et activité pharmacologique d'amino-2 tétralines apparentées à divers psychotomimétiques"**

(73) Titulaire : **ADIR ET COMPAGNIE**
**1 rue Carle Hébert**
**F-92415 Courbevoie Cédex (FR)**

(72) Inventeur : **Peglion, Jean-Louis**
**5 allée des Bégonias**
**F-78110 Le Vésinet (FR)**
Inventeur : **Poignant, Jean-Claude**
**La Guyonnerie 13 rue de la Fontaine St-Mathieu**
**F-91440 Bures sur Yvette (FR)**
Inventeur : **Vian, Joel**
**12 avenue Ste-Marie**
**F-92370 Chaville (FR)**

EP 0 286 516 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de l'amino tétrahydro-5,6,7,8 naphto [2,3b] furanne, leurs procédés de préparation et les compositions pharmaceutiques qui les contiennent.

On connait certain composés tricycliques dérivés de l'amino tetrahydro naphtalène pharmacologiquement actifs. En effet, quelques dérivés de la tétrahydro-7,8,9,10 benzo [h] quinolyl-9 amine, dont l'activité antidépréssive n'a été évalué que par des essais in vitro sont mentionnés dans la littérature (Brevet US 4,251,423). Des tétrahydro-6,7,8,9 benzo [g] indolyl-8 amines et des tétrahydro-6,7,8,9 naphto [1,2] furannyl-8 amines douées d'une activité stimulante dopaminergique sont décrites dans les brevets US 4,510,157 et US 4,470,990. La littérature mentionne aussi des dérivés d'amino-2 tétralines ayant des propriétés sédatives et analgésiques. Toutefois, ces derniers composés sont efficaces chez les animaux à des doses proches de celles amenant la létalité (Violland et al, Chimie Thérapeutique, 1971, vo. 6,N°3,p. 196-202). Des dérivés aminés du hexahydro-5a,6,7,8,9,9a dibenzofuranol ayant des propriétés antidépressives sont décrits dans le brevet GB 1,084,439 mais leur activité n'est prouvée par aucun essai pharmacologique.

La demanderesse a maintenant découvert que certains dérivés de l'amino tétrahydro-5,6,7,8 naphto [2,3b] furanne, de structure originale, sont doués de propriétés pharmacologiques très intéressantes. En effet, les composés de la présente invention possèdent une activité antidépres-sive et psychostimulante importante prouvée par des essais in vivo et des propriétés dopaminergiques.

La présente invention a plus particulièrement pour objet les dérivés de formule générale I,

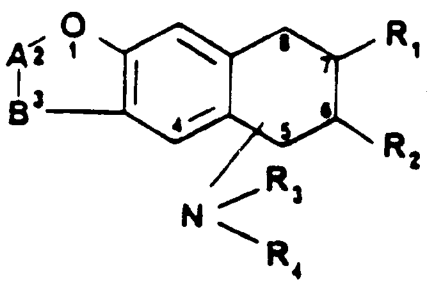

(I)

dans laquelle :

– $R_1$ représente un atome d'hydrogène ou, à condition toutefois que le radical amino soit en position 7, un radical alkyle linéaire ou ramifié renfermant 1 à 4 atomes de carbone,

– $R_2$ représente un atome d'hydrogène ou, à condition toutefois que le radical amino soit en position 6, un radical alkyle linéaire ou ramifié renfermant 1 à 4 atomes de carbone,

– $R_3$ et $R_4$ identiques ou différents représentent chacun un atome d'hydrogène, un radical benzyle, un radical cyclohexylméthyle, un radical alkylène linéaire ou ramifié renfermant de 1 à 5 atomes de carbone, un radical alkyle linéaire ou ramifié renfermant de 1 à 10 atomes de carbone (éventuellement substitué par un radical hydroxy, par un radical carboxy ou par un radical alcoxy de 1 à 5 atomes de carbone, par un radical alcoxy carbonyle de 2 à 6 atomes de carbone, par un radical alkylphényle de 7 à 16 atomes de carbone ou par un radical alkylthiényle-2 de 5 à 14 atomes de carbone) un radical alkyle halogéné contenant de 1 à 5 atomes de carbone, ou forment ensemble avec l'azote auquel ils sont attachés un radical oxo-2 pyrrolidinyl-1,

– A-B représente avec l'oxygène auquel il est attaché un radical

$$-CH_2-CH_2-O-,$$

ou à condition toutefois que le radical amino soit en position 7 et $R_1$ représente un atome d'hydrogène, un radical

$$-CH=CH-O-,$$

un radical

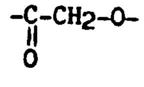

,

ou un radical

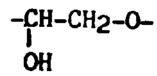 ,

sous forme racémique ou d'isomères optiques,

et leurs sels d'addition à un acide minéral ou organique, pharmaceutiquement acceptable.

La présente invention a également pour objet le procédé de préparation des composés de formule générale I, caractérisé en ce que :

soit

que l'on condense le dihydro-2,3 benzofuranne de formule II,

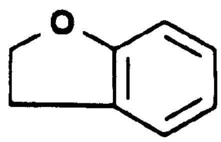

(II)

avec l'anhydride succinique, en présence de chlorure d'aluminium dans un solvant organique chloré et à une température inférieure à 5°C, pour former l'acide (dihydro-2,3 benzofurannyl-5)-4 oxo-4 butanoïque de formule III,

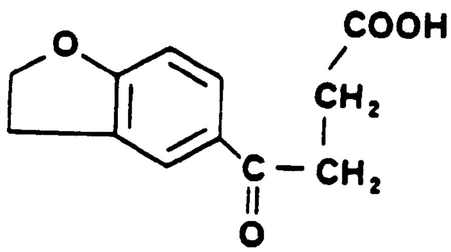

(III)

que l'on réduit en milieu acide à chaud et en présence de zinc et de chlorure mercurique pour former l'acide (dihydro-2,3 benzofurannyl-5)-4 butanoïque de formule IV,

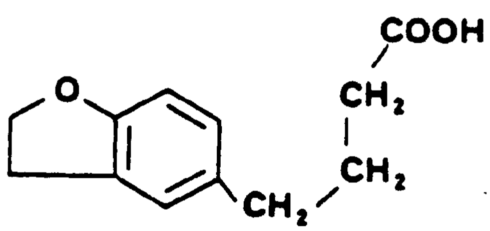

(IV)

lequel ensuite que l'on soumet à l'action de l'acide polyphosphorique dans un solvant organique apolaire et à une température comprise entre 80°C et 100°C pour former la tétrahydro-5,6,7,8 naphto [2,3b] dihydro-2,3 furannone-8 de formule V,

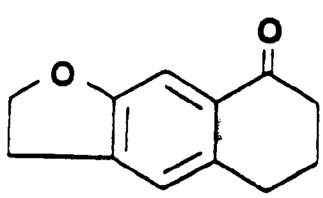

(V)

laquelle ensuite :

– ou

que l'on soumet à l'action d'un sel de l'hydroxylamine, à chaud, dans un alcool de petit poids moléculaire, pour former la tétrahydro-5,6,7,8 naphto [2,3b] dihydro-2,3 furannone-8 oxime de formule VI,

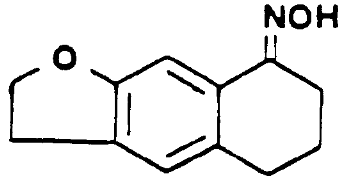

(VI)

laquelle ensuite :

– soit

que l'on condense avec le para-toluènesulfochlorure, dans un milieu organique basique et à une température inférieure à 5°C pour former le para-toluènesulfonate de tétrahydro-5,6,7,8 naphto [2,3b] dihydro-2,3 furannone-8 oxime de formule VII,

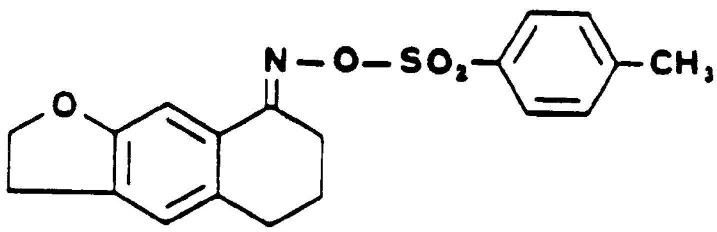

(VII)

que l'on fait agir avec l'éthanolate de sodium dans un solvant organique apolaire anhydre à une température d'environ 0°C pour former l'acétylamino-7 tétrahydro-5,6,7,8 naphto [2,3b] dihydro-2,3 furannone-8 de formule VIII,

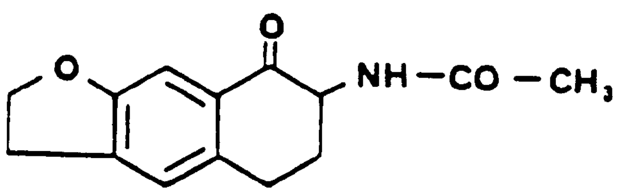

(VIII)

que l'on soumet à une hydrogénation à température ambiante en présence d'un acide et du palladium sur charbon à 5% pour former l'acétylamino-7 tétrahydro-5,6,7,8 naphto [2,3b] dihydro-2,3 furanne de formule IX,

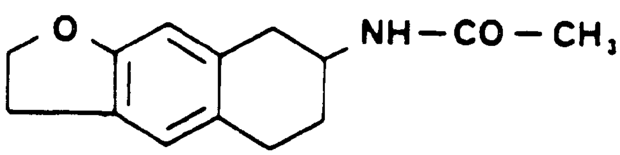

(IX)

lequel ensuite, que l'on transforme par hydrolyse acide à chaud en amino-7 tétrahydro-5,6,7,8 naphto [2,3b] dihydro-2,3 furanne de formule Ia,

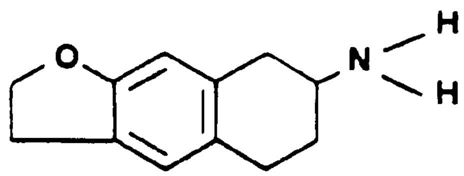

(Ia)

**lequel ensuite** que l'on peut alcoyler pour former les amines secondaires ou tertiaires correspondantes,

. ou en le condensant avec un composé de formule générale X,

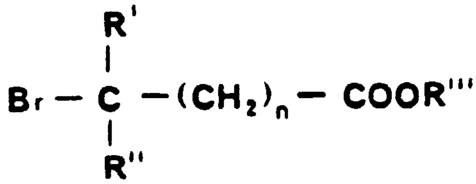

(X)

dans laquelle R′ et R″ identiques ou différents représentent chacun un atome d'hydrogène ou un radical alkyle inférieur de 1 à 4 atomes de carbone, R‴ représente un radical alkyle inférieur de 1 à 5 atomes de carbone, et n est un nombre entier de 0 à 9, pour former un composé de formule générale $Ia_1$,

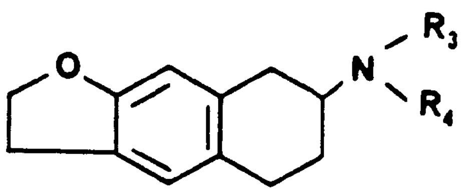

($Ia_1$)

dans laquelle $R_4$ représente un hydrogène et $R_3$ représente un radical alkyle linéaire ou ramifié de 1 à 10 atomes de carbone substitué par un radical alcoxycarbonyle de 2 à 6 atomes de carbone,
lequel ensuite que l'on peut soumettre à l'action d'une base minérale forte pour former un composé de formule générale $Ia_1$,
dans laquelle $R_4$ représente un hydrogène et $R_3$ représente un radical alkyle de 1 à 10 atomes de carbone linéaire ou ramifié substitué par un radical carboxy,
lequel ensuite que l'on peut soumettre à l'action d'un hydrure métallique double pour former un composé de formule générale $Ia_1$,
dans laquelle $R_4$ représente un hydrogène et $R_3$ représente un radical alkyle de 1 à 10 atomes de carbone, linéaire ou ramifié substitué par un radical hydroxy,

. ou en le faisant réagir avec un chlorure d'acide de formule générale XI,

$$W(CH_2)_nCOCl \qquad (XI)$$

dans laquelle n est un nombre entier de 0 à 9 et W représente un radical phényle ou un radical thiényle-2, ou $W(CH_2)_n$ représentent ensemble un radical alkyle halogéné contenant de 1 à 5 atomes de carbone, et ensuite, en réduisant le composé issu de cette réaction par un hydrure métallique double, pour former un composé de formule générale $Ia_1$ dans laquelle $R_3$ représente ou un radical alkyle halogéné contenant de 1 à 5 atomes de carbone ou un radical alkyl de 1 à 10 atomes de carbone substitué par un radical phényle ou un radical thiényle-2, et $R_4$ représente un atome d'hydrogène,

. ou en le faisant réagir avec une quantité adéquate de formaldéhyde et d'acide formique pour obtenir les composés de la formule générale $Ia_1$, dans laquelle $R_3$ et $R_4$ identiques représentent chacun un radical méthyle,

. ou en le faisant réagir avec le chlorure de chloro-4 butyryle, pour former, après une condensation à l'aide d'un hydrure métallique, un composé de formule générale $Ia_1$ dans laquelle $R_3$ et $R_4$ forment ensemble avec l'azote auquel ils sont attachés un radical oxo-2 pyrrolidinyl-1,

. ou en le faisant réagir avec un iodure ou chlorure d'alkyle de formule générale $XII_a$ et $XII_b$,

$$IR \qquad (XIIa)$$
$$ClR \qquad (XIIb)$$

dans laquelle R représente un radical alkyle linéaire ou ramifié renfermant de 1 à 10 atomes de carbone (éventuellement substitué par un radical hydroxy ou par un radical alcoxy de 1 à 5 atomes de carbone) ou un radical cyclohexylméthyle, à chaud dans un solvant organique et en présence d'une base minérale, pour former les composés de formule générale $Ia_1$, dans laquelle $R_3$ et $R_4$ identiques ont la même signification que R,

. ou en le soumettant à l'action du benzaldéhyde à chaud et en présence d'un alcool de petit poids moléculaire, pour former la benzylimine correspondante, et ensuite, à une hydrogénation catalytique, et puis à l'action de l'acide formique et du formaldéhyde, pour former les composés de formule générale $Ia_1$ dans laquelle $R_3$ représente un radical méthyle et $R_4$ un radical cyclohexylméthyle,

. ou en le soumettant d'abord à l'action du benzaldéhyde en présence d'un solvant aromatique inerte et apolaire et ensuite, après élimination du solvant utilisé, à l'action de borohydrure de sodium, en présence d'un alcool aliphatique polaire de petit poids moléculaire, pour obtenir un composé de formule générale $Ia_1$, dans laquelle $R_3$ représente un hydrogène et $R_4$ un radical benzyle,

lequel ensuite,

que l'on peut soumettre :

. soit à l'action du formaldéhyde et de l'acide formique pour former les composés de formule générale Ia dans laquelle $R_3$ représente un radical méthyle et $R_4$ un radical benzyle,

. soit à l'action d'un iodure d'alkyle de formule générale XII, pour former les composés de formule générale $Ia_1$ dans laquelle $R_3$ a la même signification que R et $R_4$ représente un radical benzyle,

lequel ensuite,

que l'on peut soumettre à une hydrogénation catalytique pour former les composés de formule générale $Ia_1$ dans laquelle $R_3$ a la signification donnée ci-dessus et $R_4$ représente un atome d'hydrogène,

lequel ensuite,

que l'on peut soumettre à l'action d'un iodure d'alkyle de formule générale XIIa pour former les composés de formule générale $Ia_1$ dans laquelle $R_3$ et $R_4$ identiques ou différents représentent chacun un radical alkyle renfermant de 1 à 10 atomes de carbone (éventuellement substitué par un radical hydroxy ou un radical alcoxy de 1 à 5 atomes de carbone) ou un radical cyclohexylméthyle,

– soit

que l'on soumet à une hydrogénation catalytique en présence du palladium sur charbon à 5% à température ambiante, pour obtenir l'amino-8 tétrahydro-5,6,7,8 naphto [2,3b] dihydro-2,3 furanne de formule $I_b$,

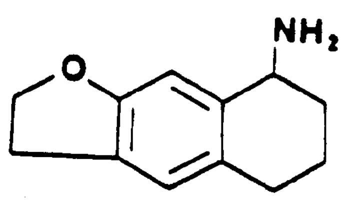

$(I_b)$

lequel ensuite que l'on peut alcoyler selon les méthodes décrites ci-dessus pour les composés de formule générale Ia, pour former les amines secondaires ou tertiaires correspondantes de formule générale $Ib_1$,

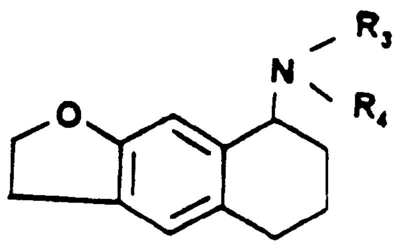

$(Ib_1)$

dans laquelle $R_3$ et $R_4$ ont la signification précédemment donnée pour la formule générale I, sauf qu'ils ne représentent jamais simultanément un atome d'hydrogène,

– **ou**

que l'on soumet à l'action d'un excès de bromure de pyridinium perbromé dans un solvant organique polaire chloré et à une température proche de 0°C pour former la dibromo-7,7 tétrahydro-5,6,7,8 naphto [2,3b]

dihydro-2,3 furannone-8 de formule XIII,

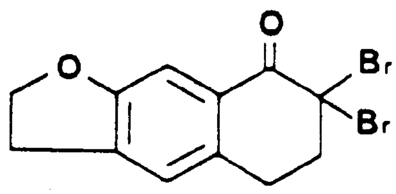

(XIII)

que l'on condense ensuite avec un magnésien d'iodure d'alkyle linéaire ou ramifié renfermant 1 à 4 atomes de carbone, en présence de bromure de cuivre, dans un solvant organique anhydre et à une température comprise entre -20°C et -40°C, pour former les composés de formule générale XIV,

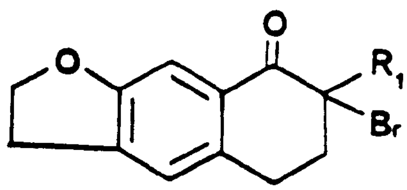

(XIV)

dans laquelle la signification de $R_1$ est celle donnée pour la formule générale I,
que l'on fait réagir avec l'azide de sodium dans un milieu organique acide et à une température d'environ 0°C, pour former un dérivé de l'azido-7 alkyl-7 tétrahydro-5,6,7,8 naphto [2,3b] dihydro-2,3 furannone-8 de formule générale XV,

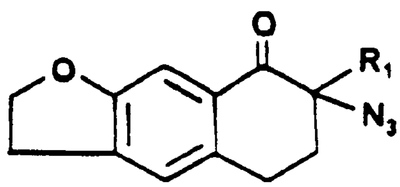

(XV)

dans laquelle $R_1$ a la même signification que précédemment,
que l'on réduit ensuite à l'aide de borohydrure de sodium dans un solvant alcoolique anhydre pour former un dérivé de l'azido-7 alkyl-7 hydroxy-8 tétrahydro-5,6,7,8 naphto [2,3b] dihydro-2,3 furanne de formule générale XVI,

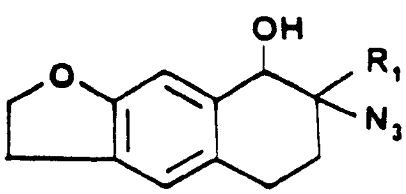

(XVI)

dans laquelle la signification de $R_1$ est identique à celle donnée ci-dessus,
que l'on soumet ensuite à l'action du triéthylsilane en présence d'acide trifluoroacétique sous atmosphère inerte pour former un dérivé de l'azido-7 alkyl-7 tétrahydro-5,6,7,8 naphto [2,3b] dihydro-2,3 furanne de formule générale XVII,

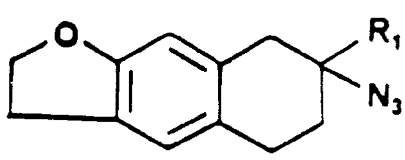

(XVII)

dans laquelle $R_1$ a la même signification que précédemment,
et que l'on fait réagir avec l'hydrazine en présence de Nickel de Raney en milieu anhydre pour former les composés de formule générale Ic,

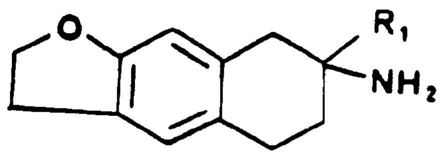

(Ic)

dans laquelle la signification de $R_1$ reste identique à celle mentionnée ci-dessus,
lesquels ensuite que l'on peut alcoyler selon les méthodes décrites pour l'alcoylation du composé Ia, pour former les amines secondaires ou tertiaires correspondantes, de formule générale $Ic_1$,

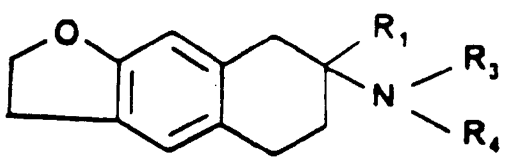

($Ic_1$)

dans laquelle $R_1$ a la signification indiquée ci-dessus, $R_3$ et $R_4$ ont la signification donnée pour la formule générale I, sauf qu'ils ne représentent jamais simultanément un atome d'hydrogène,
– **ou**
que l'on réduit à l'aide d'un amalgame de zinc métallique et de chlorure mercurique en milieu acide et à chaud pour former le tétrahydro-5,6,7,8 naphto [2,3b] dihydro-2,3 furanne de formule XVIII,

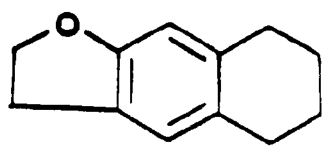

(XVIII)

que l'on oxyde à l'aide de l'anhydride chromique en milieu acide organique et à une température inférieure à 5°C pour former la tétrahydro-5,6,7,8 naphto [2,3b] dihydro-2,3 furannone-5 de formule XIX,

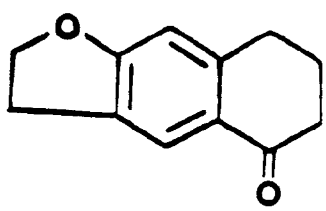

(XIX)

laquelle ensuite,
– soit
que l'on condense avec un sel d'hydroxylamine à une température d'environ 100°C, dans un alcool de petit poids moléculaire pour former la tétrahydro-5,6,7,8 naphto [2,3b] dihydro-2,3 furannone-5 oxime de formule XX,

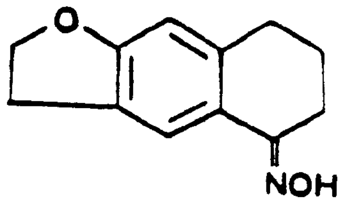

(XX)

laquelle ensuite que l'on soumet à une hydrogénation catalytique et à la température ambiante en présence de palladium sur charbon à 5% pour former l'amino-5 tétrahydro-5,6,7,8 naphto [2,3b] dihydro-2,3 furanne de formule Id,

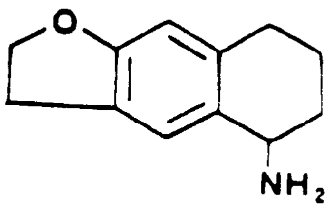

(Id)

lequel ensuite que l'on peut alcoyler selon les méthodes décrites pour l'alcoylation du composé Ia, pour former les amines secondaires ou tertiaires correspondantes de formule générale $Id_1$,

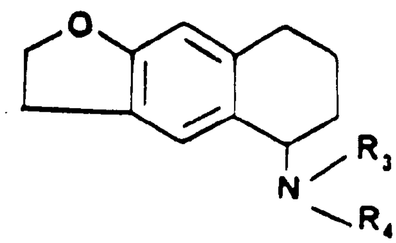

($Id_1$)

dans laquelle $R_3$ et $R_4$ ont la même signification que dans la formule générale I, sauf qu'ils ne représentent jamais simultanément un atome d'hydrogène,

– soit

que l'on soumet à l'action d'une quantité adéquate de bromure de pyridinium perbromé dans un solvant organique polaire pour former un composé de formule générale XXI,

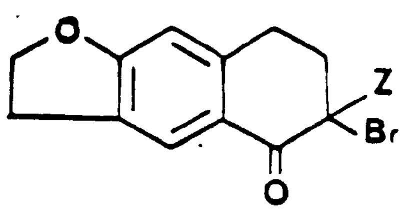

(XXI)

dans laquelle Z représente un atome d'hydrogène quand la quantité de bromure de pyridinium perbromé utilisée est environ équimolaire, ou un atome de brome quand la réaction de bromation a été effectuée avec une quantité au moins double de réactif de bromation,

lequel ensuite,

. que l'on condense, quand il contient en position 6 deux atomes de brome, d'abord avec un magnésien d'iodure d'alkyle linéaire ou ramifié renfermant de 1 à 4 atomes de carbone, en présence de bromure de cuivre, et ensuite que l'on fait réagir avec l'azide de sodium,

ou

. que l'on fait réagir directement avec l'azide de sodium, quand Z représente un atome d'hydrogène, pour former un dérivé de l'azido-6 tétrahydro-5,6,7,8 naphto [2,3b] dihydro-2,3 furannone-5 de formule générale XXII,

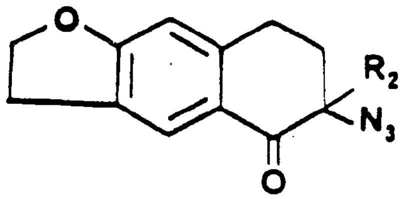

(XXII)

dans laquelle $R_2$ représente un atome d'hydrogène,
que l'on réduit ensuite à l'aide de borohydrure de sodium en présence d'un solvant alcoolique anhydre pour former un dérivé de l'azido-6, hydroxy-5 tétrahydro-5,6,7,8 naphto [2,3b] dihydro-2,3 furanne de formule générale XXIII,

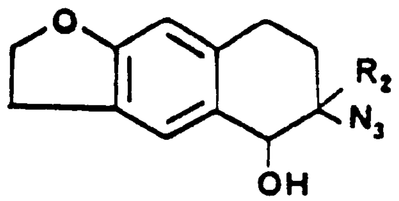

(XXIII)

dans laquelle $R_2$ a la signification indiquée ci-dessus,
que l'on soumet ensuite à l'action du triéthylsilane en présence d'acide trifluoroacétique sous atmosphère inerte pour former un dérivé de l'azido-6 tétrahydro-5,6,7,8 naphto [2,3b] dihydro-2,3 furanne de formule générale XXIV,

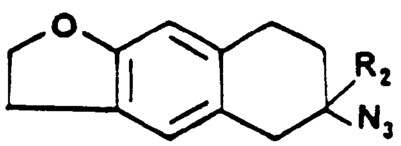

(XXIV)

dans laquelle la signification de $R_2$ reste identique à celle donnée précédemment,
que l'on fait ensuite réagir avec l'hydrazine en présence de nickel de Raney en milieu anhydre pour former un dérivé de l'amino-6 tétrahydro-5,6,7,8 naphto [2,3b] dihydro-2,3 furanne de formule générale Ie,

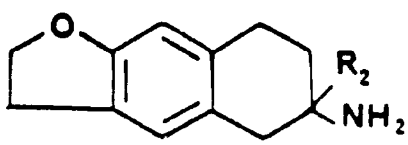

(Ie)

dans laquelle $R_2$ a la signification indiquée ci-dessus,
lequel ensuite que l'on peut alcoyler selon les méthodes décrites précédemment pour l'alcoylation du composé Ia, pour former les amines secondaires ou tertiaires correspondantes de formule générale $Ie_1$,

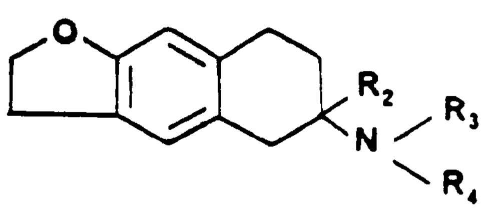

($Ie_1$)

dans laquelle $R_2$, $R_3$ et $R_4$ ont la même signification que dans la formule générale I sauf $R_3$ et $R_4$ ne représentent jamais simultanément un atome d'hydrogène,

soit

que l'on soumet le diméthoxy-2,7 naphtalène de formule XXV,

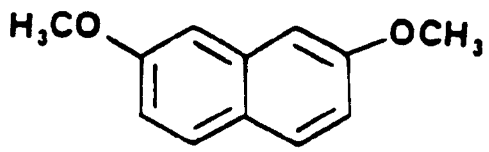

(XXV)

à l'action du sodium métal, à chaud et en présence d'un alcool anhydre, pour former le méthoxy-7 tétrahydro-1,2,3,4 naphtalènone-2 de formule XXVI,

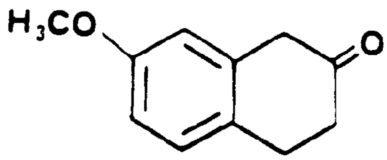

(XXVI)

laquelle ensuite, que l'on fait réagir à chaud dans un solvant alcoolique avec un sel d'hydroxylamine pour former la méthoxy-7 tétrahydro-1,2,3,4 naphtalènone-2 oxime de formule XXVII,

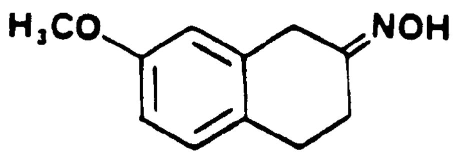

(XXVII)

laquelle que l'on soumet à une hydrogénation catalytique, en solution dans un alcool, en présence de Nickel de Raney et d'ammoniac pour former l'amino-2 méthoxy-7 tétrahydro-1,2,3,4 naphtalène de formule XXVIII,

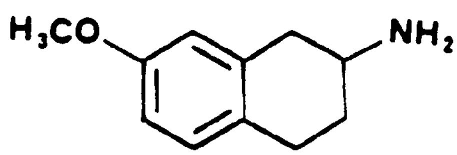

(XXVIII)

que l'on condense avec l'anhydride acétique en milieu acide acétique pour former l'acétamido-2 méthoxy-7 tétrahydro-1,2,3,4 naphtalène de formule XXIX,

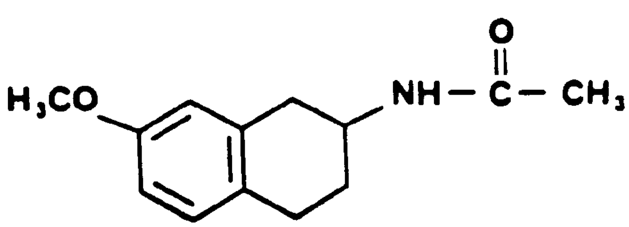

(XXIX)

que l'on soumet à l'action de tribromure de bore, à température ambiante dans un solvant organique halogéné, pour former l'acétamido-2 hydroxy-7 tétrahydro-1,2,3,4 naphtalène de formule XXX,

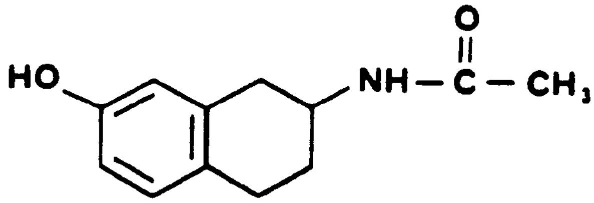

(XXX)

lequel ensuite que l'on fait réagir avec le chloroacétonitrile, en présence de trichlorure de bore et de chlorure d'aluminium, pour former l'acétamido-2 [(chloro-2 oxo-1)éthyl]-6 hydroxy-7 tétrahydro-1,2,3,4 naphtalène de formule XXXI,

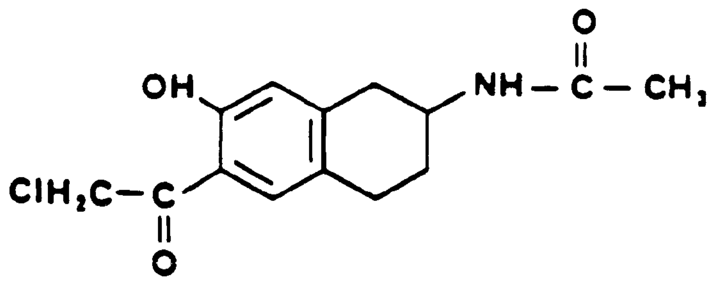

(XXXI)

que l'on soumet à l'action de la triéthylamine, à chaud dans un solvant organique halogéné, pour former l'acétamido-7 tétrahydro-5,6,7,8 naphto [2,3b] dihydro-2,3 furannone-3 de formule XXXII,

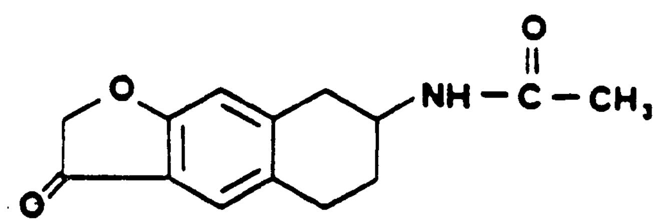

(XXXII)

laquelle ensuite,
– ou
que l'on soumet à l'action d'une base minérale forte pour former le composé de formule If,

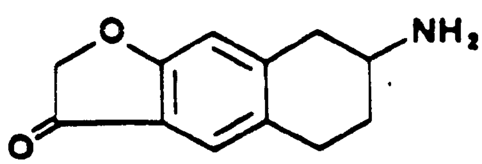

(If)

lequel ensuite que l'on peut alcoyler selon les méthodes décrites ci-dessus pour l'alcoylation du composé Ia pour former les composés de formule générale $If_1$,

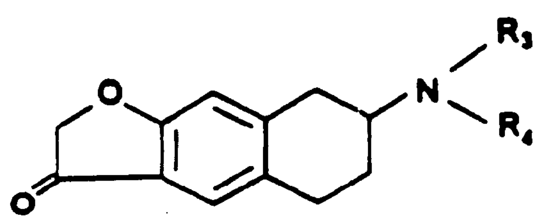

($If_1$)

dans laquelle la définition de $R_3$ et $R_4$ est identique à celle donnée pour la formule générale I, sauf que $R_3$ et $R_4$ ne représentent jamais simultanément un atome d'hydrogène,

– ou

que l'on réduit dans un solvant éthanolique à la température ambiante, par le borohydrure de sodium pour former l'acétamido-7 hydroxy-3 tétrahydro-5,6,7,8 naphto [2,3b] dihydro-2,3 furanne de formule XXXIII,

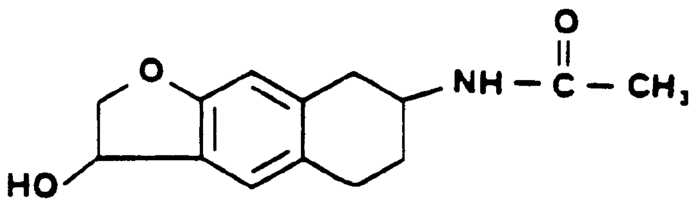

(XXXIII)

lequel ensuite que l'on soumet,

– soit

à l'action d'une base forte pour former le composé de formule Ig,

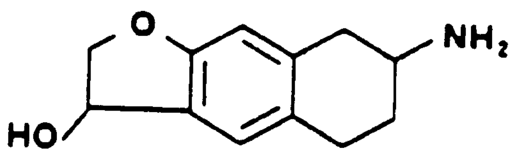

(Ig)

lequel ensuite qu l'on peut alcoyler selon les procédés décrits précédemment pour le composé Ia, pour former les composés de formule générale $Ig_1$,

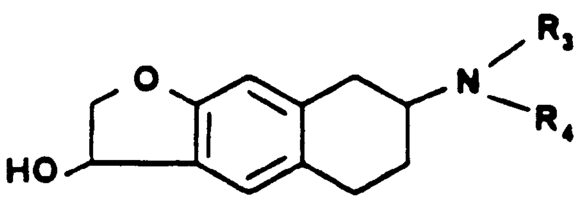

($Ig_1$)

dans laquelle $R_3$ et $R_4$ ont la même signification que dans la formule générale I, sauf que $R_3$ et $R_4$ ne représentent jamais simultanément un atome d'hydrogène,

– soit

à l'action d'un acide minéral fort à température ambiante, pour former l'acétamido-7 tétrahydro-5,6,7,8 naphto [2,3b] furanne de formule XXXIV,

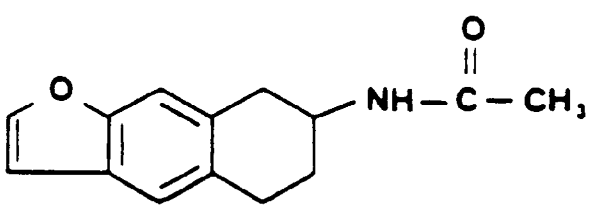

(XXXIV)

lequel ensuite que l'on soumet à l'action d'une base minérale forte en milieu alcoolique pour former le composé de formule Ih,

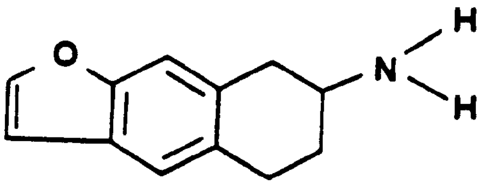

(Ih)

lequel ensuite que l'on peut soumettre à une alcoylation selon les méthodes décrites ci-dessus pour les amines primaires pour former les composés de formule $Ih_1$,

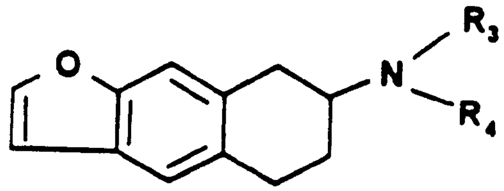

($Ih_1$)

dans laquelle la signification de $R_3$ et $R_4$ est identique à celle de la formule générale I, sauf qu'ils ne représentent jamais simultanément un atome d'hydrogène.

L'ensemble des composés de formule Ia à Ih et $Ia_1$ à $Ih_1$ forme l'ensemble des composés de formule générale I, qui peuvent être salifiés par un acide minéral ou organique pharmaceutiquement acceptable, ou séparés d'abord en leurs isomères optiques et ensuite éventuellement salifiés.

Parmi les acides pharmaceutiquement acceptables pour la préparation des sels d'addition aux composés de formule générale I, on peut citer les acides chlorhydrique, phosphorique, fumarique, citrique, oxalique, sulfurique, tartrique, maléïque, mandélique, méthanesulfonique etc... Les composés de formule générale I peuvent-être séparés en leurs isomères optiques après formation des sels avec les acides (d) et (1) camphosulfonique, dibenzoyl tartrique ou tartrique. Le procédé de préparation des composés Ia, Ib et Ic est illustré dans la planche des formules N°1. Le procédé de préparation des composés Id et Ie est illustré dans la planche des formules N°2, et le procédé de préparation des composés If ,Ig et Ih est illustré dans la planche des formules N°3.

L'intermédiaire de synthèse, la tétrahydro-5,6,7,8 naphto [2,3b] dihydro-2,3 furannone-5 (composé de formule XIX) est un produit nouveau et à ce titre fait partie de la présente invention.

Parmi les composés de l'invention, on peut citer de manière préférentielle les composés de formule générale I dans laquelle le radical amino est en position 7 ou en position 6. Parmi les composés de l'invention, on peut aussi citer de manière préférentielle les composés de formule générale I dans laquelle $R_3$ et $R_4$ identiques représentent chacun un atome d'hydrogène.

Les composés selon l'invention, ainsi que leurs sels et leurs isomères optiques sont doués de propriétés pharmacologiques fort intéressantes. En effet, les essais pharmacologiques in vivo ont montré que les composés possèdent de puissantes propriétés antidépressives, antiagressives et psychostimulantes. Ces propriétés ont été mises en évidence au moyen d'essais classiquement utilisés chez l'animal permettant de présager avec une très bonne précision l'activité chez l'homme. ("Antidepressants : Neurochemical Behavioral and Clinical Perspectives" Enna S.J., Malick J., Richelson E., Raven Press Ed., 1981,N.Y. et "Industrial Pharmacology Antidepressants (II)" Fielding Stuart, Harbans Lal, Futura Publ. Comp. Ed., 1975 N.Y.)

Les résultats des études pharmacologiques ont aussi mis en évidence que les composés de l'invention ont une action dopaminergique, les isomères ayant une activité agoniste plus ou moins intense. Récemment, il a été constaté que, lors de la maladie de Parkinson, il existe une déplétion en dopamine des noyaux gris centraux du système extrapyramydal. C'est ainsi que les agonistes dopaminergiques comme les composés de la présente invention peuvent avoir des effets thérapeutiques très intéressants pour le traitement symptomatique de cette maladie. (Burgers Medicinal Chemistry 4th Ed., Part III, p. 413-430, (1981), J.Wiley and Sons Edt.). La dopamine exerce également un effet puissamment freinateur à la sécrétion de prolactine, hormone ayant comme principale action le développement et le maintien de la lactogénèse.Les composés de la présente invention peuvent donc être utilisés pour le traitement des troubles neuroendocriniens dûs à un déficit dopaminergique, tels que hyperprolactinémie et galactorrhée. (The Pharmacological basis of Therapeutics, 7th Ed.,p.1374-1385, Goodman Gilman A Ed., Macmillan Publish Comp. N.Y.).

L'invention s'étend aussi aux compositions pharmaceutiques renfermant comme principe actif au moins un composé de formule générale I, l'un de ses isomères ou l'un de ses sels avec un acide minéral ou organique

pharmaceutiquement compatible, en association avec un ou plusieurs excipients inertes, et appropriés.

Les compositions pharmaceutiques ainsi obtenues sont présentées avantageusement sous des formes diverses, telles que par exemple comprimés, dragées, gélules, glossettes ou autres préparations galéniques appropriées pour une administration sublinguale, suppositoires, solutions injectables ou buvables.

La posologie peut varier largement en fonction de l'âge, du poids du patient, de la nature et de la sévérité de l'affection ainsi que de la voie d'administration.

La voie d'administration préférée est la voie orale ou parentérale. D'une manière générale, la posologie unitaire s'échelonnera entre 0,5 et 100 mg et la posologie journalière, utilisable en thérapeutique humaine entre 10 et 100 mg.

Les exemples suivants, donnés à titre non-limitatif, illustrent l'invention.

Les points de fusion sont mesurés selon la technique Micro-Köfler. Les spectres de résonance magnétique nucléaire du proton (R.M.N.) ont été enregistrés à 60 MHz.

**EXEMPLE 1**

**Tétrahydro-5,6,7,8 naphto [2,3b] dihydro-2,3 furannone-8**

STADE A

Acide (dihydro-2,3 benzofurannyl-5)-4 oxo-4 butanoïque

Une suspension refroidie à 0°C de 120 g de dihydro-2,3 benzofuranne et de 128 g de chlorure d'aluminium dans 260 ml de dichloroéthane, est ajoutée dans un mélange de 96 g d'anhydride succinique et de 260 g de chlorure d'aluminium dans 512 ml de dichloroéthane. La température est maintenue à environ 5°C. Le mélange réactionnel maintenu 2 heures à cette température est ensuite versé sur une solution de 3 litres d'eau contenant 400 ml d'acide chlorhydrique concentré. Après décantation et extraction au chlorure de méthylène, la phase organique est épuisée à l'hydroxyde de sodium 1N. Les phases basiques réunies sont acidifiées à froid par l'acide chlorhydrique 6N, et l'huile formée est extraite au chlorure de méthylène. La phase organique est lavée à neutralité et ensuite séchée.

Rendement : 65%

Point de fusion : 138°C

Spectre RMN ($CDCl_3$ + DMSO-$d_6$) : 2,65 à 3,2 ppm,t,4H; 3,2 ppm,t,2H; 4,65 ppm,t,2H; 6,8 ppm,m,1H; 7,8 ppm,m,2H; 10,9 ppm 1H échangeable.

STADE B

Acide (dihydro-2,3 benzofurannyl-5)-4 butanoïque

300 g de zinc et 30 g de chlorure mercurique sont ajoutés sous agitation dans un mélange contenant 510 ml d'acide chlorhydrique concentré dans 240 ml d'eau, 141 g d'acide (dihydro-2,3 benzofurannyl-5)-4 oxo-4 butanoïque obtenus ci-dessus, et 300 ml de toluène. Le milieu réactionnel est porté au reflux pendant 6 heures. On ajoute 150 ml d'acide chlorhydrique concentré et on continue le reflux la nuit.

Après refroidissement et décantation, la phase toluénique est épuisée à l'aide de l'hydroxyde de sodium 1N.

De cette phase aqueuse, acidifiée et extraite au chlorure de méthylène, on recueille l'acide (dihydro-2,3 benzofurannyl-5)-4 butanoïque que l'on recristallise dans l'éther isopropylique.

Rendement : 64%

Point de fusion : 84-85°C

Spectre RMN ($CDCl_3$) : 2 ppm,m,2H; 2,4 à 2,5 ppm,t,4H; 3,2 ppm,m,2H; 4,55 ppm,t,2H; 6,7 ppm,m,1H; 6,95 ppm,m,1H; 7,05 ppm,m,1H; 11,15 ppm,1H échangeable.

STADE C

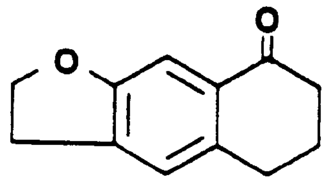

Dans un mélange, porté à 95-100°C, de 258 g d'acide polyphosphorique et de 4,3 litres de xylène, on introduit une solution de 86 g de l'acide obtenu au stade précédent dans 900 ml de xylène.

Après 30 minutes à cette température, le mélange est hydrolysé avec 4,3 litres d'eau et de glace. Après extraction, on lave les phases organiques avec une solution de bicarbonate de sodium à 10%, puis à l'eau saturée de chlorure de sodium.

On obtient après évaporation le produit attendu que l'on recristallise dans l'éther isopropylique.
Rendement : 58,5%
Point de fusion : 67°C
SPECTRE RMN ($CDCl_3$) : 2,15 ppm,m,2H; 2,4 et 3,2 ppm,m,4H; 3,25 ppm,t,2H; 4,65 ppm,t,2H; 7,5 et 7,2 ppm,m,2H

**EXEMPLE 2**

**Chlorhydrate de (d,l) amino-7 tétrahydro-5,6,7,8 naphto [2,3b] dihydro-2,3 furanne**

STADE A

Tétrahydro-5,6,7,8 naphto [2,3b] dihydro-2,3 furannone-8 oxime

Un mélange contenant 46 g du composé de l'exemple 1, 60 g de chlorhydrate d'hydroxylamine et 60 g d'acétate de sodium anhydre dans 410 ml d'éthanol est porté au reflux pendant 5 heures. Le mélange est ensuite refroidi et dilué à l'eau, extrait par le chlorure de méthylène, séché et évaporé à siccité. Le résidu sec est recristallisé dans l'éthanol.
Rendement : 78%
Point de fusion : 165°C
SPECTRE RMN ($CDCl_3$) : 1,8 ppm,m,2H; 2,5 à 2,9 ppm,m,4H; 3,15 ppm,t,2H; 4,55 ppm,t,2H; 7 à 7,4 ppm,m,2H; 10,3 ppm,m,1H.

STADE B

Para-toluènesulfonate de tétrahydro-5,6,7,8 naphto [2,3b] dihydro-2,3 furannone-8 oxime

Un mélange de 35 g de l'oxime obtenue au stade précédent et de 38 g de para-toluènesulfochlorure dans 160 ml de pyridine est agité 16 heures à une température inférieure à 5°C.

Après dilution dans 1 litre d'eau, on extrait le milieu réactionnel par le chlorure de méthylène, on sèche, et on évapore.
Rendement : 94%
Point de fusion : 141-143°C
SPECTRE RMN ($CDCl_3$) : 1,17 ppm,m,2H; 2,4 ppm,s,3H; 2,6 ppm,m,4H; 3,15 ppm,t,2H; 4,5 ppm, t,2H; 6,95 à 7,2 ppm,m,2H; 7,3 ppm,m,2H; 7,9 ppm,m,2H;

STADE C

Acétylamino-7 tétrahydro-5,6,7,8 naphto [2,3b] dihydro-2,3 furannone-8

A une solution refroidie à 0°C d'éthylate de sodium (4,46 g de Na dans 140 ml d'éthanol) est ajoutée une suspension de 57 g du composé obtenu au stade précédent dans 340 ml de benzène anhydre en 1 heure. Le mélange est agité 5 heures à 0°C puis conservé la nuit en glacière.

Après addition de 260 ml d'acide chlorhydrique 2N, le mélange est filtré. La phase acide est alcalinisée puis extraite au chlorure de méthylène. Après séchage et évaporation, l'huile est immédiatement acétylée par un mélange de 44 ml d'acide acétique et de 11 ml d'anhydride acétique.

On dilue à l'eau, extrait au chlorure de méthylène et ensuite on lave à l'eau la phase organique. Après évaporation, le résidu est recristallisé dans l'acétonitrile.

Rendement : 7%

Point de fusion : 180°C

SPECTRE RMN ($CDCl_3$) : 2,1 ppm,s,3H; 1,5 à 3,2 ppm,m,4H; 3,3 ppm,t,2H; 4,6 ppm,m,1H; 4,65 ppm,t,2H; 7,0 ppm, 1H échangeable; 7,15 ppm,m,1H; 7,4 ppm,m,1H.

STADE D

Acétylamino-7 tétrahydro-5,6,7,8 naphto [2,3b] dihydro-2,3 furanne

2,4 g du composé obtenu au stade précédent sont réduits dans un appareil de Parr pendant 5 heures à température ambiante sous pression de 5 kg d'hydrogène, en solution dans 54 ml d'acide acétique et 2 ml d'acide perchlorique à 70% en présence de palladium sur charbon à 5%. Le milieu réactionnel est ensuite dilué à l'aide de chlorure de méthylène, lavé avec une solution de bicarbonate de sodium à 10% dans l'eau, puis à l'eau pure. Après évaporation du solvant et recristallisation dans l'acétonitrile, on obtient le produit pur.

Rendement : 50%

Point de fusion : 185-186°C

SPECTRE RMN ($CDCl_3$) : 1,2 à 2,5 ppm,m,5H; 2,5 à 3,3 ppm,m,6H; 3,5 à 4,5 ppm,m,1H; 4,5 ppm,t,2H; 5,5 à 6,1 ppm 1H échangeable; 6,5 ppm,s,1H; 7 ppm,s,1H.

STADE E

1 g d'amide obtenu au stade précédent est porté au reflux 18 heures avec 25 ml d'acide chlorhydrique 4N.

Après refroidissement, le milieu réactionnel est alcalinisé, et extrait par le chlorure de méthylène. Après séchage et évaporation, on obtient l'amino-7 tétrahydro-5,6,7,8 naphto [2,3b] dihydro-2,3 furanne.

Point de fusion : 60°C

Le chlorhydrate correspondant est obtenu après dissolution de la base obtenue ci-dessus dans l'acétate d'éthyle et addition d'une quantité adéquate d'acide chlorhydrique en solution dans l'éther éthylique.

Rendement global : 55%

Point de fusion : 275°C.

Le spectre RMN de ce composé est indiqué dans le tableau I.

**EXEMPLE 3**

**Chlorhydrate de (d) amino-7 tétrahydro-5,6,7,8 naphto [2,3b] dihydro-2,3 furanne**

STADE A

Camphosulfonate de (d) amino-7 tétrahydro-5,6,7,8 naphto [2,3b] dihydro-2,3 furanne

Ce sel a été obtenu en faisant réagir le composé de l'exemple 2 avec une quantité équimolaire de l'acide (d)camphosulfonique. Après deux recristallisations dans l'éthanol suivies de deux recristallisations dans le méthanol, le sel est obtenu optiquement pur.

Point de fusion : 253-261°C

Pouvoir rotatoire d'une solution à 0,5% dans l'eau :

| λ nm | $[\alpha]_D^{23°C}$ |
|---|---|
| 589 | + 41,3° |
| 578 | + 43,3° |
| 546 | + 51,1° |
| 436 | + 105,6° |
| 365 | + 235,0° |

STADE B

Le camphosulfonate obtenu au stade précédent, est mis en solution dans l'acétate d'éthyle et ensuite le milieu est basifié par l'hydroxyde de sodium. La phase organique est séparée, séchée sur sulfate de sodium anhydre et évaporée. L'huile obtenue est reprise dans l'acétonitrile, et ensuite une quantité stoechiométrique d'éther chlorhydrique est additionnée, pour obtenir le chlorhydrate de (d)amino-7 tétrahydro-5,6,7,8 naphto [2,3b] dihydro-2,3 furanne optiquement pur.
Point de fusion : 261-264°C
Pouvoir rotatoire d'une solution à 0.25% dans le D.M.S.O.:

| λ nm | $[\alpha]_D^{23°C}$ |
|---|---|
| 589 | + 86,4° |
| 578 | + 90,4° |
| 546 | + 104,4° |
| 436 | + 191,6° |
| 365 | + 350,2° |

**EXEMPLE 4**

**Chlorhydrate de (l) amino-7 tétrahydro-5,6,7,8 naphto [2,3b] dihydro-2,3 furanne**

Ce chlorhydrate a été obtenu selon le procédé décrit dans l'exemple 3 mais en utilisant au stade A l'acide (l) camphosulfonique.
Point de fusion : 262-264°C
Pouvoir rotatoire d'une solution à 0.25% dans le D.M.S.O.:

| λ nm | $[\alpha]_D^{23°C}$ |
|---|---|
| 589 | - 86,4° |
| 578 | - 90,4° |
| 546 | - 104,4° |
| 436 | - 191,6° |
| 365 | - 350,2° |

**EXEMPLE 5**

**(d,l) (N,N-dipropylamino)-7 tétrahydro-5,6,7,8 naphto [2,3b] dihydro-2,3 furanne**

A 1,5 g du composé de l'exemple 2 dissout dans 10 ml d'acétonitrile sont ajoutés 13,4 g d'iodure de propyle et 11 g de carbonate de potassium.

Après 4 jours sous agitation efficace à température ambiante, le mélange est filtré et le solvant concentré sous vide. Le résidu est recristallisé dans le pentane pour donner le produit attendu.

Rendement : 45%

Point de fusion : 46-48°C

Le spectre RMN du composé est indiqué dans le tableau I.

**EXEMPLE 6**

**Chlorhydrate de (d,l) (N,N-diméthylamino)-7 tétrahydro-5,6,7,8 naphto [2,3b] dihydro-2,3 furanne**

A une solution de 1,1 g du composé de l'exemple 2 dans 2 ml d'acide formique concentré sont ajoutés 3,35 ml d'une solution de formaldéhyde à 37% dans l'eau. Le mélange est porté 2 heures au reflux, refroidi, dilué avec une solution d'hydroxyde de sodium à 10% dans l'eau et ensuite extrait au chlorure de méthylène. La phase organique est concentrée et ensuite est ajoutée une quantité adéquate d'éther chlorhydrique. Le sel ainsi obtenu est recristallisé dans l'acétonitrile.

Rendement : 64%

Point de fusion : 230°C

Le spectre RMN du (d,l) (N,N-diméthylamino)-7 tétrahydro-5,6,7,8 naphto [2,3b] dihydro-2,3 furanne est indiqué dans le tableau I.

**EXEMPLE 7**

**(d,l) (N-benzylamino)-7 térahydro-5,6,7,8 naphto [2,3b] dihydro-2,3 furanne**

45 g du composé de l'exemple 2, 25,4 g d'aldéhyde benzoïque et 300 ml de benzène sont portés au reflux, en éliminant l'eau formée. On évapore à sec, reprend par 300 ml d'éthanol et ajoute par portions en gardant la température à environ 18°C, 9 g de borohydrure de sodium. Après une nuit à la température ambiante, l'éthanol est éliminé sous vide et le résidu est repris par une solution d'acide chlorhydrique diluée. La phase acide est basifiée puis extraite à l'éther éthylique. Après évaporation du solvant, l'huile obtenue est purifiée sur colonne de silice en utilisant comme solvant d'élution un mélange de chlorure de méthylène et de méthanol (95:5 V/V).

Rendement : 37,7%

Le spectre RMN de ce composé est indiqué dans le Tableau I.

**EXEMPLE 8**

**(d,l) (N-benzyl N-méthyl amino)-7 tétrahydro-5,6,7,8 naphto [2,3b] dihydro-2,3 furanne**

A une solution de 21 g du composé de l'exemple 7 dans 26 ml d'acide formique concentré maintenu à 0°C sont ajoutés 44 ml d'une solution d'aldéhyde formique à 37% dans l'eau. Après 2 heures de reflux, le milieu réactionnel refroidi est dilué avec une solution d'hydroxyde de sodium à 40% dans l'eau et extrait au chlorure de méthylène. La phase organique est lavée, séchée puis évaporée sous vide. Le résidu purifié par passage sur colonne de silice conduit à l'obtention d'une huile. Le solvant d'élution utilisé est un mélange de chlorure de méthylène et d'acétate d'éthyle (85:15 V/V)
Rendement : 33,3%
Le spectre RMN du composé est indiqué dans le Tableau I.

**EXEMPLE 9**

**Chlorhydrate de (d,l) (N-méthylamino)-7 tétrahydro-5,6,7,8 naptho [2,3b] dihydro-2,3 furanne**

7 g du composé de l'exemple 8 dans 240 ml d'éthanol sont introduits dans un appareil de Parr sous pression de 5 kg/cm$^2$ d'hydrogène, à température ambiante, avec 0,5 g de palladium à 5% sur charbon. Après filtration du catalyseur et évaporation du solvant, on obtient la base brute. Le chlorhydrate est formé après dilution de la base dans l'acétate d'éthyle et addition d'éther chlorhydrique.
Rendement : 67%
Point de fusion : 231-234°C
Le spectre RMN de ce composé est indiqué dans le Tableau I.

**EXEMPLE 10**

**Chlorhydrate de (d,l) (N-cyclohexylméthyl, N-méthyl amino)-7 tétrahydro-5,6,7,8 naptho [2,3b] dihydro-2,3 furanne**

STADE A

(N-benzyl imino)-7 tétrahydro-5,6,7,8 naphto [2,3b] dihydro-2,3 furanne

10 g du composé de l'exemple 2 sont portés au reflux dans 200 ml d'éthanol en présence de 2,8 g de benzaldéhyde. Un échantillon est prélevé, concentré et recristallisé dans l'éthanol.
Point de fusion : 122°C
Spectre RMN ($CDCl_3$) : 1,5 à 2,3 ppm,m,2H; 2,7 à 3,3 ppm,m,4H; 3 à 3,4 ppm,t,2H; 3,3 à 3,9 ppm,m,1H; 4,55 ppm,t,2H; 6,6 ppm, s,1H; 7 ppm,s,1H; 7,3 à 7,6 ppm,m,3H; 7,6 à 8 ppm,m,2H; 8,5 ppm,s,1H.

STADE B

L'imine obtenue au stade précédent est ensuite hydrogénée en présence de 0,3 g d'oxyde de platine (IV) sous pression de 10 kg/cm$^2$ d'hydrogène pendant 4 heures à 70°C. Après filtration du catalyseur et évaporation du solvant sous vide, on obtient 9 g d'une huile contenant un mélange de dérivés benzénique et cyclohexanique.
Ce mélange est alors traité tel quel par 9,9 ml d'acide formique à 0°C, puis par 16,7 ml d'une solution de formaldéhyde à 37% dans l'eau. Après chauffage pendant 2 heures à reflux, on neutralise avec une solution d'hydroxyde de sodium à 20% dans l'eau, on extrait au chlorure de méthylène et on lave à l'eau. Le solvant est évaporé sous vide, pour obtenir une huile qu'on purifie sur colonne de silice en utilisant comme éluant un mélange de chlorure de méthylène et d'acétate d'éthyle. On obtient le [d,l] (N-cyclohexylméthyl, N-méthylamino)-7 tétrahydro-5,6,7,8 naphto [2,3b] dihydro-2,3 furanne. Après dilution de ce dernier composé dans l'acétonitrile et addition d'éther chlorhydrique, on obtient le chlorhydrate de [d,l] (N-cyclohexylméthyl N-méthyl amino)-7 tétrahydro-5,6,7,8 naphto [2,3b] dihydro-2,3 furanne.
Rendement : 6,5%
Le spectre RMN de ce composé est indiqué dans le Tableau I.

**EXEMPLE 11**

**(d,l) [N-(tétrahydro-5,6,7,8 naphto [2,3b] dihydro-2,3 furannyl-7) amino]-7 heptanoate d'éthyle**

Un mélange de 5,5 g du composé de l'exemple 2, 7,11 g de bromo-7 heptanoate d'éthyle et de 4,25 g de carbonate de sodium en solution dans 35 ml d'éthanol sont portés au reflux pendant 20 heures. Après dilution par 20 ml d'hydroxyde de sodium 1N, on extrait à l'acétate d'éthyle, on lave à l'eau, on sèche et on évapore sous vide le solvant. Le résidu est repris sous agitation par une quantité stoechiométrique d'acide chlorhydrique 1N, jusqu'à cristallisation complète. Après filtration et séchage, on recueille le chlorhydrate.
Rendement : 50%
Point de fusion : 161-166°C

Le spectre RMN de ce composé est indiqué dans le Tableau I.

**EXEMPLE 12**

**Chlorhydrate de l'acide (d,l) [N-(tétrahydro-5,6,7,8 naphto [2,3b] dihydro-2,3 furannyl-7) amino]-7 heptanoïque**

A une suspension de 4,7 g du composé de l'exemple 11 sous forme de chlorhydrate dans l'éthanol est additionnée une solution de 1,4 g d'hydroxyde de potassium dans 10 ml d'eau. Après 24 heures à température ambiante, on ajoute de nouveau une quantité de la solution de l'hydroxyde de potassium et on agite 2 heures à température ambiante. L'éthanol est évaporé sous vide sans chauffer. Le résidu est repris par de l'acide chlorhydrique 1N, et ensuite il est recristallisé dans l'eau.
Rendement : 62%
Point de fusion : 197-200°C.

Le spectre RMN de ce composé est indiqué dans le Tableau I.

**EXEMPLE 13**

**(d,l) [N-(éthoxy-2) éthyl) amino]-7 tétrahydro-5,6,7,8 naptho [2,3b] dihydro-2,3 furanne**

Un mélange contenant 7,8 g du composé de l'exemple 2, 6,7 g de bromo-2 éthyl éthyléther et 6,2 g de carbonate de sodium dans l'éthanol est porté au reflux sous agitation pendant 20 heures.

Après filtration et lavage du résidu par l'éthanol, les phases éthanoliques sont évaporées. L'huile résiduelle est reprise par l'eau et l'éther et après décantation, la phase éthérée est épuisée à l'acide chlorhydrique 1N. La phase aqueuse acide basifiée à froid, extraite à l'éther, séchée et ensuite évaporée, conduit à une huile de structure attendue.
Rendement : 44%

Le spectre RMN de ce composé est indiqué dans le Tableau I.

L'huile obtenue ci-dessus mise en solution dans l'éthanol et après acidification par l'éther chlorhydrique donne le chlorhydrate correspondant.
Point de fusion : 180-181°C.

**EXEMPLE 14**

**(d,l) [N-(hydroxy-2 éthyl) amino]-7 tétrahydro-5,6,7,8 naphto [2,3b] dihydro-2,3 furanne**

7,7 g d'amino-7 tétrahydro-5,6,7,8 naphto [2,3b] dihydro-2,3 furanne, 5 g de bromoéthanol, 40 ml de chlorure de méthylène, 12 ml d'hydroxyde de sodium à 40% dans l'eau et 1 g de triton B sont agités vigoureusement à la température ambiante pendant 48 heures. On décante, on évapore à froid le chlorure de méthylène, on reprend à l'éther éthylique, on épuise à l'acide chlorhydrique. Les phases acides basifiées conduisent à une huile de structure attendue.
Rendement : 71%

Le spectre RMN de ce composé est indiqué dans le tableau I.

**EXEMPLE 15**

**Chlorhydrate de (d,l) amino-8 tétrahydro-5,6,7,8 naphto [2,3b] dihydro-2,3 furanne**

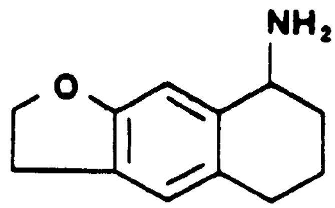

2 g d'oxime obtenue au stade A de l'exemple 2 dans 200 ml d'éthanol sont introduits dans un appareil de Parr sous pression de 5 kg/cm$^2$ d'hydrogène avec 0,5 g de palladium sur charbon à 5% pendant 5 heures. Après filtration du catalyseur et évaporation du solvant sous vide, on obtient 1,7 g de base brute. Le chlorhydrate est obtenu dans l'acétate d'éthyle après addition d'éther chlorhydrique.
Rendement : 53,3%
Point de fusion : >260°C
Spectre RMN ($D_2O$) : 1,85 ppm,m,4H; 2,6 ppm,t,2H; 4,35 ppm,m,1H; 3 ppm,t,2H; 4,4 ppm,t,2H; 6,7 à 7 ppm,m,2H

**EXEMPLE 16**

**Tétrahydro-5,6,7,8 naptho [2,3b] dihydro-2,3 furanne**

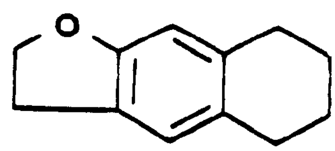

Un amalgame de zinc et de chlorure mercurique (72 g Zn et 7,1 g $HgCl_2$) est ajouté dans un mélange contenant 120 ml d'acide chlorhydrique concentré, 57 ml d'eau, 30 g du composé de l'exemple 1 et 71 ml de toluène, sous agitation.

Le milieu réactionnel est porté au reflux pendant 4 heures. Après refroidissement et décantation, la phase toluénique est lavée par une solution de bicarbonate de sodium, puis par une solution de chlorure de sodium saturée. La phase organique est séchée puis concentrée sous vide et le résidu est distillé sous vide pour obtenir le composé attendu.
Rendement : 59%
Spectre RMN ($CDCl_3$) : 1,7 ppm,m,4H; 2,65 ppm,m,4H; 3,05 ppm,t,2H; 4,45 ppm,t,2H; 6,5 ppm,m, 1H; 6,9 ppm,m,1H

**EXEMPLE 17**

**Tétrahydro-5,6,7,8 naptho [2,3b] dihydro-2,3 furannone-5**

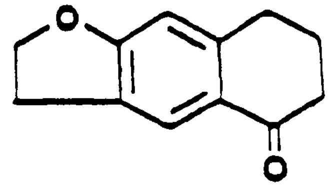

30 g du composé de l'exemple 16 sont dissouts dans 130 ml d'acide acétique et 32 ml d'acide propionique puis refroidis entre 0° et 5°C. Une solution de réactif de Jones est ajoutée en maintenant la température environ

à 5°C. Le mélange maintenu une heure à cette température est concentré sous vide, puis repris par une solution de bicarbonate de sodium en présence d'éther. Après filtration, la phase éthérée est décantée et lavée par une solution saturée de chlorure de sodium, pour obtenir après évaporation et séchage une huile qui cristallise.
Rendement : 56%
Point de fusion : 78°C
Spectre RMN ($CDCl_3$) : 2,1 ppm,m,2H; 2,6 ppm,t,2H; 2,9 ppm,t,2H; 3,2 ppm,t,2H; 4,65 ppm,t,2H; 6,6 ppm,m,1H; 7,9 ppm,m,1H.

**EXEMPLE 18**

**Chlorhydrate de (d,l) amino-5 tétrahydro-5,6,7,8 naphto [2,3b] dihydro-2,3 furanne**

STADE A

Tétrahydro-5,6,7,8 naptho [2,3b] dihydro-2,3 furannone-5 oxime

18 g du composé de l'exemple 17, 23,5 g de chlorhydrate d'hydroxylamine et 23,5 g d'acétate de sodium dans 160 ml d'éthanol sont portés au reflux pendant 2 heures. Le mélange est ensuite refroidi, dilué à l'eau puis extrait au chlorure de méthylène. Après séchage de la phase organique sur sulfate de magnésium anhydre et évaporation à siccité, on obtient, après recristallisation dans l'éthanol, l'oxime.
Rendement : 60,7%
Point de fusion : 155-156°C
Spectre RMN ($CDCl_3$) : 1,75 ppm,m,2H; 2,6 ppm,m,4H; 3,1 ppm,t,2H; 4,5 ppm,t,2H; 6,5 ppm,m,1H; 7,7 ppm,m,1H; 8,3 ppm 1H échangeable.

STADE B

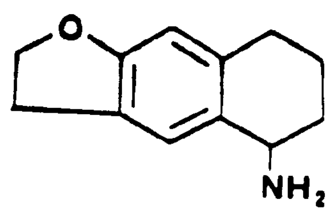

5 g d'oxime obtenu au stade précédent dans 750 ml d'éthanol sont introduits dans un appareil de Parr sous pression de 5 kg/$cm^2$ d'hydrogène avec 2 g de palladium sur charbon à 5% pendant environ 2,30 heures. Après filtration du catalyseur et évaporation du solvant, on obtient 4,5 g de base brute. Le chlorhydrate est obtenu après dissolution de la base dans l'acétate d'éthyle et addition d'éther chlorhydrique.
Rendement : 36%
Point de fusion : 208°C
Spectre RMN ($D_2O$) : 1,9 ppm,t,4H; 2,65 ppm,t,2H; 3,1 ppm,t, 2H; 4 à 4,5 ppm,m,3H; 6,6 ppm,m,1H; 7,2 ppm,m,1H.

**EXEMPLE 19**

**Chlorhydrate de (d,l) amino-6 tétrahydro-5,6,7,8 naphto [2,3b] dihydro-2,3 furanne.**

STADE A

Bromo-6 tétrahydro-5,6,7,8 naphto [2,3b] dihydro-2,3 furannone-5

A une solution de 27,1 g de tétrahydro-5,6,7,8 naphto [2,3b] dihydro-2,3 furannone-5, composé de l'exemple 17, dans 1,5 l de chloroforme, sont ajoutés par portions à 0°C, 55,33 g de bromure de pyridinium perbromé. On abandonne une nuit à température ambiante. Le mélange réactionnel est ensuite lavé à l'eau et la phase organique séchée sur sulfate de magnésium anhydre. L'évaporation du chloroforme conduit à 39,85 g d'un mélange qui est ensuite purifié sur colonne de silice en utilisant comme solvant un mélange de chlorure de méthylène et de cyclohexane (80:20 V/V). Le produit obtenu est ensuite recristallisé dans l'acétate d'éthyle.

Rendement : 48%
Point de fusion : 106°C
Spectre RMN ($CDCl_3$) : 3,2 ppm,m,4H; 3,2 ppm,t,2H; 4,5 à 4,8 ppm,m,3H; 6,65 ppm,s,1H; 8 ppm,s,1H

STADE B

Azido-6 tétrahydro-5,6,7,8 naphto [2,3b] dihydro-2,3 furannone-5

A une solution de 18,4 g du composé obtenu au stade précédent dans 535 ml de diméthylformamide et 13 ml d'acide acétique, est ajoutée, goutte à goutte, à 0°C, une solution de 4,50 g d'azide de sodium dans 65 ml d'eau distillée. Le milieu réactionnel est conservé une nuit à -18°C et ensuite il est dilué à l'eau et repris par le dichlorométhane. La phase organique est séchée et évaporée pour obtenir 19 g du produit attendu.

STADE C

Azido-6 hydroxy-5 tétrahydro-5,6,7,8 naphto [2,3b] dihydro-2,3 furanne

19 g du composé obtenu au stade précédent sont mis en solution dans l'éthanol anhydre. A cette solution sont ajoutés 1,3 g de borohydrure de sodium. Après une heure à température ambiante, le mélange réactionnel est concentré sous vide, repris à l'eau et extrait au chlorure de méthylène. La phase organique est séchée sur sulfate de magnésium anhydre et évaporée. On obtient 9,8 g d'azido-6 hydroxy-5 tétrahydro-5,6,7,8 naphto [2,3b] dihydro-2,3 furanne que l'on utilise au stade suivant sans autre purification.

STADE D

Azido-6 tétrahydro-5,6,7,8 naphto [2,3b] dihydro-2,3 furanne

Le composé obtenu au stade précédent est mis en solution dans 288 ml de triéthylsilane et 288 ml de tétrachlorure de carbone. Sous azote, sont ajoutés, goutte à goutte, 144 ml d'acide trifluoroacétique. Le mélange réactionnel est abandonné pendant 36 heures à température ambiante, puis il est versé sur de la glace et décanté. La phase organique est lavée à l'hydroxyde de sodium 1N, séchée sur sulfate de magnésium, puis évaporée. On obtient 5,9 g d'azido-6 tétrahydro-5,6,7,8 naphto [2,3b] dihydro-2,3 furanne qui sont aussitôt remis en réaction.

STADE E

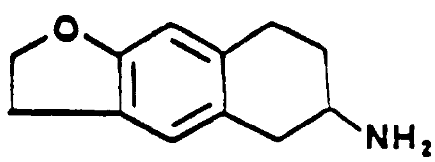

Le composé obtenu au stade précédent est mis en solution dans 400 ml d'un mélange d'éthanol et de tétrahydrofuranne (V/V) anhydre, contenant 10 ml de Nickel de Raney. Sont ajoutés ensuite, goutte à goutte, 4 ml d'hydrate d'hydrazine dilués dans 20 ml d'éthanol. On abandonne une nuit à température ambiante et ensuite le mélange réactionnel est évaporé à sec, repris avec le chlorure de méthylène et filtré. Le filtrat est séché et ensuite, évaporé. Le chlorhydrate de l'amine est formé par dissolution du résidu de l'évaporation dans l'éthanol et addition d'éther chlorhydrique. La solution est évaporée à sec. Le chlorhydrate est recristallisé dans le méthanol.
Point de fusion : 241-243°C
Spectre RMN ($D_2O$ + 1gt DCl) : 1,5 à 2,5 ppm,m,2H; 2,5 à 3,7 ppm,m, 1H + t,2H, + m,4H; 4,4 ppm,t,2H; 6,6 ppm,s,1H; 7,1 ppm, s,1H.

**EXEMPLE 20**

**Chlorhydrate de (d,l) amino-6 méthyl-6 tétrahydro-5,6,7,8 naptho [2,3b] dihydro-2,3 furanne**

STADE A

Dibromo-6,6 tétrahydro-5,6,7,8 naphto [2,3b] dihydro-2,3 furannone-5

A une solution de 6,21 g du composé de l'exemple 17 dans 350 ml de chloroforme sont ajoutés par portions, 24,44 g de bromure de pyridinium perbromé. Après 10 heures de contact, le mélange réactionnel est versé sur de la glace, décanté et la phase organique est lavée au thiosulfate de sodium 0,1 N, puis à l'eau et ensuite séchée et évaporée. Le résidu est recristallisé dans l'acétate d'éthyle pour obtenir la dibromo-6,6 tétrahydro-5,6,7,8 naphto [2,3b] dihydro-2,3 furannone-5 pure.
Rendement : 61%
Point de fusion : 138-142°C
Spectre RMN (DMSO-$d_6$) : 3 ppm,s,4H; 3 à 3,4 ppm,t,2H; 4,5 à 4,8 ppm,t,2H; 6,65 ppm,s,1H; 7,9 ppm,s,1H.

STADE B

Bromo-6 méthyl-6 tétrahydro-5,6,7,8 naphto [2,3b] dihydro-2,3 furannone-5

A une solution de 5,94 g de bromure de cuivre (I) dans 120 ml de tétrahydrofuranne anhydre sont ajoutés rapidement à -40°C 42 ml d'iodure de méthylmagnésium 2N. On laisse remonter la température à 0°C et après 45 minutes, la température du milieu est portée à -60°C et sont additionnés rapidement 6,92 g du composé obtenu au stade précédent. Après 10 minutes de contact à -60°C, la température de milieu est remontée à environ 20°C et sont ajoutés 5 ml d'iodure de méthylène. On laisse reposer le milieu réactionnel à 20°C et ensuite le mélange est hydrolysé à -60°C avec 80 ml d'acide sulfurique 4N. Le milieu réactionnel est extrait au pentane, et ensuite la phase organique est séchée et évaporée. Après recristallisation dans l'éther isopropylique, on obtient la bromo-6 méthyl-6 tétrahydro-5,6,7,8 naphto [2,3b] dihydro-2,3 furannone-5 pure.
Rendement : 12%
Point de fusion : 95-98°C
Spectre RMN ($CDCl_3$) : 2 ppm,s,3H; 1,9 à 3,2 ppm,m,4H; 3,2 ppm,t,2H; 4,7 ppm,t,2H; 6,6 ppm,s,1H; 2 ppm,s,1H.

STADE C

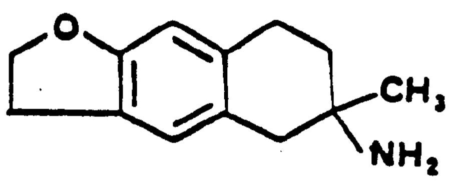

Ce composé a été obtenu à partir de la bromo-6 méthyl-6 tétrahydro-5,6,7,8 naphto (2,3b) dihydro-2,3 furannone-5 et selon les procédés décrits dans l'exemple 19, stades B, C, D, et E.

**EXEMPLE 21**

**Chlorhydrate de (d,l) amino-7 tétrahydro-5,6,7,8 naphto [2,3b] furanne**

STADE A

méthoxy-7 tétrahydro-1,2,3,4 naphtalènone-2

46,84 g (0,249 m) de diméthoxy-2,7 naphtalène sont mis en suspension dans 508 ml d'éthanol anhydre. On ajoute le plus rapidement possible 48,7 g de sodium métal puis on chauffe jusqu'à dissolution totale du sodium. Après avoir hydrolysé avec précaution par 425 ml d'eau, on ajoute le plus rapidement possible, en refroidissant, 468 ml d'acide chlorhydrique concentré. On observe la formation d'un précipité. On porte à reflux

pendant environ 2 heures. On laisse refroidir puis on extrait par 1200 ml d'éther éthylique, on lave la phase organique à neutralité avec de l'eau et on la sèche sur sulfate de magnésium.

L'huile obtenue est reprise sous agitation par 53 ml d'une solution d'hydrosulfite de sodium à 50% et 30 ml d'eau. Après 30 minutes de contact, l'addition d'eau entraîne la formation d'un précipité. On le filtre. Le résidu est lavé à l'éther éthylique, et ensuite repris dans 250 ml de carbonate de sodium à 10% et 100 ml d'éther éthylique. On agite les deux phases jusqu'à limpidité pour obtenir 20,2 g de produit attendu.

STADE B

Méthoxy-7 tétrahydro-1,2,3,4 naphtalènone-2 oxime

4,12 g du composé obtenu au stade précédent sont dissouts dans 40 ml d'éthanol en présence de 6,27 g de chlorhydrate d'hydroxylamine et 5,8 g d'acétate de sodium. Le mélange réactionnel est porté pendant 5 heures à reflux et ensuite versé sur 100 ml d'eau, puis extrait par 240 ml de chlorure de méthylène. La phase organique est lavée avec une solution saturée de chlorure de sodium et séchée sur sulfate de magnésium anhydre. Après évaporation du solvant, le résidu obtenu est recristallisé dans 30 ml d'éther isopropylique pour obtenir le composé attendu pur.
Rendement : 42%
Point de fusion : 120-122°C
Spectre RMN ($CDCl_3$) : 2,5 à 3 ppm,m,4H; 3,5 ppm,s,2H; 3,8 ppm,s,3H; 6,6 à 7,3 ppm,m,3H; 8,1 ppm, 1H échangeable

STADE C

Amino-2 méthoxy-7 tétrahydro-1,2,3,4 naphtalène

2 g du composé obtenu au stade précédent dissouts dans 50 ml d'éthanol, sont hydrogénés en présence de 5 ml de Nickel de Raney et de 2 ml d'ammoniaque. Après absorption de la quantité théorique d'hydrogène, on évapore l'éthanol, reprend par 50 ml de chlorure de méthylène et extrait par 4 fois 40 ml d'acide chlorhydrique 1N. La phase aqueuse acide est basifiée à l'aide d'hydroxyde de sodium 1N extraite au chlorure de méthylène, lavée à neutralité, séchée sur sulfate de magnésium anhydre et évaporée pour obtenir 1,1 g d'une huile dont la structure correspond à celle de l'amine attendue.
Rendement : 64%
Spectre RMN ($CDCl_3$) : 1,4 ppm, 2H échangeables, 1,5 à 3,4 ppm,m,7H, 3,8 ppm,s,3H; 6,6 à 6,9 ppm,m,2H; 7,1 ppm,d,1H.

STADE D

Acétamido-2 méthoxy-7 tétrahydro-1,2,3,4 naphtalène

La quantité totale obtenue au stade précédent est traitée par 1 ml d'anhydride acétique en présence de 10 ml d'acide acétique. Après environ 3 heures de contact, on reprend par 50 ml d'eau et 50 ml d'acide chlorhydrique 1N, on extrait au chlorure de méthylène, on lave à neutralité et on sèche sur sulfate de magnésium anhydre. Après évaporation du solvant, on obtient l'amide attendu.
Rendement : 63%
Point de fusion : 106-108°C
Spectre RMN ($CDCl_3$) : 1,9 ppm,s,3H; 1,3 à 2,1 ppm,m,2H; 2,2 à 3,3 ppm,m,4H; 3,7 ppm,s,3H; 4,2 ppm,m,1H; 5,4 à 6 ppm 1H échangeable, 6,3 à 7,2 ppm,m,3H.

STADE E

Acétamido-2 hydroxy-7 tétrahydro-1,2,3,4 naphtalène

On dissout 6 g du composé obtenu à l'étape précédente dans 80 ml de chloroforme anhydre, puis on ajoute goutte à goutte 5,17 ml de tribromure de bore. On laisse 2 heures en contact à la température ambiante. On traite avec 15 ml d'éthanol anhydre pour entraîner la formation d'un précipité qui après filtration est agité dans 100 ml d'eau. On décante l'eau et lave le précipité restant jusqu'à absence d'ions bromures pour obtenir l'amide pur.

Rendement : 86%
Point de fusion : 199°C
Spectre RMN (DMSO-$d_6$) : 1,1 à 1,2 ppm,m,2H; 1,8 ppm,s,3H; 2,3 à 2,9 ppm,m,4H; 3,5 à 4,2 ppm,m,1H; 6,3 à 6,7 ppm,m,2H; 6,9 ppm,d,1H; 7,2 à 8,5 pmm,2H échangeables.

STADE F

Acétamido-2 [(chloro-2 oxo-1) éthyl]-6 hydroxy-7 tétrahydro-1,2,3,4 naphtalène

5 g du phénol obtenu au stade précédent en suspension dans le chlorure de méthylène sont ajoutés à 58 ml d'une solution molaire de trichlorure de bore dans le chlorure de méthylène. On ajoute ensuite 4,4 g de chloroacétonitrile puis, par portions, 3,69 g de chlorure d'aluminium, tout en maintenant la température aux environs de 0°C. On laisse agiter 4 heures à 0°C puis on abandonne une nuit à la température ambiante et ensuite on hydrolyse avec 15 ml d'eau et 27 ml d'une solution aqueuse d'acide chlorhydrique à 10%. On laisse une heure sous agitation puis on basifie par de l'ammoniaque à 20% dans l'eau. On filtre le précipité obtenu sur celite, on lave au chlorure de méthylène, décante les deux phases et on sèche la phase organique sur sulfate de magnésium anhydre. Après évaporation du solvant, le résidu est recristallisé dans l'éthanol.
Rendement : 52%
Point de fusion : 166°C
Spectre RMN (DMSO-$d_6$) : 1,3 à 2,3 ppm,m,2H; 1,9 ppm,s,3H; 2,4 à 3,2 ppm,m,4H; 3,5 à 4,2 ppm,m,1H; 5,1 ppm,s,2H; 6,8 ppm,s,1H; 7,7 ppm,s,1H; 8 ppm, 1H échangeable; 10,9 ppm 1H échangeable.

STADE G

Acétamido-7 tétrahydro-5,6,7,8 naphto [2,3b] dihydro-2,3 furannone-3

On porte 2 heures à reflux un mélange de 3,5 g du composé obtenu au stade précédent, 9 ml de triéthylamine et 70 ml de chloroforme. On évapore à sec et reprend le résidu par 100 ml d'eau, et on précipite à l'éther le produit attendu. On filtre, on lave plusieurs fois le précipité à l'eau jusqu'à absence d'ions chlorures pour obtenir après séchage l'amide pur.
Rendement : 92%
Point de fusion : 191°C
Spectre RMN ($CDCl_3$ + DMSO-$d_6$) : 1,4 à 2,5 ppm,m,2H; 2 ppm,s,3H; 2,5 à 3,5 ppm,m,4H; 3,9 à 4,6 ppm,m,1H; 4,6 ppm,s,2H; 6,7 ppm, 1H échangeable; 6,9 ppm,s,1H; 7,4 ppm,s,1H.

STADE H

Acétamido-7 hydroxy-3 tétrahydro-5,6,7,8 naphto [2,3b] dihydro-2,3 furanne

2,8 g de la cétone obtenue ci-dessus sont mis en suspension dans 27 ml de méthanol et 13,5 ml d'une solution à 10% de bicarbonate de sodium. On ajoute par portions 0,87 g de borohydrure de sodium en maintenant la température à 18°C. On abandonne le milieu réactionnel une nuit à la température ambiante. On hydrolyse par 300 ml d'eau et 70 ml d'acide chlorhydrique. La phase aqueuse saturée en chlorure de sodium est extraite par du chlorure de méthylène pour donner le produit attendu.
Rendement : 37%
Point de fusion : 254°C
Spectre RMN ($CDCl_3$) : 1,1 à 2,4 ppm,m,2H; 2 ppm,s,3H; 2,5 à 3,3 ppm,m,4H; 3,8 à 4,4 ppm,m,1H; 4,4 ppm,m,2H; 4,6 ppm,1H échangeable; 4,5 à 4,9 ppm,m,1H; 6,5 ppm,s,1H; 6,3 à 7 ppm,1H échangeable; 7,1 ppm,s,1H.

STADE I

Acétamido-7 tétrahydro-5,6,7,8 naphto [2,3b] furanne

1 g du composé obtenu au stade précédent est mis en suspension dans 7,4 ml d'acide chlorhydrique à 10% dans l'eau. Après 2 heures à la température ambiante, on reprend par 100 ml de chlorure de méthylène; la phase organique est lavée avec de l'eau, séchée sur sulfate de magnésium anhydre. Après évaporation du solvant, on obtient l'amide attendu.

Rendement : 81%
Point de fusion : 154°C
Spectre RMN ($CDCl_3$) : 1,5 à 2,5 ppm,m,2H; 2 ppm,s,3H; 2,7 à 3,4 ppm,m,4H; 4,1 à 4,7 ppm,m,1H; 5,4 à 6,1 ppm,1H échangeable; 6,8 ppm,d,1H; 7,3 ppm,s,1H; 7,4 ppm,s,1H; 7,65 ppm,d,1H.

STADE J

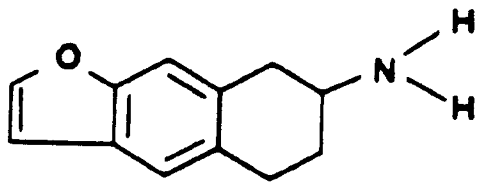

0,55 g du composé précédent est dissout dans un mélange de 10 ml de méthanol, 1 ml d'eau et 0,67 g d'hydroxyde de potassium. On porte à reflux pendant une nuit, puis encore 6 heures, après avoir rajouté 0,2 g d'hydroxyde de potassium dans 2 ml de méthanol et 1 ml d'eau. On évapore le solvant, reprend par du chlorure de méthylène, épuise avec de l'acide chlorhydrique 1N. Les phases acides sont rebasifiées par de l'hydroxyde de sodium 1N et on extrait au chlorure de méthylène. Après évaporation du solvant, on obtient l'amino-7 tétrahydro-5,6,7,8 naphto [2,3b] furanne.
Spectre RMN ($CDCl_3$) : 1,2 à 2,5 ppm,m,2H + 2H échangeables, 2,5 à 3,4 ppm,m,5H; 6,6 ppm,d,1H; 7,2 ppm,s,1H; 7,3 pm,s,1H; 7,5 ppm,d,1H.

Après l'addition de la quantité nécessaire d'éther chlorhydrique, on obtient le chlorhydrate correspondant. Point de fusion : 253-255°C.

**EXEMPLE 22**

**Chlorhydrate de la (d,l) amino-7 tétrahydro-5,6,7,8 naphto [2,3b] dihydro-2,3 furannone-3**

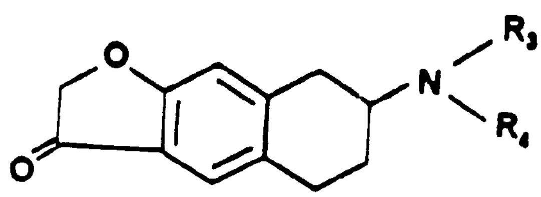

Ce composé a été obtenu à partir de l'acétamido-7 tétrahydro-5,6,7,8 naphto [2,3b] dihydro-2,3 furannone-3 selon le procédé décrit au stade J de l'exemple précédent.
Spectre RMN de la base correspondante (CDCl3 + 1gt DMSO-$d_6$) : 1,25 à 2,65 ppm, 2H échangeables; 1,3 à 2,5,m,2H; 2,3 à 3,3 ppm,m,5H; 4,55 ppm,s,2H; 6,8 ppm,s,1H; 7,3 ppm,s,1H.

**EXEMPLE 23**

**Chlorhydrate de (d,l) amino-7 hydroxy-3 tétrahydro-5,6,7,8 naphto [2,3b] dihydro-2,3 furanne**

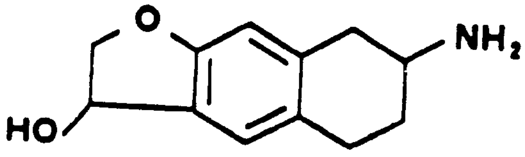

Ce composé a été obtenu à partir de l'acétamido-7 hydroxy-3 tétrahydro-5,6,7,8 naphto [2,3b] dihydro-2,3 furanne selon le procédé décrit au stade J de l'exemple 21.
Spectre RMN de la base correspondante ($CDCl_3$) : 0,95 à 2,3 ppm,m,2H; 1,3 à 2,4 ppm 2H échangeables; 2,4

à 3,45 ppm,m,5H; 4,3 ppm,m,2H; 4,6 ppm 1H échangeable: 4,4 à 4,9 ppm,m,1H; 6,5 ppm,s,1H; 7,2 ppm,s,1H.

**EXEMPLE 24**

**Chlorhydrate de (d,l) [N-(tétrahydro-5,6,7,8 naphto [2,3b] dihydro-2,3 furannyl-7) amino]-6 hexanoate d'éthyle**

Un mélange de 7,7 g du composé de l'exemple 2 de 8,9 g de bromo-6 hexanoate d'éthyle, de 9 g de carbonate de sodium et de 0,1 g d'iodure de potassium dans 50 ml d'éthanol est porté à reflux sous azote pendant 24 heures. Après refroidissement, le milieu est filtré et évaporé sous vide puis repris par l'acide chlorhydrique 1N en présence d'éther. Après filtration et cristallisation dans l'eau 9,5 g de chlorhydrate sont obtenus.
Rendement : 64%
Point de fusion : 166-168°C.

Le spectre RMN de ce composé est indiqué dans le Tableau I.

**EXEMPLE 25**

**Chlorhydrate de l'acide (d,l) [N-(tétrahydro-5,6,7,8 naphto [2,3b] dihydro-2,3 furannyl-7) amino]-6 hexanoïque**

7,9 g du composé de l'exemple 24 sont agités pendant 3 heures à température ambiante en présence de 45 ml d'hydroxyde de sodium 1N et de 45 ml d'éthanol. Après évaporation de l'alcool sous vide, 45 ml d'acide chlorhydrique sont ajoutés. Le chlorhydrate obtenu est filtré, et recristallisé dans l'eau.
Rendement : 46%
Point de fusion : 203-204°C.

Le spectre RMN de ce composé est indiqué dans le Tableau I.

**EXEMPLE 26**

**Chlorhydrate de (d,l) [N-(tétrahydro-5,6,7,8 naphto [2,3b] dihydro-2,3 furannyl-7) amino]-5 méthyl-5 pentanoate d'éthyle**

Un mélange de 7 g du chlorhydrate de l'exemple 2, de 7 g de bromo-5 hexanoate d'éthyle (J.A.C.S.,55,806,1933) de 7 g de carbonate de sodium dans 40 ml d'éthanol est porté à reflux et sous agitation pendant 20 heures. Après évaporation sous vide de l'éthanol, le milieu est repris à l'éther éthylique et extrait à l'acide chlorhydrique 1N. Les phases aqueuses sont ensuite basifiées extraites à l'éther, séchées sur sulfate de sodium anhydre. Après évaporation à siccité, 3,2 g de produit sont obtenus.
Rendement : 31%
Point de fusion : 194°C

Le spectre RMN de ce composé est indiqué dans le Tableau I.

**EXEMPLE 27**

**Acide (d,l) [N-(tétrahydro-5,6,7,8 naphto [2,3b] dihydro-2,3 furannyl-7) amino]-5 pentanoïque**

4,5 g d'ester décrit dans l'exemple 26 sont agités avec 14 ml d'hydroxyde de sodium 1N et la quantité nécessaire d'éthanol pour homogénéiser, pendant une nuit. Après évaporation, on ajoute la quantité stoéchiométrique d'acide chlorhydrique et l'acide cristallisé.
Rendement : 58,5%
Point de fusion : 190-192°C

Le spectre RMN de ce composé est indiqué dans le Tableau I.

**EXEMPLE 28**

**Chlorhydrate de (d,l) [N-(tétrahydro-5,6,7,8 naphto [2,3b] dihydro-2,3 furannyl-7) amino]-6 méthyl-6 hexanoate d'éthyle**

Un mélange de 10 g du composé de l'exemple 2 de 12,5 g du bromo-6 heptanoate d'éthyle

(J.A.C.S.,55,806,1933) de 16,5 g de carbonate de potassium anhydre, de 0,1 g d'iodure de sodium dans 100 ml d'acétonitrile est porté à reflux pendant 24 heures. Après une nouvelle addition de 8,2 g de carbonate de potassium, le reflux est poursuivi pendant 48 heures. Le milieu est ensuite refroidi, filtré et évaporé à siccité et repris à l'éther éthylique en présence d'acide chlorhydrique 1N. 10 g de chlorhydrate sont obtenus.
Rendement : 55%
Point de fusion : 175-180°C
Le spectre RMN du composé est indiqué dans le Tableau I.

**EXEMPLE 29**

**Chlorhydrate d'acide (d,l) [N-(tétrahydro-5,6,7,8 naphto [2,3b] dihydro-2,3 furannyl-7) amino]-6 méthyl-6 hexanoïque**

10 g du composé de l'exemple 28 sont agités pendant 3 heures à température ambiante en présence de 60 ml d'hydroxyde de sodium 1N et de 60 ml d'éthanol. Après évaporation du milieu réactionnel, sont ajoutés 60 ml d'acide chlorhydrique 1N. Le chlorhydrate obtenu après filtration est recristallisé dans l'eau.
Rendement : 42%
Point de fusion : 231-232°C

**EXEMPLE 30**

**Chlorhydrate de (d,l) [N-(tétrahydro-5,6,7,8 naphto [2,3b] dihydro-2,3 furannyl-7) amino]-7 méthyl-7 heptanoate d'éthyle**

Ce composé a été préparé selon le procédé décrit dans l'exemple 28 mais en utilisant le bromo-7 octanoate d'éthyle (J.A.C.S.,55,806,1933) au lieu du bromo-6 heptanoate d'éthyle
Rendement : 33%
Point de fusion : 260°C
Le spectre RMN de ce composé est indiqué dans le Tableau I.

**EXEMPLE 31**

**Chlohydrate de l'acide (d,l) [N-(tétrahydro-5,6,7,8 naphto [2,3b] dihydro-2,3 furannyl-7) amino]-7 méthyl-7 heptanoïque**

Ce composé a été préparé à partir de l'ester obtenu dans l'exemple 30 et selon le procédé décrit dans l'exemple 29.
Rendement : 46%
Point de fusion : 220°C
Le spectre RMN de ce composé est indiqué dans le Tableau I.

**EXEMPLE 32**

**Chlorhydrate de (d,l) [N-(hydroxy-6 hexyl) amino]-7 tétrahydro-5,6,7,8 naphto [2,3b] dihydro-2,3 furanne**

A une suspension de 1 g d'hydrure de lithium et d'aluminium dans 30 ml d'éther éthylique anhydre sont additionnés 8,3 g d'ester éthylique de l'acide N-[tétrahydro-5,6,7,8 naphto [2,3b] dihydro-2,3 furannyl-7] amino-6 hexanoïque dans 80 ml d'éther éthylique anhydre et 30 ml de tétrahydrofuranne. Le milieu réactionnel est porté à reflux pendant 4 heures. Après refroidissement, il est ensuite hydrolysé par 0,7 ml d'eau, 0,55 ml d'hydroxyde de sodium à 20%, puis 2,5 ml d'eau. Le précipité est filtré et le filtrat évaporé a siccité. Le résidu sec est repris par l'isopropanol et une quantité stoéchiométrique d'éther chlorhydrique est ajoutée. Le précipité obtenu est filtré et rincé par quelques millilitres d'éthanol glacé.
Rendement : 31%
Point de fusion : 182-184°C
Le spectre RMN de ce chlohydrate est indiqué dans le Tableau I.

**EXEMPLE 33**

**Chlorhydrate de (d,l) (N-allyl amino)-7 tétrahydro-5,6,7,8 naphto [2,3b] dihydro-2,3 furanne**

5,2 g d'amino-7 tétrahydro-5,6,7,8 naphto [2,3b] dihydro-2,3 furanne en solution dans 27,6 ml de chlorure de méthylène sont agités à température ambiante en présence de 8,3 ml d'hydroxyde de sodium à 40%, de 3,3 g de bromure d'allyle et de 1 ml de Triton B à 40% dans le méthanol. Après 20 heures d'agitation, on rajoute 1 ml de Triton B puis de nouveau 2 ml après 24 heures d'agitation. Après 96 heures d'agitation, le mélange est décanté et évaporé sous vide. Le résidu repris dans l'éther éthylique est lavé à l'eau puis séché sur sulfate de sodium anhydre. Après évaporation, on obtient 4,6 g de base. Le chlorhydrate correspondant est obtenu dans l'acétonitrile par addition d'une quantité stoéchiométrique d'éther chlorhydrique, et ensuite recristallisé dans l'eau.
Rendement : 26%
Point de fusion : > 260°C

Le spectre RMN du chlorhydrate est indiqué dans le Tableau I.

**EXEMPLE 34**

**Chlorhydrate de (d,l) [N-n-propyl) amino]-7 tétrahydro-5,6,7,8 naphto [2,3b] dihydro-2,3 furanne**

STADE A

(d,l) (N-propionyl amino)-7 tétrahydro-5,6,7,8 naphto [2,3b] dihydro-2,3 furanne

A une solution de 5 g du composé de l'exemple 2 dans 100 ml de benzène et 4,2 ml de triéthylamine est ajoutée goutte à goutte une solution de chlorure de propionyle dans 50 ml de benzène. Après 1 heure et demie de reflux, le mélange refroidi est dilué à l'eau, décanté, extrait au benzène et la phase organique est lavée à l'eau, séchée sur sulfate de sodium anhydre et évaporée sous vide. Le résidu sec est recristallisé dans l'acétonitrile.
Rendement : 38%
Point de fusion : 125-126°C
Spectre RMN ($CDCl_3$) : 1,2 ppm,t,3H; 1,5 à 3,4 ppm,m,+q+m +t,2+2+4+2H; 3,8 à 4,7 ppm,m,1H; 4,5 ppm,t,2H; 5,3 à 5,9 ppm, 1H échangeable; 6,5 ppm,s,1H; 6,9 ppm,s,1H.

STADE B

A une suspension de 0,36 g d'hydrure de lithium et d'aluminium dans 25 ml de tétrahydrofuranne est ajouté sous azote 2,3 g du composé obtenu au stade A dans 75 ml de tétrahydrofuranne. Après 5 heures de reflux, une deuxième portion de 0,36 g d'hydrure est ajoutée et le reflux est ensuite poursuivi pendant une nuit. Le milieu réactionnel est alors refroidi, hydrolysé par 0,55 ml d'eau puis par 0,45 ml d'hydroxyde de sodium à 20%, et ensuite par 2 ml d'eau. Les sels d'aluminium sont ensuite filtrés, rincés au tétrahydrofuranne et le filtrat obtenu est ensuite évaporé à siccité. Le résidu sec est repris à l'acide chlohydrique 1N et à l'éther éthylique. Le précipité obtenu est filtré, séché puis recristallisé dans le méthanol.
Rendement : 34%
Point de fusion : > 260°C (sublimation)

Le spectre RMN du chlorhydrate est indiqué dans le Tableau I.

**EXEMPLE 35**

**Chlorhydrate de (d,l) [N-(trifluoro-2,2,2 éthyl) amino]-7 tétrahydro-5,6,7,8 naphto [2,3b] dihydro-2,3 furanne**

STADE A

(d,l) (trifluoroacétyl amino)-7 tétrahydro-5,6,7,8 naphto [2,3b] dihydro-2,3 furanne

Un mélange contenant 5 g du composé de l'exemple 2, 3,35 ml de triéthylamine et 4 ml de trifluoro acétate d'éthyle dans 7 ml de méthanol est agité 4 heures à température ambiante. Le mélange est ensuite évaporé

à siccité et le résidu sec obtenu est recristallisé dans l'éther isopropylique.
Rendement : 50%
Point de fusion : 131°C
Spectre RMN ($CDCl_3$) : 1,5 à 2,3 ppm,m,2H; 2,3 à 3,5 ppm,m,6H; 3,8 à 4,5 ppm,m,1H; 4,5 ppm,t,2H; 5,4 à 7,0 ppm,m,1H; 6,5 ppm,s,1H; 6,95 ppm,s,1H.

STADE B

Ce composé est préparé à partir du composé obtenu au stade précédent et selon le procédé décrit dans l'exemple 34 stade B. Le temps de réaction avec l'hydrure de lithium et d'aluminium est d'environ 2 heures et 30 minutes.
Rendement : 26%
Point de fusion : >260°C
Le spectre RMN du composé est indiqué dans le Tableau I.

**EXEMPLE 36**

**Chlorhydrate de (d,l) [N-n.propyl N-(thiényl-2 éthyl) amino]-7 tétrahydro-5,6,7,8 naphto [2,3b] dihydro-2,3 furanne**

STADE A

(d,l) [N-n.propyl N-(thiényl-2 acétyl) amino]-7 tétrahydro-5,6,7,8 naphto [2,3b] dihydro-2,3 furanne

A une solution de 13 g du composé de l'exemple 34 dans 160 ml de benzène et 10,2 g de triéthylamine est ajoutée goutte à goutte une solution de chlorure de (thiophen-2-yl)-acétyle dans 15 ml de benzène. Après agitation à température ambiante pendant 3 heures, le mélange est dilué à l'eau extrait à l'acétate de sodium anhydre puis évaporé sous vide. Après passage sur une colonne de silice en utilisant comme éluant, un mélange de chlorure de méthylène et d'acétate d'éthyle (95:5), 4 g d'huile sont obtenus.
Rendement : 22%
Spectre RMN ($CDCl_3$) : 0,95 ppm,t,3H; 1,3 à 2,2 ppm,m,4H; 2,7 à 3,5 ppm,m,9H; 4,0 ppm,s,2H; 4,60 ppm,t,2H; 6,55 ppm,s,1H; 7,0 ppm,m,3H; 7,2 à 7,4 ppm,m,1H.

STADE B

Le chlorhydrate attendu est obtenu à partir du composé obtenu au stade A décrit ci-dessus et en utilisant le procédé décrit dans l'exemple 34, stade B.
Rendement : 50%
Le spectre RMN de ce composé est indiqué dans le Tableau I.

**EXEMPLE 37**

**(d,l) (oxo-2 pyrrolidinyl-1)-7 tétrahydro-5,6,7,8 naphto [2,3b] dihydro-2,3 furanne**

STADE A

N-(chloro-4 butyramido)-7 tétrahydro-5,6,7,8 naphto [2,3b] dihydro-2,3 furanne

A un mélange de 5 g du composé de l'exemple 2, de 4,2 g de triéthylamine dans 60 ml de benzène sont ajoutés à température ambiante 4,2 g de chlorure de chloro-4 butyrile. Après une heure à température ambiante, le milieu est dilué à l'eau, filtré et la phase aqueuse est extraite au benzène. Le filtrat et le précipité sont réunis et le benzène est évaporé sous vide. Le résidu sec est concrété dans l'éther.
Rendement : 60%
Point de fusion : 135°C
Spectre RMN ($CDCl_3$) : 0,95 ppm,t,3H; 1,3 à 2,2 ppm,m,4H; 2,7 à 3,5 ppm,m,9H; 4,00 ppm,s,2H; 4,60 ppm,t,2H; 6,55 ppm,s,1H; 7,0 ppm,m,3H; 7,2 à 7,4 ppm,m,1H.

STADE B

Un mélange de 3,3 g du composé décrit ci-dessus est porté au reflux pendant 6 heures avec 0,32 g d'hydrure de sodium à 50% dans le tétrahydrofuranne. Après refroidissement, le milieu est dilué à l'eau, extrait à l'éther éthylique, lavé à l'acide chlorhydrique 1N, puis à l'eau jusqu'à neutralité. La phase organique est évaporée à siccité et le résidu est lavé à l'hexane puis recristallisé dans la méthyl éthyl cétone.
Rendement : 35%
Point de fusion : 131-133°C
Le spectre de ce composé est indiqué dans le Tableau I.

**EXEMPLE 38**

**Chlorhydrate de (d) (N-méthylamino)-7 tétrahydro-5,6,7,8 naptho [2,3b] dihydro-2,3 furanne**

STADE A

Dibenzoyl tartrate de (N-méthyl amino)-7 tétrahydro-5,6,7,8 naphto [2,3b] dihydro-2,3 furanne

Ce sel a été obtenu en faisant réagir le composé de l'exemple 9 avec une quantité demi équimoléculaire de l'acide (1) dibenzoyl tartrique. Après une recristallisation de l'éthanol, une recristallisation du méthanol suivie de 3 recristallisations dans un mélange de méthanol et d'eau (1:1), le sel est obtenu optiquement pur.
Point de fusion : 210-215°C

STADE B

Le dibenzoyl tartrate obtenu au stade précédent est mis en suspension dans l'éther éthylique et basifié par l'hydroxyde de sodium. La phase organique est séparée, séchée sur sulfate de sodium anhydre et évaporée. L'huile obtenue est reprise dans l'acétonitrile et ensuite une quantité stoéchiométrique d'éther chlorhydrique est ajoutée pour obtenir le chlorhydrate.
Point de fusion : 257-260°C
**Pouvoir rotatoire d'une solution à 0.25% dans le DMSO ;**

| λ nm | $[\alpha]_D^{23°C}$ |
|---|---|
| 589 | + 84,6 |
| 578 | + 89,0 |
| 546 | + 102,0 |
| 436 | + 187,0 |
| 365 | + 339,0 |

**EXEMPLE 39**

**Chlorhydrate de (l) (N-méthylamino)-7 tétrahydro-5,6,7,8 naphto [2,3b] dihydro-2,3 furanne**

Ce chlorhydrate a été obtenu selon le procédé décrit ci-dessus mais en utilisant l'acide (d) dibenzoyl tartrique
Point de fusion : 258-260°C
**Pouvoir rotatoire d'une solution à 0.25% dans le DMSO ;**

| λ nm | $[\alpha]_D^{23°C}$ |
|---|---|
| 589 | - 89,2 |
| 578 | - 92,4 |
| 546 | - 106,8 |
| 436 | - 194,0 |
| 365 | - 351,4 |

**EXEMPLE 40**

**Chlorhydrate de (d) (N,N-diméthylamino)-7 tétrahydro-5,6,7,8 naphto [2,3b] dihydro-2,3 furanne**

STADE A

Tartrate de (d) (N,N-diméthylamino)-7 tétrahydro-5,6,7,8 naptho [2,3b] dihydro-2,3 furanne

Ce sel a été obtenu en faisant réagir le composé de l'exemple 6 avec une quantité équimoléculaire de l'acide (l) tartrique. Après 7 recristallisations dans l'eau, le sel est obtenu optiquement pur.
Point de fusion : 95-100°C

**Pouvoir rotatoire d'une solution à 0.25% dans le DMSO ;**

| λ nm | $[\alpha]_D^{23°C}$ |
|---|---|
| 589 | + 52,1 |
| 578 | + 54,6 |
| 546 | + 62,0 |
| 436 | + 110,0 |
| 365 | + 193,1 |

STADE B

Le tartrate obtenu au stade précédent est mis en solution dans l'acétate d'éthyle puis basifié par l'hydroxyde de sodium. La phase organique est séparée, séchée sur sulfate de sodium anhydre et évaporée. L'huile obtenue est reprise dans l'éthanol et ensuite une quantité stoéchiométrique d'éther chlorhydrique est additionnée pour obtenir le chlorhydrate de (d) (N,N-diméthylamino)-7 tétrahydro-5,6,7,8 naphto [2,3b] dihydro-2,3 furanne optiquement pur.
Point de fusion : 226-228°C

**Pouvoir rotatoire d'une solution à 0.25% dans le DMSO ;**

| λ nm | $[\alpha]_D^{23°C}$ |
|---|---|
| 589 | + 90,5 |
| 578 | + 94,9 |
| 546 | + 109,1 |
| 436 | + 200,8 |
| 365 | + 365,6 |

## EXEMPLE 41

**Chlorhydrate de (l) (N,N-diméthylamino)-7 tétrahydro-5,6,7,8 naphto [2,3b] dihydro-2,3 furanne**

Ce chlorhydrate a été obtenu selon le procédé décrit dans l'exemple 40 mais en utilisant au stade A l'acide (d) tartrique

Point de fusion : 226-228°C

**Pouvoir rotatoire d'une solution à 0.25% dans le DMSO ;**

| λ nm | $[\alpha]_D^{23°C}$ |
|---|---|
| 589 | - 91,1 |
| 578 | - 95,9 |
| 546 | - 110,4 |
| 436 | - 201,9 |
| 365 | - 367,1 |

TABLEAU I

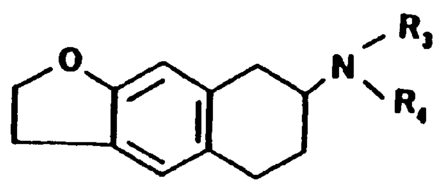

| EX. | $R_3$ | $R_4$ | R M N (solvant) |
|---|---|---|---|
| 2 | H | H | ($CDCl_3$ + DMSO-d6)<br>1,7-3,7 ppm,m,9H; 4,6 ppm,t,2H; 6,5 ppm,m,1H;<br>7 ppm,m,1H; 8,65 ppm,3H échangeables |
| 5 | $-CH_2CH_2CH_3$ | $-CH_2CH_2CH_3$ | ($CDCl_3$)<br>0,8 ppm,t,6H; 1,6 ppm,m,6H; 2 ppm,m,1H; 2,3 à 2,8 ppm,m,8H; 3,1 ppm,t,2H; 4 à 5 ppm,t,2H; 6,5 ppm,s,1H; 6,8 ppm,s,1H |
| 6 | $-CH_3$ | $-CH_3$ | ($CDCl_3$)<br>1,2 à 2,5 ppm,m,2H; 2,3 ppm,s,6H; 2,5 à 3,2 ppm,m,5H; 3,1 ppm,t,2H; 4,5 ppm,t,2H; 6,5 ppm,s,1H; 6,9 ppm,s,1H |

**T A B L E A U I**
**(SUITE 1)**

| EX. | $R_3$ | $R_4$ | R M N (solvant) |
|---|---|---|---|
| 7 | H | $-CH_2$ 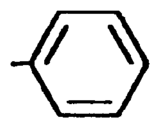 | ($CDCl_3$)<br>1,6 ppm, 1H échangeable, 1,3 à 2,3 ppm,m,2H; 2,5 à 3,2 ppm,m,1H; 3,1 ppm,t,2H; 3,9 ppm,s,2H; 4,5 ppm,t,2H; 6,6 ppm,s,1H; 7,0 ppm,s,1H; 7,4 ppm,s,5H |
| 8 | $-CH_3$ | $-CH_2$ 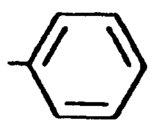 | ($CDCl_3$)<br>1,2 à 2,3 ppm,m,2H; 2,3 ppm,m,3H; 2,5 à 3,1 ppm, m,5H; 3,1 ppm,t,2H; 3,7 ppm,s,2H; 4,5 ppm, t,2H; 6,6 ppm,s,1H; 7,0 ppm,s,1H; 7,4 ppm,m,5H |
| 9 | $-CH_3$ | H | ($CDCl_3$ + DMSO-d6)<br>1,9 à 2,5 ppm,m,2H; 2,5 à 3,9 ppm,m,10H; 4,5 ppm, t,2H; 6,5 ppm,s,1H; 7,0 ppm,s,1H; 9,7 ppm,2H échangeables |

TABLEAU I
(SUITE 2)

| EX. | $R_3$ | $R_4$ | R M N (solvant) |
|---|---|---|---|
| 10 | $-CH_3$ | $-CH_2$ 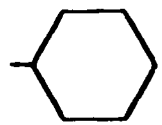 | ($CDCl_3$)<br>1 à 2,5 ppm,m,13H; 2,7 à 3,8 ppm,m,12H; 4,5 ppm, t,2H; 6,5 ppm,s,1H; 6,9 ppm,s,1H |
| 11 | -H | $-CH_2-(CH_2)_5-COO-C_2H_5$ | ($CDCl_3$)<br>1,2 ppm,t,3H; 1,2 à 2,6 ppm,m,12H; 2,6 à 3,6 ppm,m,9H; 4,2 ppm,q,2H; 4,5 ppm,t,2H; 6,5 ppm,s,1H; 7,0 ppm,s,1H; 9,8 ppm,2H échangeables |
| 12 | -H | $-CH_2-(CH_2)_5-COOH$ | (DMSO-$d_6$)<br>1 à 2,1 ppm,m,10H; 2 à 2,5 ppm,m,2H; 2,5 à 3,8 ppm,m,11H (2H échangeables); 4,5 ppm,t,2H; 6,6 ppm,s,1H; 7 ppm, s,1H; 9 à 10,5 ppm,m,1H échangeable |

TABLEAU I
(SUITE 3)

| EX. | $R_3$ | $R_4$ | R M N (solvant) |
|---|---|---|---|
| **13** | -H | $-CH_2CH_2OC_2H_5$ | ($CDCl_3$) 1,2 ppm,t,3H; 1,7 ppm,1H échangeable, 1,4 à 2,5 ppm,m,2H; 2,6 à 3,4 ppm,t+m,2+7H; 3,3 à 3,8 ppm,q+t,4H; 4,55 ppm,t,2H; 6,55 ppm,s,1H; 6,95 ppm,s,1H |
| **14** | -H | $-CH_2CH_2OH$ | ($CDCl_3$) 1,75 ppm 2H échangeables; 1,4 à 2,5 ppm,m,2H; 2,5 à 3,8 ppm,t,2H,m,6H,m,2H,m,1H; 4,5 ppm,t,2H; 6,6 ppm,s,1H; 7 ppm,s,1H |
| **24** | -H | $-CH_2-(CH_2)_4COOC_2H_5$ | (DMSO-$d_6$) 1,21 ppm,t,3H; 1,4 à 2,0 ppm,m,8H; 2,0 à 2,5 ppm,m,5H; 2,5 à 3,4 ppm,m,6H; 4,0 ppm,q,2H; 4,4 ppm,t,2H; 6,45 ppm,s,1H; 6,90 ppm,s,1H; 9,25 ppm 2H échangeables |

TABLEAU I
(SUITE 4)

| EX. | $R_3$ | $R_4$ | R M N (solvant) |
|---|---|---|---|
| **25** | -H | $-CH_2-(CH_2)_4COOH$ | ($DMSO-d_6$)<br>1,2 à 4 ppm,m+t+m+t+m,8+2+6+2+1H; 4,5 ppm,t,2H; 6,6 ppm,s,1H; 7 ppm,s,H; 8 à 11,5 ppm,3H échangeables |
| **26** | -H | $-\underset{CH_3}{CH}-(CH_2)_3COOC_2H_5$ | ($CDCl_3$)<br>1 à 1,3 ppm,t+d,3+3H; 1,4 ppm,1H échangeable; 1,2 à 3 ppm,m+m+m,4+2+6H; 3,1 ppm,t,2H; 3 à 4 ppm, m+t,2+2H; 4 à 4,4 ppm,q,2H; 4,5 ppm,t,2H; 6,6 ppm,s,1H; 7 ppm,s,1H |
| **27** | -H | $-\underset{CH_3}{CH_2}-(CH_2)_3COOH$ | ($D_2O$+DCl)<br>1,4 ppm,d,3H; 1,5 à 4 ppm,t+m,2+2H; 4,6 ppm,t,2H; 6,6 ppm,t+s,2+1H; 7 ppm,s,1H |

TABLEAU I
(SUITE 5)

| EX. | $R_3$ | $R_4$ | R M N (solvant) |
|---|---|---|---|
| 28 | -H | $-CH-(CH_2)_4COOC_2H_5$<br>\|<br>$CH_3$ | ($CDCl_3$)<br>1 à 2 ppm,t+d+t,3+3+8H; 2 à 3,7 ppm,t+m+t+m,2+4+2+2H; 4,1 ppm,q,2H; 4,5 ppm,t,2H; 6,6 ppm,s,1H; 7 ppm,s,1H; 9,3 ppm,2H échangeables |
| 29 | -H | $-CH-(CH_2)_4COOH$<br>\|<br>$CH_3$ | (DMSO-$d_6$)<br>1 à 2 ppm,d+m,3+8H; 2 à 2,5 ppm,m,2H; 2,5 à 3,8 ppm,m,2H; 4,5 ppm,t,2H; 6,6 ppm,s,1H; 7,05 ppm,s,1H; 8 à 11 ppm,3H échangeables |
| 30 | -H | $-CH-(CH_2)_5COOC_2H_5$<br>\|<br>$CH_3$ | (DMSO-$d_6$)<br>1 à 2 ppm,t+d+m,3+3+10H; 2 à 3,7 ppm,t+m+t+m,2+4+2+2H; 4,08 ppm,q,2H; 4,5 ppm,t,2H; 6,5 ppm,s,1H; 7 ppm,s,1H; 9,1 ppm,2H échangeables |

TABLEAU I

(SUITE 6)

| EX. | $R_3$ | $R_4$ | R M N (solvant) |
|---|---|---|---|
| 31 | -H | $-CH(CH_3)-(CH_2)_5COOH$ | (DMSO-$d_6$)<br>1 à 2 ppm,t+d+m,2+3+10H; 2 à 2,5 ppm,m,2H; 2,5 à 3,8 ppm,m+t+m,4+2+2H; 4,5 ppm,t,2H; 6,6 ppm,s,1H; 7,05 ppm,s,1H; 8 à 11 ppm,3H échangeables |
| 32 | -H | $-CH_2-(CH_2)_5OH$ | (DMSO-$d_6$)<br>1 à 2,3 ppm,m,10H; 2,3 à 3,8 ppm,1H échangeable + m+m+t+t+m,2+4+2+2+1H; 4,5 ppm,t,2H; 6,6 ppm,s,1H; 7 ppm,s,1H; 9 à 9,6 ppm,2H échangeables |
| 33 | -H | $-CH_2-CH=CH_2$ | ($D_2O$+DCl)<br>1,4 à 2,5 ppm,m,2H; 2,5 à 3,9 ppm,m+t+m+d, 4+2+1+2H; 4,5 ppm,t,2H; 5 à 6,5 ppm,t+m,2+2H; 6,6 ppm,s,1H; 7,1 ppm,s,1H |

**TABLEAU I**
**(SUITE 7)**

| EX. | $R_3$ | $R_4$ | R M N (solvant) |
|---|---|---|---|
| 34 | -H | $-CH_2CH_2CH_3$ | ($CD_3OD$)<br>1,05 ppm,t,3H; 1,4 à 2,6 ppm,m,4H; 2,6 à 3,9 ppm, m+t+m+m,4H+2H+2H+1H; 4,5 ppm,t,2H; 6,55 ppm,s,1H; 7 ppm,s,1H |
| 35 | -H | $-CH_2CF_3$ | (DMSO)<br>1,5 à 3,7 ppm,m+m+t,2+4+2H; 4,1 ppm,q,2H; 4,5 ppm,t,2H; 6,5 ppm,s,1H; 7 ppm,s,1H; 7 à 12 ppm,2H échangeables |
| 36 | $-CH_2CH_2CH_3$ | $-CH_2CH_2$ 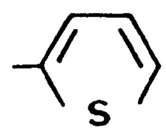 | ($CDCl_3$)<br>0,7 à 2,2 ppm,t+m,3+4H; 2,3 à 3,6 ppm,m+m+m+t, 4+6+1+2H; 4,5 ppm,t,2H; 6,55 ppm,s,1H; 6,7 à 7,4 ppm,s+m,1+3H |

**T A B L E A U   I**
**(SUITE 8)**

| EX. | $R_3$ $R_4$ | R M N (solvant) |
|---|---|---|
| 37 | 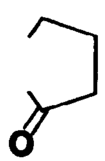 | ($CDCl_3$)<br>1,5 à 2,7 ppm,m+m,4+2H; 2,5 à 3 ppm,m,4H; 3,2 ppm,t,2H; 3,3 ppm,t,2H; 4 à 4,6 ppm,m,1H; 4,5 ppm,t,2H; 6,5 ppm,s,1H; 6,9 ppm,s,1H |

## ETUDE PHARMACOLOGIQUE

## EXEMPLE 42

### Antagonisme du comportement de verticalisation induit par l'apomorphine

Les composés de formule générale I étudiés sur le test d'antagonisme de la verticalisation induite par l'apomorphine à la suite de l'administration d'une dose de 0,75 $mg.kg^{-1}$ de cette substance par voie sous-cutanée chez la souris, agissent de manière nette pour des doses supérieures ou égales à 2,5 $mg.kg^{-1}$ (voie i.p.). Les mêmes résultats sont observés quand les composés de formule générale I sont administrés par voie orale à la dose de 20 $mg.kg^{-1}$. L'activité des composés a été évaluée selon la méthode décrite par Protais P, Costentin J et Schwartz J.C. dans Psychopharmacology (1976),50,p.1-6. Les résultats de cet essai sont rapportés dans les tableaux II et III.
(s = variation significative $p < 0,05$).

TABLEAU II

| COMPOSE | % D'INHIBITION DE LA VERTICALISATION POUR UNE DOSE i.p. ($mg.kg^{-1}$) | | | | |
|---|---|---|---|---|---|
| | 2,5 | 5 | 10 | 20 | 40 |
| Exemple 2 | | - 56s | - 58s | - 91s | - 100s |
| Exemple 3 | | | - 72s | - 79s | |
| Exemple 4 | | | - 24 | - 86s | |
| Exemple 5 | | | | + 27 | - 54s |
| Exemple 6 | - 17 | - 54s | - 94s | - 100s | |
| Exemple 9 | - 46s | - 65s | - 100s | - 100s | |
| Exemple 10 | | | + 11 | + 11 | - 96s |
| Exemple 12 | | | - 17 | - 20 | |
| Exemple 15 | | | | - 9 | 0 |
| Exemple 18 | | | | - 12 | + 6 |
| Exemple 19 | | | | - 57 | - 90s |
| Exemple 21 | | | - 56s | - 84s | |
| Exemple 27 | | | - 46s | - 63s | |
| Exemple 31 | | | | + 5 | + 22s |
| Exemple 32 | | | | - 32 | - 40s |
| Exemple 34 | | | - 23 | - 32 | - 100s |
| Exemple 35 | | | - 30 | - 38 | |
| Exemple 38 | - 76s | - 100s | | | |
| Exemple 40 | - 75s | - 100s | | | |

TABLEAU III

| COMPOSE | % D'INHIBITION DE LA VERTICALISATION POUR UNE DOSE p.o. ($mg.kg^{-1}$) | |
|---|---|---|
| | 10 | 20 |
| 2 | $-77^{s}$ | $-100^{s}$ |

## EXEMPLE 43

**Antagonisme des stéréotypies induites par l'apomorphine ou l'amphétamine**

L'activité inhibitrice des composés de l'invention sur les stéréotypies induites par l'apomorphine ou l'amphétamine chez le rat a été évaluée en utilisant l'échelle de cotation des mouvements stéréotypés décrite par Quinton RM et Halliwell G. dans Nature (1963),200,p.178-179.

L'apomorphine ou l'amphétamine ont été administrées chez les animaux à la dose de 1,5 $mg.kg^{-1}$ par voie intrapéritonéale. Le pourcentage d'inhibition (-) ou de potentialisation (+) du score de stéréotypies (% I.S.S.) indiqué dans les tableaux IV et V pour l'apomorphine correspond au temps de mesure de 30 minutes. Par contre, le pourcentage d'inhibition du score de stéréotypies indiqué dans le tableau VI pour l'amphétamine correspond au temps de mesure de 3 heures.
(s = variation significative $p < 0,05$).

TABLEAU IV

| COMPOSE | % I.S.S. POUR UNE DOSE i.p. mg.kg-1 | | |
|---|---|---|---|
| | 5 | 10 | 20 |
| Exemple 2 | - 16 | - 62s | - 48s |
| Exemple 5 | - 3 | 0 | |
| Exemple 6 | | - 48s | - 58s |
| Exemple 12 | | + 19 | - 3 |
| Exemple 25 | | + 9 | |
| Exemple 27 | | - 5 | - 8 |
| Exemple 29 | | + 11 | |
| Exemple 31 | | + 5 | - 3 |
| Exemple 32 | | - 3 | - 5 |
| Exemple 34 | | - 68s | - 70s |
| Exemple 37 | | - 6 | - 14 |
| Exemple 38 | - 60s | - 54s | |
| Exemple 40 | | - 61s | - 67s |
| Exemple 41 | | + 11 | + 3 |

TABLEAU V

| COMPOSE | % I.S.S. POUR UNE DOSE p.o. mg.kg-1 | |
|---|---|---|
| | 10 | 20 |
| Exemple 2 | - 55s | - 55s |

TABLEAU VI

| COMPOSE | % I.S.S. POUR UNE DOSE i.p. $mg.kg^{-1}$ | |
|---|---|---|
| | 5 | 10 |
| Exemple 2 | + $32^s$ | + $39^s$ |
| Exemple 12 | + 11 | + $14^s$ |

## EXEMPLE 44

**Antagonisme vis à vis de l'hypothermie induite par l'apomorphine**

La recherche d'un antagonisme vis à vis de l'hypothermie induite par l'apomorphine chez la souris a été effectuée selon la méthode décrite par CHERMAT R et PONCELET M dans J. Pharmac. (1983),14,N°1,p.93-97. La dose d'apomorphine administrée par voie sous-cutanée était de 1 $mg.kg^{-1}$. Le tableau VII et VIII indique le pourcentage d'antagonisme de l'hypothermie observé après 2 heures. (s = significative $p < 0,05$).

TABLEAU VII

| COMPOSE | % D'ANTAGONISME D'HYPOTHERMIE POUR UNE DOSE i.p. | | | | |
|---|---|---|---|---|---|
| | 1 | 2,5 | 5 | 10 | 20 |
| Exemple 2 | - 11 | | - 57$^{s}$ | - 54$^{s}$ | - 80$^{s}$ |
| Exemple 3 | | - 72$^{s}$ | - 79$^{s}$ | - 91$^{s}$ | - 86$^{s}$ |
| Exemple 4 | | - 8 | - 3 | - 11 | - 19 |
| Exemple 5 | | | - 20 | - 47$^{s}$ | - 68$^{s}$ |
| Exemple 6 | | + 6 | - 6 | - 25$^{s}$ | - 48$^{s}$ |
| Exemple 9 | | - 39$^{s}$ | - 67$^{s}$ | - 86$^{s}$ | - 84$^{s}$ |
| Exemple 12 | | | | - 4 | - 2 |
| Exemple 27 | | | | - 5 | - 32$^{s}$ |
| Exemple 32 | | | | - 10 | - 13 |
| Exemple 34 | | | | - 7 | - 16 |
| Exemple 35 | | | | - 16 | - 31$^{s}$ |
| Exemple 38 | | - 48$^{s}$ | - 91$^{s}$ | - 112$^{s}$ | - 119$^{s}$ |
| Exemple 40 | | | | - 44$^{s}$ | - 73$^{s}$ |

TABLEAU VIII

| COMPOSE | % D'ANTAGONISME D'HYPOTHERMIE POUR UNE DOSE p.o. | | | |
|---|---|---|---|---|
| | 2,5 | 5 | 10 | 20 |
| Exemple 2 | - 35$^{s}$ | - 59$^{s}$ | - 70$^{s}$ | - 86$^{s}$ |

**EXEMPLE 45**

**Evaluation des effets anti-agressifs**

Les effets anti-agressifs des composés de l'invention ont été recherchés par deux méthodes, l'une recherchant une inhibition des conduites agressives chez la souris préalablement isolée. (Yen C.Y., Stanger A.L., Millman N, Arch. Int. Pharmacodyn. Therap. (1959),123,p.179-185), l'autre chez le rat isolé et bulbectomisé (Garattini S and Sigg E.B. "Agressive Behaviour" p.47-55 Ed. Excerpta Medica Found., Amsterdam, 1969). Les composés de l'invention administrés par voie intrapéritonéale inhibent significativement l'agressivité d'isolement chez la souris et l'agressivité du rat bulbectomisé isolé.
(Tableaux IX et X s = significatif p < 0,05).

TABLEAU IX

| COMPOSE | % DE COUPLES DE SOURIS SANS MANIFESTATION D'ATTAQUE DOSE $mg.kg^{-1}$ | | | | |
|---|---|---|---|---|---|
| | 2,5 | 5 | 10 | 20 | 40 |
| Exemple 2 | 40s | 70s | 100s | 100s | |
| Exemple 3 | 56s | 78s | | | |
| Exemple 4 | 22 | 44 | | | |
| Exemple 5 | | | | 50s | 89s |
| Exemple 6 | 40s | 44 | 78s | | |
| Exemple 9 | 67s | 56s | 89s | | |
| Exemple 10 | | 0 | 11 | 60s | |
| Exemple 25 | | | 20 | | |
| Exemple 32 | | 10 | | 30 | |
| Exemple 38 | | 70s | | | |
| Exemple 39 | 70s | 90s | | | |
| Exemple 40 | | 22 | 50s | | |
| Exemple 41 | | 66s | 90s | | |

TABLEAU X

| COMPOSE | % DES RATS RENDUS NON AGRESSIFS DOSE $mg.kg^{-1}$ | | | |
|---|---|---|---|---|
| | 1,25 | 2,5 | 5 | 10 |
| Exemple 2 | 17 | 50 | $73^{s}$ | 17 |
| Exemple 3 | | $64^{s}$ | | |
| Exemple 4 | | 12 | | |
| Exemple 6 | | 30 | | |
| Exemple 9 | | 27 | $58^{s}$ | |
| Exemple 38 | | 37 | $75^{s}$ | |
| Exemple 39 | | | $58^{s}$ | |
| Exemple 40 | | | | $42^{s}$ |
| Exemple 41 | | | | $50^{s}$ |

**EXEMPLE 46**

**Evaluation des effets antidépresseurs**

Les effets antidépresseurs des composés de l'invention ont été recherchés en évaluant l'antagonisme de l'hypothermie induite par la réserpine et l'antagonisme des ondes ponto-géniculo-occipitales induites par le composé Ro4-1284 chez le chat.

1. Antagonisme de l'hypothermie induite par la réserpine

L'étude est réalisée sur des souris mâles Swiss CD (27-28 g). Après répartition en groupes de 10 animaux chacun, la réserpine est injectée par voie intrapéritonéale à la dose de 2,5 $mg.kg^{-1}$. Trois heures plus tard, les composés soumis à l'essai sont administrés par voie intrapéritonéale. La température rectale des animaux est mesurée une heure et deux heures après le second traitement et comparée à la température initiale To de ces mêmes animaux, mesurée immédiatement avant l'administration des composés soumis à l'essai.

2. Antagonisme des ondes ponto-géniculo-occipitales (P.G.O.) induites par le Ro4-1284

Cette étude a été effectuée selon la méthode de Ruch-Monachon M.A., Jalfre M. et Haefely W. décrite dans Arch.Int.Pharm.Therap.,(1976), 219,N°2,p. 251-346, et elle a permis d'évaluer la dose efficace (i.v.) inhibant de 50% [$DE_{50}$] le nombre des ondes P.G.O..

Les résultats de ces études sont indiqués dans les tableaux XI et XII (s = p < 0,05).

TABLEAU XI

| COMPOSE | % D'INHIBITION DE L'HYPOTHERMIE DOSE i.p. $mg.kg^{-1}$ | | | |
|---|---|---|---|---|
| | 2,5 | 5 | 10 | 20 |
| Exemple 2 | - 62s | - 27 | - 32s | - 47s |
| Exemple 6 | - 1 | + 1 | - 24 | - 20 |
| Exemple 9 | - 8 | + 37 | + 16 | + 11 |
| Exemple 12 | | - 23 | - 45s | - 18 |
| Exemple 27 | | | - 13 | - 21 |
| Exemple 32 | | | - 1 | - 29s |
| Exemple 35 | | | - 20 | - 4 |
| Exemple 38 | - 28 | - 35 | - 21 | |
| Exemple 39 | + 37 | + 14 | + 6 | + 15 |
| Exemple 40 | | | + 14 | + 14 |
| Exemple 41 | | | + 13 | + 29s |

TABLEAU XII

| COMPOSE | $DE_{50}$ ($mg.kg^{-1}$ i.v.) |
|---|---|
| Exemple 2 | 0,146 |
| Exemple 3 | 0,130 |
| Exemple 6 | 0,498 |
| Exemple 9 | 0,173 |
| Exemple 12 | > 2,52 |

**EXEMPLE 47**

**Discrimination de drogue chez le rat**

La propriété dopamino-stimulante des composés de l'invention a été mise en évidence par des expériences de discrimination de drogue chez le rat. Des rats ont été entraînés à discriminer l'effet de stimulus intéroceptif de l'apomorphine (0,2 $mg.kg^{-1}$ i.p.) de celui du sérum physiologique, selon un principe décrit par Colpaert F. dans "Drug discrimination application in C.N.S. Pharmacology" Ed. Elsevier Biomedical 1982, Amsterdam. Après la période d'entrainement, les composés de l'invention ont été administrés aux animaux à la dose de

2,5 mg.kg$^{-1}$ par voie intrapéritonéale, à la place de l'apomorphine, 30 minutes avant le test proprement dit.

Dans les conditions expérimentales réalisées, les animaux présentent un comportement superposable à celui noté en cas d'administration d'apomorphine. Les composés étudiés exercent donc chez le rat un phénomène de généralisation, ce qui permet de conclure qu'ils possèdent une activité dopaminergique de nature agoniste. Les résultats de cette étude sont indiqués dans le tableau XIII.

TABLEAU XIII

| COMPOSE | DOSE mg.kg$^{-1}$ i.p. | NOMBRE D'ANIMAUX PRESENTANT L'EFFET DE GENERALISATION AVEC L'APOMORPHINE | % D'ANIMAUX GENERALISANT |
|---|---|---|---|
| Exemple 2 | 1,0 | 2/10 | 20 |
| Exemple 2 | 2,5 | 7/9 | 77 |
| Exemple 2 | 5,0 | 2/4 | 50 |
| Exemple 3 | 2,5 | 8/9 | 88 |
| Exemple 4 | 2,5 | 7/10 | 70 |

**EXEMPLE 48**

**<u>Gélules dosées à 20 mg de chlorhydrate de (d,l) amino-7 tétrahydro-5,6,7,8 naphto [2,3b] dihydro-2,3 furanne</u>**

Chlorhydrate de (d,l) amino-7 tétrahydro-5,6,7,8 naphto [2,3b] dihydro-2,3 furanne ........ 20 mg

Amidon de maïs ........ 15 mg

Lactose ........ 25 mg

Talc ........ 5 mg

Pour une gélule N° 3.

**Revendications**

1. Composés de formule générale I,

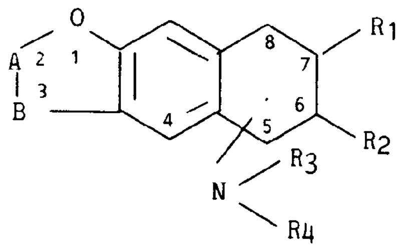

(I)

dans laquelle,

– $R_1$ représente un atome d'hydrogène **ou**, à condition toutefois que le radical amino soit en position 7, un radical alkyle linéaire ou ramifié renfermant 1 à 4 atomes de carbone,

– $R_2$ représente un atome d'hydrogène **ou**, à condition toutefois que le radical amino soit en position 6, un radical alkyle linéaire ou ramifié renfermant 1 à 4 atomes de carbone,

– $R_3$ et $R_4$ identiques ou différents représentent chacun un atome d'hydrogène, un radical benzyle, un radical cyclohexylméthyle, un radical alkyle linéaire ou ramifié renfermant de 1 à 5 atomes de carbone , un radical alkyle linéaire ou ramifié renfermant de 1 à 10 atomes de carbone (éventuellement substitué par un radical hydroxy, par un radical carboxy ou par un radical alcoxy de 1 à 5 atomes de carbone, par un radical alcoxy carbonyle de 2 à 6 atomes de carbone, par un radical alkylphényle de 7 à 16 atomes de carbone ou par un radical alkylthiényle-2 de 12 à 14 atomes de carbone) un radical alkyle halogéné contenant de 1 à 5 atomes de carbone, ou forment ensemble avec l'azote auquel ils sont attachés un radical oxo-2 pyrrolidinyl-1

– A-B représente avec l'oxygène auquel il est attaché un radical

$-CH_2-CH_2-O-$ ,

ou à condition toutefois que le radical amino soit en position 7 et $R_1$ représente un atome d'hydrogène, un radical

-CH=CH-O- ,

un radical

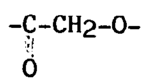

,

ou un radical

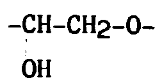

,

à condition toutefois que quand A - B représentent avec l'oxygène auquel il est atttaché un radical -CH2-CH2-O-, le radical amino est en position 7 et $R_1$ et $R_2$ représentent chacun un atome d'hydrogène, $R_3$ et $R_4$ ne représentent jamais simultanément chacun un atome d'hydrogène,

sous forme racémique ou d'isomères optiques,

et leurs sels d'addition à un acide minéral ou organique, pharmaceutiquement acceptable.

2. Composés de formule générale I selon la revendicattion 1 dans laquelle le radical amino est en position 7, leurs isomères optiques et leurs sels d'addition à un acide minéral ou organique pharmaceutiquement acceptable.

3. Composés de formule générale I selon la revendicattion 1 dans laquelle le radical amino est en position 6, leurs isomères optiques et leurs sels d'addition à un acide minéral ou organique pharmaceutiquement acceptable

4. Composés de formule générale I selon la revendicattion 1 dans laquelle $R_3$ et $R_4$ identiques, représentent chacun un atome d'hydrogène et leurs sels d'addition à un acide minéral ou organique pharmaceutiquement acceptable

5. L'amino-6 tétrahydro-5,6,7,8 naphto [2,3b] dihydro-2,3 furanne, sous forme racémique ou d'isomères optiques et ses sels d'addition à un acide minéral ou organique pharmaceutiquement acceptable

6. L'amino-7 tétrahydro-5,6,7,8 naphto [2,3b] furanne, sous forme racémique ou d'isomères optiques et ses sels d'addition à un acide minéral ou organique pharmaceutiquement acceptable.

7. Procédé de préparation des composés de formule générale I selon la revendication 1, caractérisé en ce que :

soit

que l'on condense le dihydro-2,3 benzofuranne de formule II,

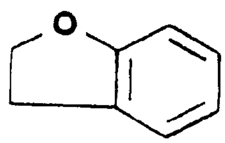

(II)

avec l'anhydride succinique, en présence de chlorure d'aluminium dans un solvant organique chloré et à une température inférieure à 5°C, pour former l'acide (dihydro-2,3 benzofurannyl-5)-4 oxo-4 butanoïque de formule III,

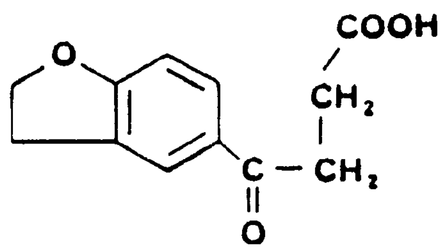

(III)

que l'on réduit en milieu acide à chaud et en présence de zinc et de chlorure mercurique pour former l'acide (dihydro-2,3 benzofurannyl-5)-4 butanoïque de formule IV,

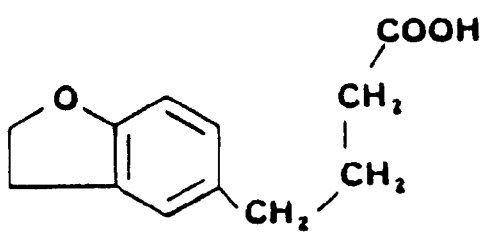

(IV)

lequel ensuite que l'on soumet à l'action de l'acide polyphosphorique dans un solvant organique apolaire et à une température comprise entre 80°C et 100°C pour former la tétrahydro-5,6,7,8 naphto [2,3b] dihydro-2,3 furannone-8 de formule V,

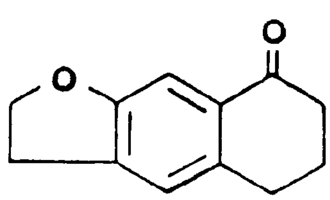

(V)

laquelle ensuite:

– ou que l'on soumet à l'action d'un sel de l'hydroxylamine, à chaud dans un alcool de petit poids moléculaire, pour former la tétrahydro-5,6,7,8 naphto [2,3b] dihydro-2,3 furannone-8 oxime de formule VI,

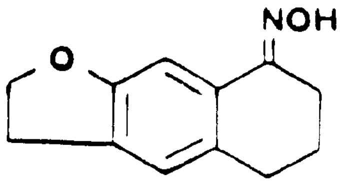

(VI)

laquelle ensuite:

– soit

que l'on condense avec le para-toluènesulfochlorure, dans un milieu organique basique et à une température inférieure à 5°C pour former le para-toluènesulfonate de tétrahydro-5,6,7,8 naphto [2,3b] dihydro-2,3 furannone-8 oxime de formule VII,

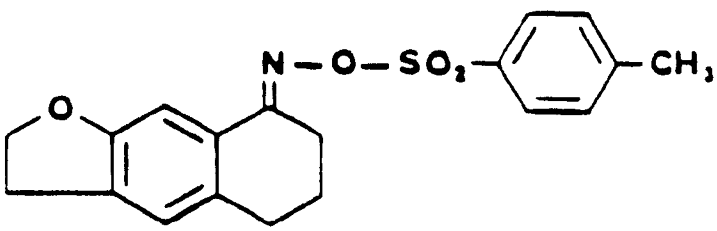

(VII)

que l'on fait agir avec l'éthanolate de sodium dans un solvant organique apolaire anhydre à une température d'environ 0°C pour former l'acétylamino-7 tétrahydro-5,6,7,8 naphto [2,3b] dihydro-2,3 furannone-8 de formule VIII,

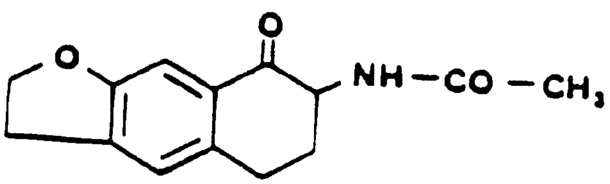

(VIII)

que l'on soumet à une hydrogénation à température ambiante en présence d'un acide et du palladium sur charbon à 5% pour former l'acétylamino-7 tétrahydro-5,6,7,8 naphto [2,3b] dihydro-2,3 furanne de formule IX,

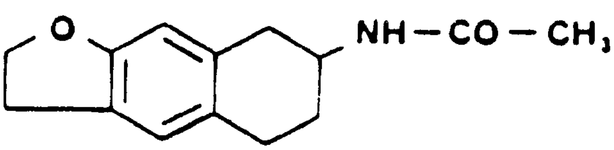

(IX)

lequel ensuite, que l'on transforme par hydrolyse acide à chaud en amino-7 tétrahydro-5,6,7,8 naphto [2,3b] dihydro-2,3 furanne de formule Ia,

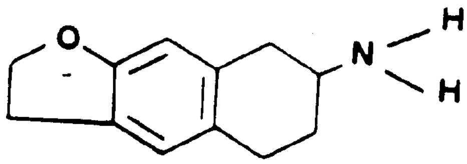

(Ia)

lequel ensuite que l'on peut lcoyler pour former les amines secondaires ou tertiaires correspondantes,

. ou en le condensant avec un composé de formule générale X,

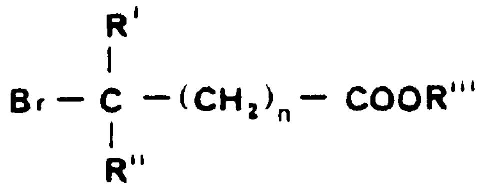

(X)

dans laquelle R′ et R″ identiques ou différents représentent chacun un atome d'hydrogène ou un radical alkyle inférieur de 1 à 4 atomes de carbone, R″ représente un radical alkyle inférieur de 1 à 5 atomes de carbone, et n est un nombre entier de 0 à 9, pour former un composé de formule générale $Ia_1$,

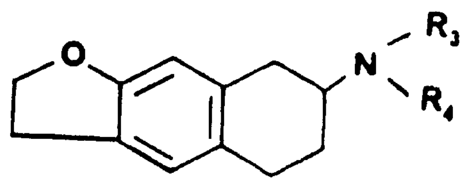

($Ia_1$)

dans laquelle $R_4$ représente un hydrogène et $R_3$ représente un radical alkyle linéaire ou ramifié de 1 à 10 atomes de carbone substitué par un radical alcoxycarbonyle de 2 à 6 atomes de carbone,
lequel ensuite que l'on peut soumettre à l'action d'une base minérale forte pour former un composé de formule générale $Ia_1$,
dans laquelle $R_4$ représente un hydrogène et $R_3$ représente un radical alkyle de 1 à 10 atomes de carbone linéaire ou ramifié substitué par un radical carboxy,
lequel ensuite que l'on peut soumettre à l'action d'un hydrure métallique double pour former un composé de formule générale $Ia_1$,
dans laquelle $R_4$ représente un hydrogène et $R_3$ représente un radical alkyle de 1 à 10 atomes de carbone, linéaire ou ramifié, substitué par un radical hydroxy,
. ou en la faisant réagir avec un chlorure d'acide de formule générale XI,

$W(CH_2)_nCOCl$ (XI)

dans laquelle n est un nombre entirr de 0 à 9 et W représente un radical phényle ou un radical thiényle-2, ou $W(CH_2)_n$ représentent ensemble un radical alkyle halogéné contenant de 1 à 5 atomes de carbone, et ensuite, en réduisant le composé issu de cette réaction par un hydrure métallique double, pour former un composé de formule générale $Ia_1$ dans laquelle $R_3$ représente ou un radical alkyle halogéné contenant de 1 à 5 atomes de carbone ou un radical alkyle de 1 à 10 atomes de carbone substitué par un radical phényle ou un radical thiényle-2,et $R_4$ représente un atome d'hydrogène,
. ou en le faisant réagir avec une quantité adéquate de formaldéhyde et d'acide formique pour obtenir les composés de la formule générale $Ia_1$, dans laquelle $R_3$ et $R_4$ identiques représentent chacun un radical méthyle,
. ou en le faisant réagir avec le chlorure de chloro-4 butyryle, pour former après une condensation à l'aide d'une hydrure métalllque un composé de formule générale $Ia_1$ dans laquelle $R_3$ et $R_4$ forment ensemble avec l'azote auquel ils sont attachés un radical oxo-2 pyrrolidinyl-1,
. ou en le faisant réagir avec un iodure ou chlorure d'alkyle de formule générale $XII_a$ et $XII_b$,

IR (XIIa)
ClR ($XII_b$)

dans laquelle R représente un radical alkyle linéaire ou ramifié renfermant de 1 à 10 atomes de carbone (éventuellement substitué par un radical hydroxy ou par un radical alcoxy de 1 à 5 atomes de carbone) ou un radical cyclohexylméthyle, à chaud dans un solvant organique et en présence d'une base minérale, pour former les composés de formule générale $Ia_1$, dans laquelle $R_3$ et $R_4$ identiques ont la même signification que R,
. ou en le soumettant à l'action du benzaldéhyde, à chaud et en présence d'un alcool de petit poids moléculaire, pour former la benzylimine correspondante, et ensuite, à une hydrogénation catalytique, et puis à l'action de l'acide formique et du formaldéhyde, pour former les composés de formule générale $Ia_1$ dans laquelle $R_3$ représente un radical méthyle et $R_4$ un radical cyclohexylméthyle,
. ou en le soumettant d'abord à l'action du benzaldéhyde en présence d'un solvant aromatique inerte et apolaire et ensuite, après élimination du solvant utilisé, à l'action de borohydrure de sodium, en présence d'un alcool aliphatique polaire de petit poids moléculaire, pour obtenir un composé de formule générale $Ia_1$, dans laquelle $R_3$ représente un hydrogène et $R_4$ un radical benzyle,

lequel ensuite,

que l'on peut soumettre :

. soit à l'action du formaldéhyde et de l'acide formique pour former les composés de formule générale Ia dans laquelle $R_3$ représente un radical méthyle et $R_4$ un radical benzyle,

. soit à l'action d'un iodure d'alkyle de formule générale XII, pour former les composés de formule générale $Ia_1$ dans laquelle $R_3$ a la même signification que R et $R_4$ représente un radical benzyle,

lequel ensuite,

que l'on peut soumettre à une hydrogénation catalytique pour former les composés de formule générale $Ia_1$ dans laquelle $RT_3$ a la signification donnée ci-dessus et $R_4$ représente un atome d'hydrogène,

lequel ensuite,

que l'on peut soumettre à l'action d'un iodure d'alkyle de formule générale XIIa pour former les composés de formule générale $Ia_1$ dans laquelle $R_3$ et $R_4$ identiques ou différent représente chacun un radical alkyle renfermant de 1 à 10 atomes de carbone (éventuellement substitué par un radical hydroxy ou un radical alcoxy de 1 à 5 atomes de carbone) ou un radical cyclohexylméthyle,

– soit

que l'on soumet à une hydrogénation catalytique en présence du palladium sur charbon à 5% à une température ambiante, pour obtenir l'amino-8 tétrahydro-5,6,7,8 naphto [2,3b] dihydro-2,3 furanne de formule $I_b$,

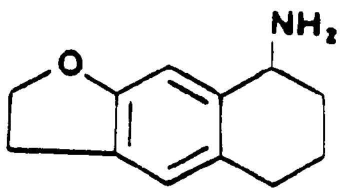

($I_b$)

lequel ensuite que l'on peut alcoyler selon les méthodes décrites ci-dessus pour les composés de formule générale Ia, pour former les amines secondaires ou tertiaires correspondantres de formule générale $Ib_1$,

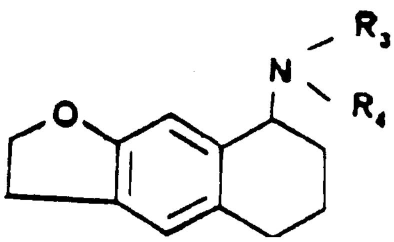

($Ib_1$)

dans laquelle $R_3$ et $R_4$ ont la signification précédemment donnée pour la formule générale I, sauf qu'ils ne représentent jamais simultanément un atome d'hydrogène,

– ou

que l'on soumet à l'action d'un excès de bromure de pyridinium perbromé dans un solvant organique polaire chloré et à température proche de 0°C pour former la dibromo-7,7 tétrahydro-5,6,7,8 naphto [2,3b] dihydro-2,3 furannone-8 de formule XIII,

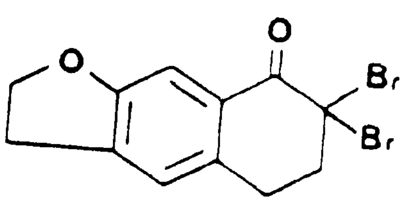

(XIII)

que l'on condense avec un magnésien d'iodure d'alkyle linéaire ou ramifié renfermant 1 à 4 atomes de carbone, en présence de bromure de cuivre, dans un solvant organique anhydre et à une température

comprise entre -20°C et -40°C, pour former las composés de formule générale XIV,

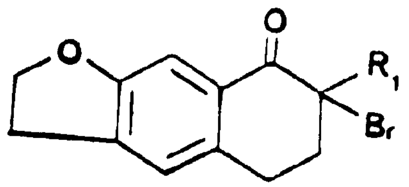

(XIV)

dans laquelle la signification de $R_1$ est celle donnée pour la formule générale I, que l'on fait réagir avec l'azide de sodium dans un milieu organique acide et à une température d'environ 0°C, pour former un dérivé de l'azido-7 alkyl-7 tétrahydro-5,6,7,8 naphto [2,3b] dihydro-2,3 furannone-8 de formule générale XV,

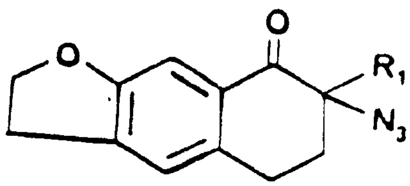

(XV)

dans laquelle $R_1$ a la même signification que précédemment, que l'on réduit ensuite à l'aide de borohydrure de sodium dans un solvant alcoolique anhydre pour former un dérivé de l'azido-7 alkyl-7 hydroxy-8 tétrahydro-5,6,7,8 naphto [2,3b] dihydro-2,3 furanne de formule générale XVI,

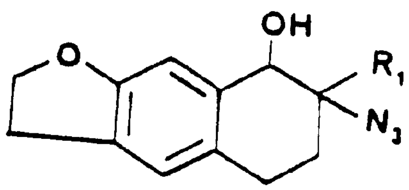

(XVI)

dans laquelle la signification de $R_1$ est identique à celle donnée ci-dessus, que l'on soumet ensuite à l'action du triéthylsilane en présence d'acide trifluoroacétique sous atmosphère inerte pour former un dérivé de l'azido-7 alkyl-7 tétrahydro-5,6,7,8 naphto [2,3b] dihydro-2,3 furanne de formule générale XVII,

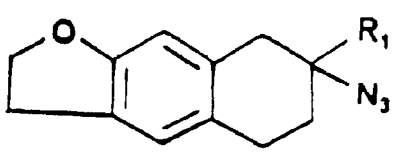

(XVII)

dans laquelle $R_1$ a la même signification que précédemment, et que l'on fait réagir avec l'hydrazine en présence de Nickel de Raney en milieu anhydre pour former les composés de formule générale Ic,

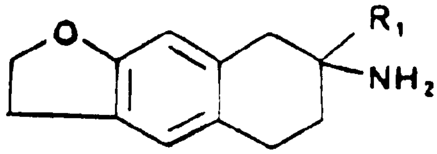

(Ic)

dans laquelle la signification de $R_1$ reste identique à celle mentionnée ci-dessus, lesquels ensuite que l'on peut alcoyler selon les méthodes décrites pour l'alcoylation du composé Ia, pour former les amines secondaires ou tertiaires correspondantes, de formule générale $Ic_1$,

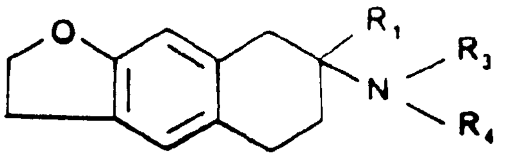

($Ic_1$)

dans laquelle $R_1$ a la signification indiquée ci-dessus, $R_3$ et $R_4$ ont la signification donnée pour la formule générale I, sauf qu'ils ne représentent jamais simultanément un atome d'hydrogène,

– ou

que l'on réduit à l'aide d'un amalgame de zinc métallique et de chlorure mercurique en milieu acide et à chaud pour former le tétrahydro-5,6,7,8 naphto [2,3b] dihydro-2,3 furanne de formule XVIII,

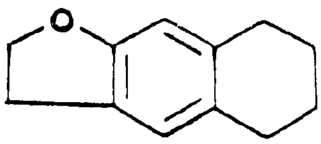

(XVIII)

que l'on oxyde à l'aide de l'anhydride chromique en milieu acide organique et à une température inférieure à 5°C pour former la tétrahydro-5,6,7,8 naphto [2,3b] dihydro-2,3 furannone-5 de formule XIX,

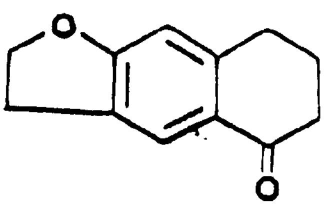

(XIX)

laquelle ensuite,

– soit

que l'on condense avec un sel d'hydroxylamine à une température d'environ 100°C, dans un alcool de petit poids moléculaire, pour former la tétrahydro-5,6,7,8 naphto [2,3b] dihydro-2,3 furannone-5 oxime de formule XX,

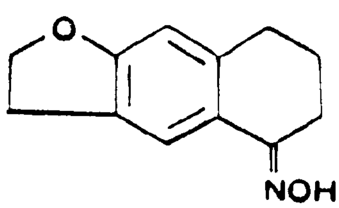

(XX)

laquelle ensuite que l'on soumet à une hydrogénation catalytique et à la température ambiante en présence de palladium sur charbon à 5% pour former l'amino-5 tétrahydro-5,6,7,8 naphto [2,3b] dihydro-2,3 furanne de formule Id,

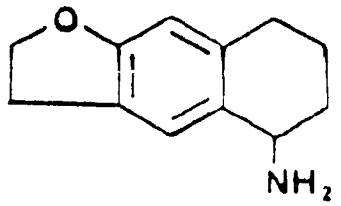

(Id)

lequel ensuite que l'on peut alcoyler méthodes décrites pour l'alcoylation du composé Ia, pour former les amines secondaires ou tertiaires correspondantes de formule générale $Id_1$,

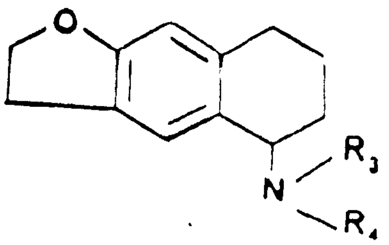

($Id_1$)

dans laquelle $R_3$ et $R_4$ ont la même signification que dans la formule generale I, sauf qu'ils ne représentent jamais simultanément un atome d'hydrogène,
– soit
que l'on soumet à l'action d'une quantité adéquate de bromure de pyridinium perbromé dans un solvant organique polaire pour former un composé de formule générale XXI,

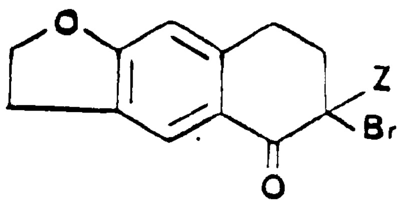

(XXI)

dans laquelle Z représente un atome d'hydrogène quand la quantité de bromure de pyridinium perbromé utilisée est environ équimolaire, ou un atome de brome quand la réaction de bromation a été effectuée avec une quantité au moins double de réactif de bromation,
lequel ensuite,
. que l'on condense, quand il contient en position 6 deux atomes de brome, d'abord avec un magnésien d'iodure d'alkyle linéaire ou ramifié renfermant de 1 à 4 atomes de carbone, en présence de bromure de cuivre, et ensuite que l'on fait réagir avec l'azide de sodium, ou
. que l'on fait réagir directement avec l'azide de sodium, quand Z représente un atome d'hydrogène, pour former un dérivé de l'azido-6 tétrahydro-5,6,7,8 naphto [2,3b] dihydro-2,3 furannone-5 de formule générale XXII,

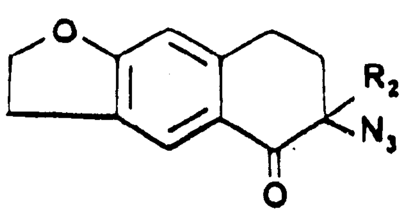

(XXII)

dans laquelle $R_2$ représente un atome d'hydroyène,
que l'on réduit ensuite à l'aide de borohydrure de sodium en présence d'un solvant alcoolique anhydre pour former un dérivé de l'azido-6, hydroxy-5 tétrahydro-5,6,7,8 naphto [2,3b] dihydro-2,3 furanne de formule générale XXIII,

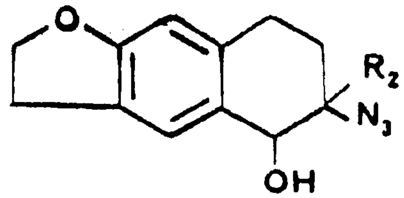

(XXIII)

dans laquelle $R_2$ a la signification indiquée ci-dessus,
que l'on soumet ensuite à l'action du triéthylsilane en présence d'acide trifluoroacétique sous atmosphère inerte pour former un dérivé de l'aziido-6 tétrahydro-5,6,7,8 naphto [2,3b] dihydro-2,3 furanne de formule générale XXIV,

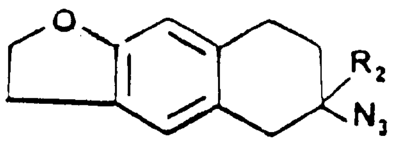

(XXIV)

dans laquelle la signification de $R_2$ reste identique à celle donnée précédemment,
que l'on fait ensuite réagir avec l'hydrazine en présence de Nickel de Raney en milieu anhydre pour former un dérivé de l'amino-6 tétrahydro-5, 6,7,8 naphto [2,3b] dihydro-2,3 furanne de formule générale Ie,

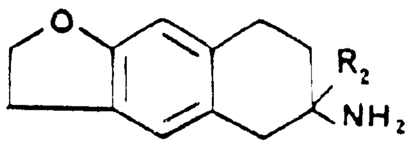

(Ie)

dans laquelle $R_2$ a la signification indiquée ci-dessus,
lequel ensuite que l'on peut alcoyler selon les méthodes décrites précédemment pour l'alcoylation du composé Ia, pour former les amines secondaires ou tertiaires correspondantes de formule générale $Ie_1$,

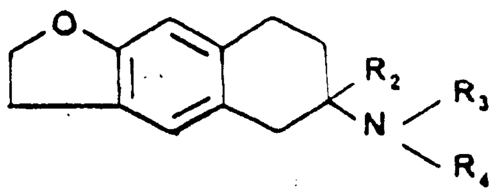

($Ie_1$)

dans laquelle $R_2$, $R_3$ et $R_4$ ont la même signification que dans la formule générale I sauf $R_3$ et $R_4$ ne représentent jamais simultanément un atome d'hydrogène,

soit

que l'on soumet le diméthoxy-2,7 naphtalène de formule XXV,

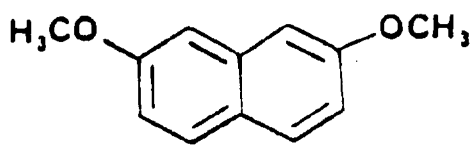

(XXV)

à l'action du sodium métal, à chaud et en présence d'un alcool anhydre, pour former le méthoxy-7 tétrahydro-1,2,3,4 naphtalenone-2 de formule XXVI,

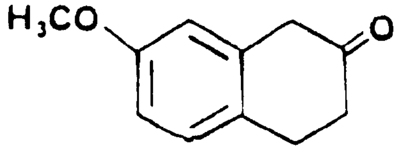

(XXVI)

laquelle ensuite, que l'on l'on fait réagir à chaud dans un solvant alcoolique avec un sel d'hydroxylamine pour former la méthoxy-7 tétrahydro-1,2,3,4 naphtalenone-oxime de formule XXVII,

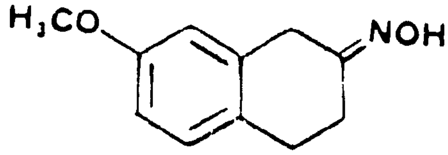

(XXVII)

laquelle que l'on soumet à une hydrogénation catalytique, en solution dans un alcool, en présence de Nickel de Raney et d'ammoniac pour former l'amino-2 méthoxy-7 tétrahydro-1,2,3,4 naphtalène de formule XXVIII,

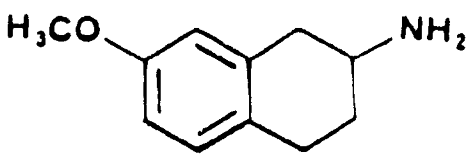

(XXVIII)

que l'on condense avec l'anhydride acétique en milieu acide acétique pour former l'acétamido-2 méthoxy-7 tétrahydro-1,2,3,4 naphtalène de formule XXIX,

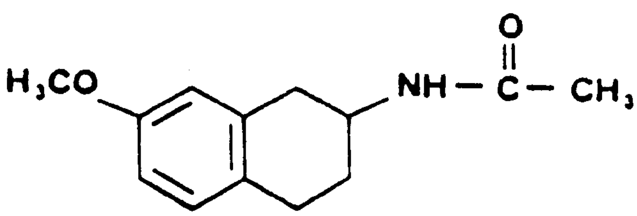

(XXIX)

que l'on soumet à l'action de tribromure de bore, à température ambiante dans un solvant organique halogéné, pour former l'acétamido-2 hydroxy-7 tétrahydro-1,2,3,4 naphtalène de formule XXX,

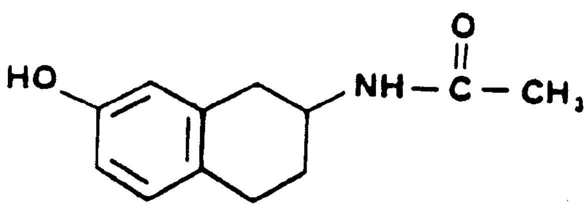

(XXX)

lequel ensuite que l'on fait réagir avec le chloroacétonitrile, en présence de trichlorure de bore et de chlorure d'aluminium, pour former l'acétamido-2 [(chloro-2 oxo-1)éthyl]-6 hydroxy-7 tétrahydro-1,2,3,4 naphtalène de formule XXXI,

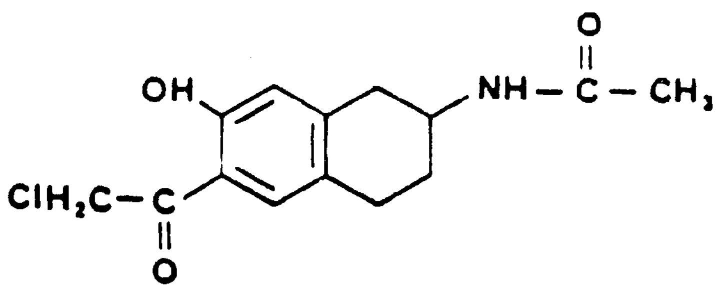

(XXXI)

que l'on soumet à l'action de triéthylamine, à chaud dans un solvant organique halogéné, pour former l'acétamido-7 tétrahydro-5,6,7,8 naphto [2,3b] dihydro-2,3 furannone-3 de formule XXXII,

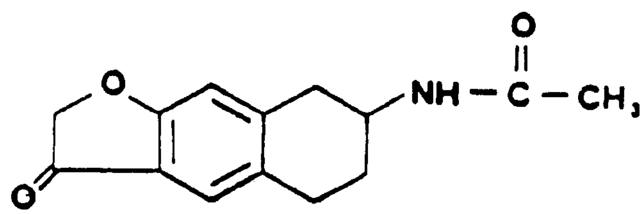

(XXXII)

laquelle ensuite,

– <u>ou</u>

que l'on soumet à l'action d'une base minérale forte pour former la composé de formule If,

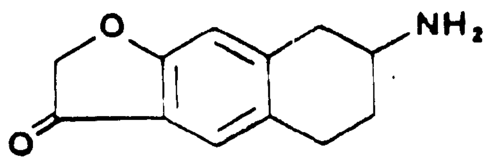

(If)

lequel ensuite que l'on peut alcoyler selon les méthodes décrites ci-dessus pour l'alcoylation du composé Ia pour former les composés de formule générale $If_1$.

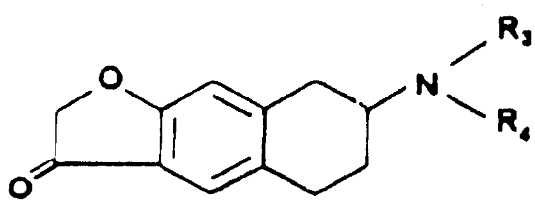

($If_1$)

dans laquelle la définition de $R_3$ et $R_4$ est identique à celle donnée pour la formule générale I, sauf que $R_3$ et $R_4$ ne représentent jamais simultanément un atome d'hydrogène,

– <u>ou</u>

que l'on réduit dans un solvant éthanolique à la température ambiante, par le borohydrure de sodium pour former l'acétamido-7 hydroxy-3 tétrahydro-5,6,7,8 naphto [2,3b] dihydro-2,3 furanne de formule XXXIII,

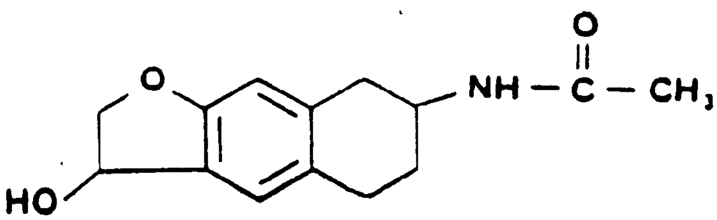

(XXXIII)

lequel ensuite que l'on soumet,

– soit
à l'action d'une base forte pour former le composé de formule Ig,

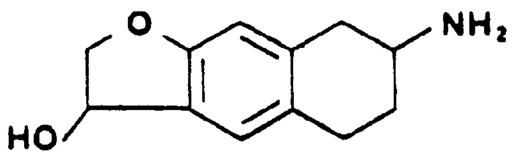

(Ig)

lequel ensuite que l'on peut alcoyler selon les procédés décrits précédemment pour le composé Ia, pour former les composés de formule générale $Ig_1$,

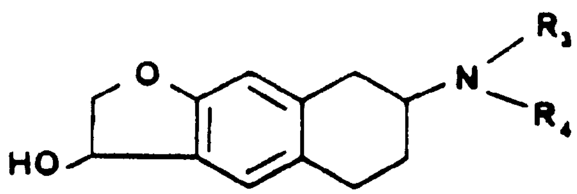

(Ig1)

dans laquelle $R_3$ et $R_4$ ont la même signification que dans la formule générale I, sauf que $R_3$ et $R_4$ ne représentent jamais simultanément un atome d'hydrogène,
– soit
à l'action d'un acide minéral fort à température ambiante, pour former l'acétamido-7 tétrahydro-5,6,7,8 naphto [2,3b] furanne de formule XXXIV,

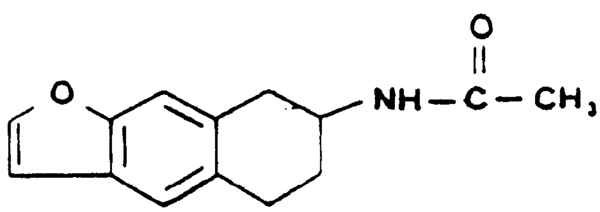

(XXXIV)

lequel ensuite que l'on soumet à l'action d'une base minérale forte en milieu alcoolique pour former le composé de formule Ih,

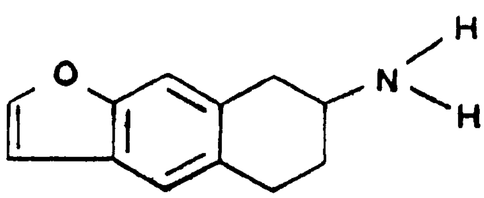

(Ih)

lequel ensuite que l'on peut soumettre à une alcoylation selon les méthodes décrites ci-dessus pour les amines primaires pour former les composés de fomule $Ih_1$,

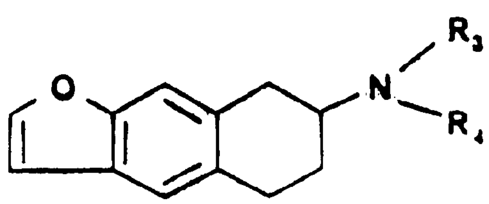

(Ih1)

dans laquelle la signification de $R_3$ et $R_4$ est identique à celle de la formule générale I, sauf qu'ils ne représentent jamais simultanément un atome d'hydrogène.

et que l'on salifie ensuite, si l'on désire, les composés de formules Ia à Ih et $Ia_1$ et $Ih_1$, qui forment l'ensemble des composés de formule I, avec un acide organique ou minéral pharmaceutiquement acceptable,
ou
que l'on sépare d'abord en leurs isomères optiques et ensuite que l'on salifie.

8. Composition pharmaceutique contenant comme principe actif un composé selon l'une quelconque des revendications 1 à 6 en association ou en mélange avec un excipient ou un véhicule inerte non toxique pharmaceutiquement acceptable.

9. Composition pharmaceutique selon la revendication 8 renfermant le principe actif à la dose de 0,5 à 100 mg.

10. Composition pharmaceutique selon les revendications 8 et 9 renfermant comme principe actif au moins un composé selon les revendications 1 à 6 utilisable dans le traitement des maladies nécessitant des médications antidépressives antiagressives ou des modulateurs dopaminergiques.

11. La tétrahydro-5,6,7,8 naphto [2,3b] dihydro-2,3 furannone-5 utile comme intérmédiaire à la préparation des composés de formule générale I selon la revendication 1.

## Claims

1. Compounds of the general formula I

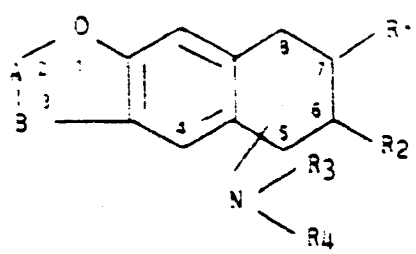

(I)

in which

– $R_1$ represents a hydrogen atom or, provided that the amino group is in the 7-position, may also represent a straight-chain or branched alkyl radical having from 1 to 4 carbon atoms,
– $R_2$ represents a hydrogen atom or, provided that the amino group is in the 6-position, may also represent a straight-chain or branched alkyl radical having from 1 to 4 carbon atoms,
– $R_3$ and $R_4$ are the same or different and each represents a hydrogen atom, a benzyl radical, a cyclohexylmethyl radical, a straight-chain or branched alkyl radical having from 1 to 5 carbon atoms, a straight-chain or branched alkyl radical having from 1 to 10 carbon atoms (optionally substituted by a hydroxy radical, by a carboxy radical or by an alkoxy radical having from 1 to 5 carbon atoms, by an alkoxycarbonyl radical having from 2 to 6 carbon atoms, by a-alkylphenyl radical having from 7 to 16 carbon atoms or by a (2-thienyl)alkyl radical having from 12 to 14 carbon atoms), or a halogenated alkyl radical having from 1 to 5 carbon atoms or, $R_3$ and $R_4$, together with the nitrogen atom to which they are attached, form a 2-oxo-1-pyrrolidinyl radical,
– A-B, together with the oxygen atom to which it is attached, represents a

$$-CH_2-CH_2-O-$$

radical or, provided that the amino group is in the 7-position and $R_1$ represents a hydrogen atom, may also represent
a

-CH=CH-O- radical,

a

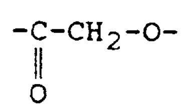

radical

or a

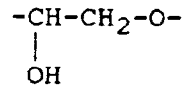

radical,
with the proviso, however, that when A-B, together with the oxygen atom to which it is attached, represents a $-CH_2-CH_2-O-$ radical, the amino group is in the 7-position and each of $R_1$ and $R_2$ represents a hydrogen atom, $R_3$ and $R_4$ never simultaneously each represent a hydrogen atom,
in racemic form or in the form of optical isomers,
and their addition salts with a pharmaceutically acceptable mineral or organic acid.

2. Compounds of the general formula I according to claim 1 in which the amino group is in the 7-position, their optical isomers and their addition salts with a pharmaceutically acceptable mineral or organic acid.

3. Compounds of the general formula I according to claim 1 in which the amino group is in the 6-position, their optical isomers and their addition salts with a pharmaceutically acceptable mineral or organic acid.

4. Compounds of the general formula I according to claim 1 in which $R_3$ and $R_4$ are identical and each represents a hydrogen atom, and their addition salts with a pharmaceutically acceptable mineral or organic acid.

5. 6-Amino-5,6,7,8-tetrahydronaphtho[2,3-b]2,3-dihydrofuran, in racemic form or in the form of optical isomers, and its addition salts with a pharmaceutically acceptable mineral or organic acid.

6. 7-Amino-5,6,7,8-tetrahydronaphtho[2,3-b]furan, in racemic form or in the form of optical isomers, and its addition salts with a pharmaceutically acceptable mineral or organic acid.

7. Process for the preparation of compounds of the general formula I according to claim 1, characterised in that:

either

2,3-dihydrobenzofuran of formula II

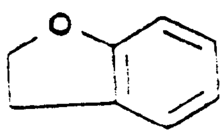

(II)

is condensed with succinic anhydride in the presence of aluminium chloride, in a chlorinated organic solvent and at a temperature below 5°C, to form 4-(2,3-dihydrobenzofuran-5-yl)-4-oxobutanoic acid of formula III

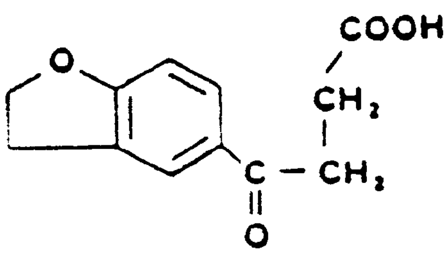

(III)

which is reduced in acid medium, with the application of heat and in the presence of zinc and mercuric chloride, to form 4-(2,3-dihydrobenzofuran-5-yl)-butanoic acid of formula IV

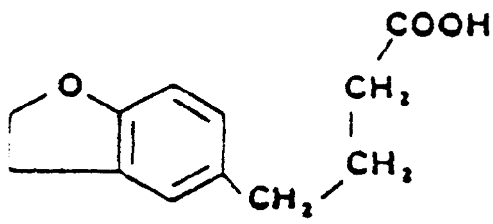

(IV)

which is then subjected to the action of polyphosphoric acid in a non-polar organic solvent, at a temperature of between 80°C and 100°C, to form 5,6,7,8-tetrahydronaphtho[2,3-b]2,3-dihydrofuran-8-one of formula V

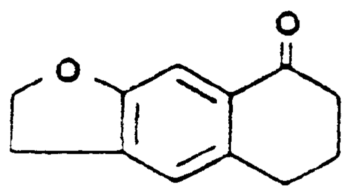

(V)

which is then:

- either subjected to the action of a hydroxylamine salt with the application of heat, in a low molecular weight alcohol, to form 5,6,7,8-tetrahydronaphtho[2,3-b]2,3-dihydrofuran-8-one oxime of formula VI

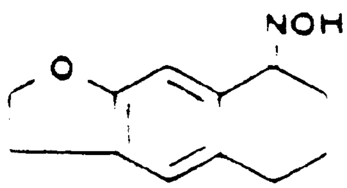

(VI)

which is then:

- either

condensed with para-toluenesulphochloride in a basic organic medium, at a temperature below 5°C, to form 5,6,7,8-tetrahydronaphtho[2,3-b]2,3-dihydrofuran-8-one oxime para-toluenesulphonate of formula VII

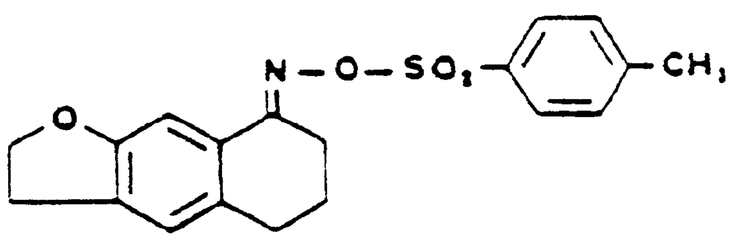

(VII)

which is reacted with sodium ethanolate in an anhydrous non-polar organic solvent, at a temperature of approximately 0°C, to form 7-acetylamino-5,6,7,8-tetrahydronaphtho[2,3-b]2,3-dihydrofuran-8-one of formula VIII

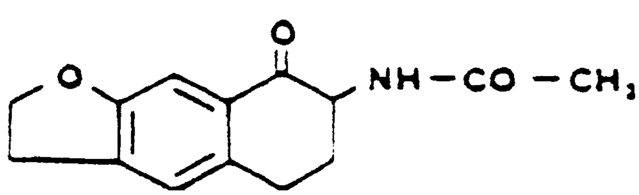

(VIII)

which is subjected to hydrogenation at room temperature, in the presence of an acid and 5 % palladium-on-carbon, to form 7-acetylamino-5,6,7,8-tetrahydronaphtho[2,3-b]2,3-dihydrofuran of formula IX

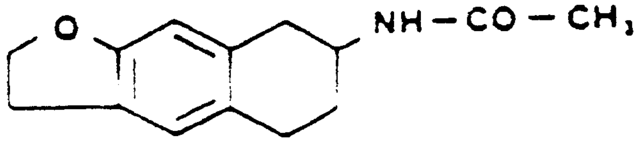

(IX)

which is then converted by acid hydrolysis, with the application of heat, into 7-amino-5,6,7,8-tetrahydronaphtho[2,3-b]2,3-dihydrofuran of formula Ia

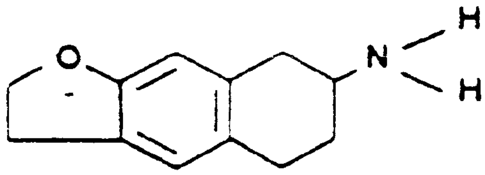

(IA)

which then may be alkylated to form the corresponding secondary or tertiary amines,

· either by condensing it with a compound of the general formula X

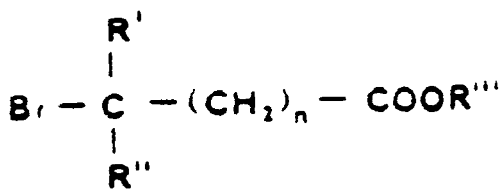

(X)

in which R′ and R″ are the same or different and each represents a hydrogen atom or a lower alkyl radical having from 1 to 4 carbon atoms, R‴ represents a lower alkyl radical having from 1 to 5 carbon atoms, and n is an integer from 0 to 9, to form a compound of the general formula $Ia_1$

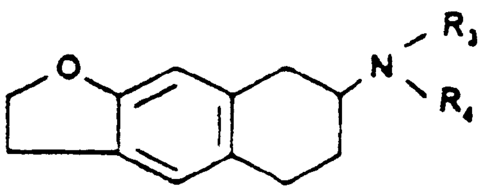

($Ia_1$)

in which $R_4$ represents a hydrogen atom and $R_3$ represents a straight-chain or branched alkyl radical having from 1 to 10 carbon atoms that is substituted by an alkoxycarbonyl radical having from 2 to 6 carbon atoms, which may then be subjected to the action of a strong mineral base to form a compound of the general formula $Ia_1$ in which $R_4$ represents a hydrogen atom and $R_3$ represents a straight-chain or branched alkyl radical having from 1 to 10 carbon atoms that is substituted by a carboxy radical, which may then be subjected to the action of a double metal hydride to form a compound of the general formula $Ia_1$ in which $R_4$ represents a hydrogen atom and $R_3$ represents a straight-chain or branched alkyl radical having from 1 to 10 carbon atoms that is substituted by a hydroxy radical,

· or by reacting it with an acid chloride of the general formula XI

$$W(CH_2)_nCOCl \quad (XI)$$

in which n is an integer from 0 to 9 and W represents a phenyl radical or a 2-thienyl radical, or $W(CH_2)_n$ together represent a halogenated alkyl radical containing from 1 to 5 carbon atoms, and then reducing the compound resulting from that reaction with a double metal hydride, to form a compound of the general formula $Ia_1$ in which $R_3$ represents a halogenated alkyl radical containing from 1 to 5 carbon atoms or an alkyl radical containing from 1 to 10 carbon atoms that is substituted by a phenyl radical or by a 2-thienyl radical, and $R_4$ represents a hydrogen atom,

· or by reacting it with an appropriate amount of formaldehyde and formic acid to obtain compounds of

the general formula $Ia_1$ in which $R_3$ and $R_4$ are the same and each represents a methyl radical,

· or by reacting it with 4-chlorobutyryl chloride to form, after condensation with a metal hydride, a compound of the general formula $Ia_1$ in which $R_3$ and $R_4$, together with the nitrogen atom to which they are attached, form a 2-oxo-1-pyrrolidinyl radical,

· or by reacting it with an alkyl chloride or iodide of the general formula $XII_a$ or $XII_b$

IR ($XII_a$)

CIR ($XII_b$)

in which R represents a straight-chain or branched alkyl radical having from 1 to 10 carbon atoms (optionally substituted by a hydroxy radical or by an alkoxy radical having from 1 to 5 carbon atoms) or a cyclohexylmethyl radical, with the application of heat, in an organic solvent and in the presence of a mineral base, to form compounds of the general formula $Ia_1$ in which $R_3$ and $R_4$ are the same and have the same meaning as R,

· or by subjecting it to the action of benzaldehyde, with the application of heat and in the presence of a low molecular weight alcohol, to form the corresponding benzylimine, and then to catalytic hydrogenation, and then to the action of formic acid and formaldehyde, to form compounds of the general formula $Ia_1$ in which $R_3$ represents a methyl radical and $R_4$ represents a cyclohexylmethyl radical,

· or by subjecting it first to the action of benzaldehyde in the presence of an inert non-polar aromatic solvent and then, after the removal of the solvent employed, to the action of sodium borohydride in the presence of a low molecular weight polar aliphatic alcohol, to obtain a compound of the general formula $Ia_1$ in which $R_3$ represents a hydrogen atom and $R_4$ represents a benzyl radical,

which then

may be subjected:

· either to the action of formaldehyde and formic acid to form compounds of the general formula Ia in which $R_3$ represents a methyl radical and $R_4$ represents a benzyl radical,

· or to the action of an alkyl iodide of the general formula XII to form compounds of the general formula $Ia_1$ in which $R_3$ has the same meaning as R and $R_4$ represents a benzyl radical,

which then

may be subjected to catalytic hydrogenation to form compounds of the general formula $Ia_1$ in which $R_3$ has the meaning given above and $R_4$ represents a hydrogen atom,

which then

may be subjected to the action of an alkyl iodide of the general formula $XII_a$ to form compounds of the general formula $Ia_1$ in which $R_3$ and $R_4$ are the same or different and each represents an alkyl radical having from 1 to 10 carbon atoms (optionally substituted by a hydroxy radical or an alkoxy radical having from 1 to 5 carbon atoms) or a cyclohexylmethyl radical,

– or

subjected to catalytic hydrogenation in the presence of 5 % palladium-on-carbon at room temperature to obtain 8-amino-5,6,7,8-tetrahydronaphtho[2,3-b]2,3-dihydrofuran of formula $I_b$

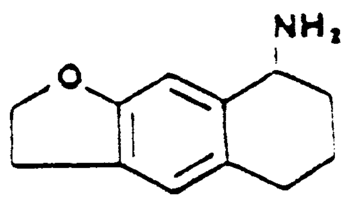

($I_b$)

which may then be alkylated, in accordance with the methods described above for compounds of the general formula Ia, to form the corresponding secondary or tertiary amines of the general formula $Ib_1$

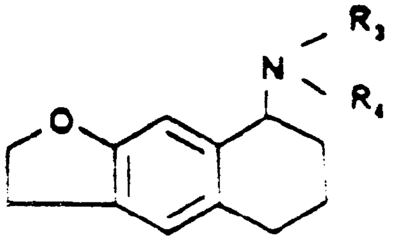

($Ib_1$)

in which $R_3$ and $R_4$ are as defined hereinbefore for the general formula I, except that they never simultaneously represent a hydrogen atom,

– or

subjected to the action of an excess of perbrominated pyridinium bromide in a chlorinated polar organic solvent, at a temperature close to 0°C, to form 7,7-dibromo-5,6,7,8-tetrahydronaphtho[2,3-b]2,3-dihydrofuran-8-one of formula XIII

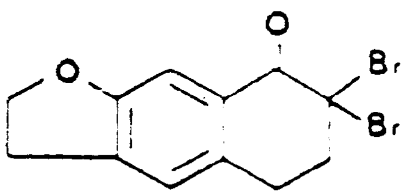

(XIII)

which is then condensed with an alkylmagnesium iodide in which the alkyl radical is straight-chain or branched and contains from 1 to 4 carbon atoms, in the presence of copper bromide, in an anhydrous organic solvent and at a temperature of between -20°C and -40°C, to form compounds of the general formula XIV

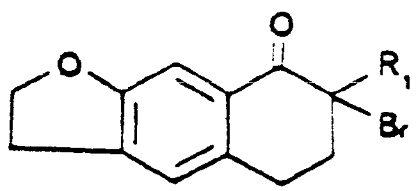

(XIV)

in which $R_1$ is as defined for the general formula I,

which is reacted with sodium azide in an acid organic medium, at a temperature of approximately 0°C, to form a 7-azido-7-alkyl-5,6,7,8-tetrahydronaphtho[2,3-b]2,3-dihydrofuran-8-one derivative of the general formula XV

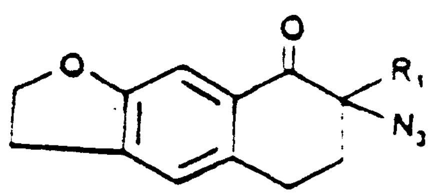

(XV)

in which $R_1$ is as defined above,

which is then reduced with sodium borohydride in an anhydrous alcoholic solvent to form a 7-azido-7-alkyl-8-hydroxy-5,6,7,8-tetrahydronaphtho[2,3-b]2,3-dihydrofuran derivative of the general formula XVI

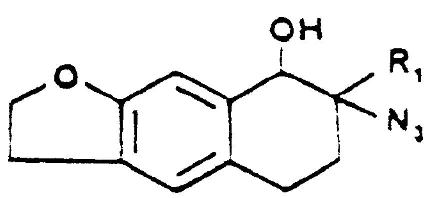

(XVI)

in which the meaning of $R_1$ is identical to that given above,

which is then subjected to the action of triethylsilane in the presence of trifluoroacetic acid, in an inert atmosphere, to form a 7-azido-7-alkyl-5,6,7,8-tetrahydronaphtho[2,3-b]2,3-dihydrofuran derivative of the general formula XVII

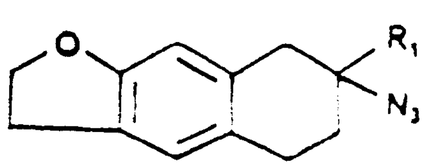

(XVII)

in which $R_1$ is as defined above,
which is reacted with hydrazine in the presence of Raney nickel, in anhydrous medium, to form compounds of the general formula Ic

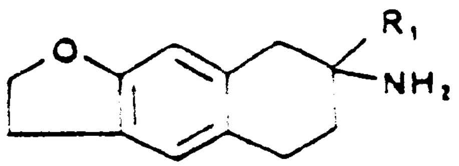

(Ic)

in which the meaning of $R_1$ is identical to that mentioned above,
which may then be alkylated, in accordance with the methods described for the alkylation of compound Ia, to form the corresponding secondary or tertiary amines of the general formula $Ic_1$

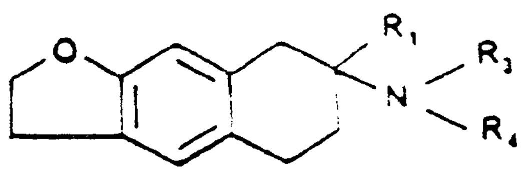

($Ic_1$)

in which $R_1$ has the meaning given above, and $R_3$ and $R_4$ are as defined for the general formula I, except that they never simultaneously represent a hydrogen atom,
– or
reduced with an amalgam of zinc metal and mercuric chloride, in acid medium and with the application of heat, to form 5,6,7,8-tetrahydronaphtho-[2,3-b]2,3-dihydrofuran of formula XVIII

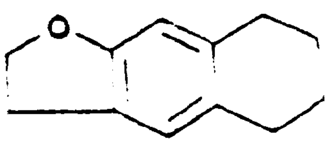

(XVIII)

which is oxidised with chromic anhydride in organic acid medium, at a temperature below 5°C, to form 5,6,7,8-tetrahydronaphtho[2,3-b]2,3-dihydrofuran-5-one of formula XIX

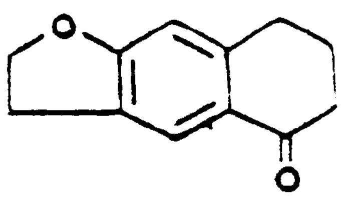

(XIX)

which is then
– either
condensed with a hydroxylamine salt at a temperature of approximately 100°C, in a low molecular weight alcohol, to form 5,6,7,8-tetrahydronaphtho[2,3-b]2,3-dihydrofuran-5-one oxime of formula XX

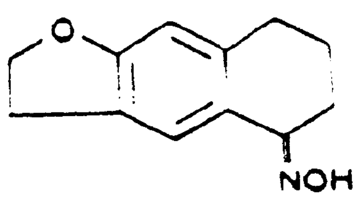

(XX)

which is then subjected to catalytic hydrogenation at room temperature, in the presence of 5 % palladium-on-carbon, to form 5-amino-5,6,7,8-tetrahydronaphtho[2,3-b]2,3-dihydrofuran of formula Id

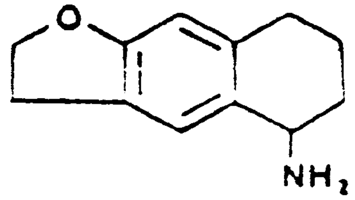

(Id)

which may then be alkylated, in accordance with the methods described for the alkylation of compound Ia, to form the corresponding secondary or tertiary amines of the general formula $Id_1$

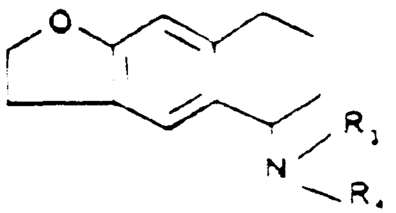

($Id_1$)

in which $R_3$ and $R_4$ are as defined for the general formula I, except that they never simultaneously represent a hydrogen atom,

– or

is subjected to the action of an appropriate amount of perbrominated pyridinium bromide, in a polar organic solvent, to form a compound of the general formula XXI

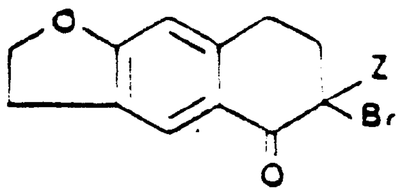

(XXI)

in which Z represents a hydrogen atom when the amount of perbrominated pyridinium bromide used is approximately equimolar, or a bromine atom when the bromination has been carried out with at least double the amount of bromination reagent,

which then

· is condensed, when it contains two bromine atoms in the 6-position, with an alkylmagnesium iodide in which the alkyl radical is straight-chain or branched and contains from 1 to 4 carbon atoms, in the presence of copper bromide, and then reacted with sodium azide, or

· is reacted directly with sodium azide when Z represents a hydrogen atom,

to form a 6-azido-5,6,7,8-tetrahydronaphtho[2,3-b]2,3-dihydrofuran-5-one derivative of the general formula XXII

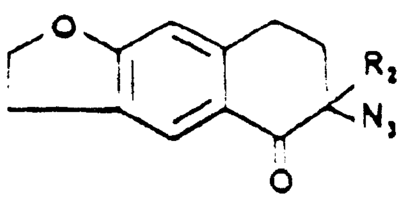

(XXII)

in which $R_2$ represents a hydrogen atom,

which is then reduced with sodium borohydride, in the presence of an anhydrous alcoholic solvent, to form a 6-azido-5-hydroxy-5,6,7,8-tetrahydronaphtho[2,3-b]2,3-dihydrofuran derivative of the general formula XXIII

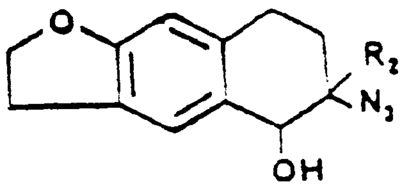

(XXIII)

in which $R_2$ has the meaning given above, which is then subjected to the action of triethylsilane in the presence of trifluoroacetic acid, in an inert atmosphere, to form a 6-azido-5,6,7,8-tetrahydronaphtho[2,3-b]2,3-dihydrofuran derivative of the general formula XXIV

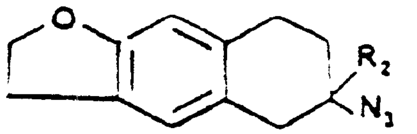

(XXIV)

in which the meaning of $R_2$ is identical to that given above, which is then reacted with hydrazine in the presence of Raney nickel, in anhydrous medium, to form a 6-amino-5,6,7,8-tetrahydronaphtho[2,3-b]2,3-dihydrofuran derivative of the general formula Ie

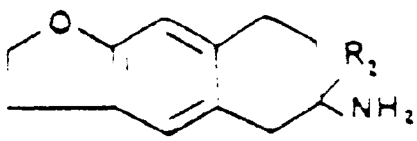

(Ie)

in which $R_2$ has the meaning given above, which may then be alkylated, in accordance with the methods described above for the alkylation of compound Ia, to form the corresponding secondary or tertiary amines of the general formula $Ie_1$

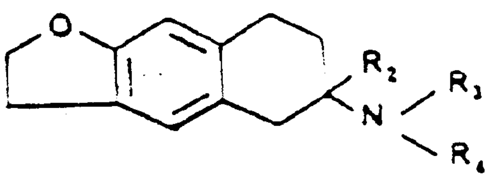

($Ie_1$)

in which $R_2$, $R_3$ and $R_4$ are as defined for the general formula I, except that $R_3$ and $R_4$ never simultaneously represent a hydrogen atom,

or

2,7-dimethoxynaphthalene of formula XXV

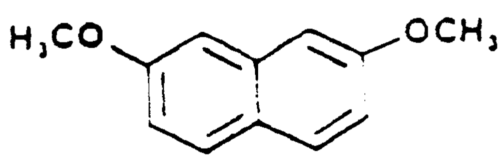

(XXV)

is subjected to the action of sodium metal, with the application of heat and in the presence of an anhydrous alcohol, to form 7-methoxy-1,2,3,4-tetrahydronaphthalen-2-one of formula XXVI

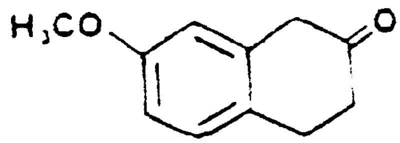

(XXVI)

which is then reacted with the application of heat, in an alcoholic solvent, with a hydroxylamine salt to form 7-methoxy-1,2,3,4-tetrahydronaphthalen-2-one oxime of formula XXVII

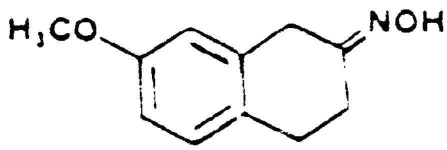

(XXVII)

which is subjected to catalytic hydrogenation, in solution in an alcohol, in the presence of Raney nickel and ammonia, to form 2-amino-7-methoxy-1,2,3,4-tetrahydronaphthalene of formula XXVIII

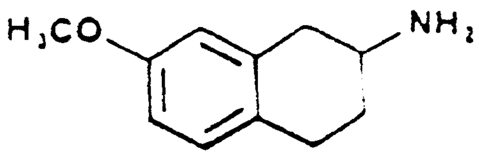

(XXVIII)

which is condensed with acetic anhydride in acetic acid medium to form 2-acetamido-7-methoxy-1,2,3,4-tetrahydronaphthalene of formula XXIX

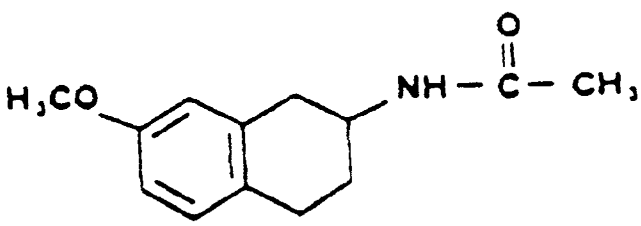

(XXIX)

which is subjected to the action of boron tribromide at room temperature, in a halogenated organic solvent, to form 2-acetamido-7-hydroxy-1,2,3,4-tetrahydronaphthalene of formula XXX

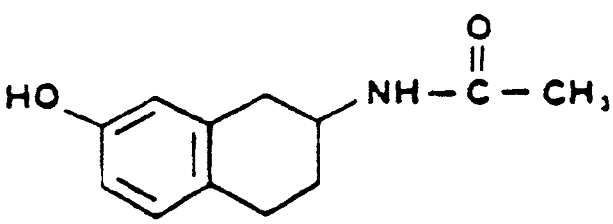

(XXX)

which is then reacted with chloroacetonitrile, in the presence of boron trichloride and aluminium chloride, to form 2-acetamido-6-[(2-chloro-1-oxo)-ethyl]-7-hydroxy-1,2,3,4-tetrahydronaphthalene of formula XXXI

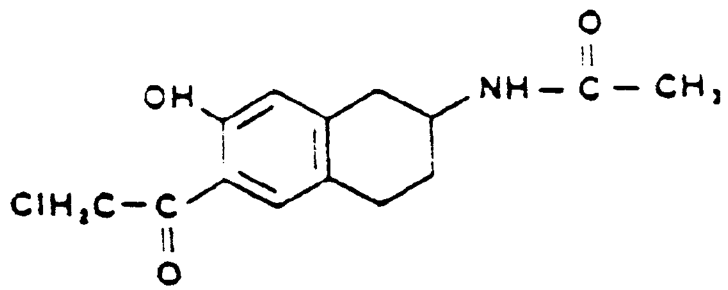

(XXXI)

which is subjected to the action of triethylamine with the application of heat, in a halogenated organic solvent, to form 7-acetamido-5,6,7,8-tetrahydronaphtho[2,3-b]2,3-dihydrofuran-3-one of formula XXXII

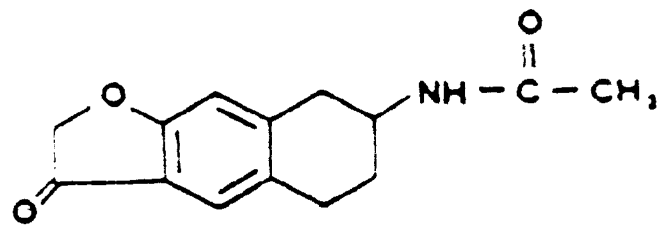

(XXXII)

which is then
- either
subjected to the action of a strong mineral base to form the compound of formula If

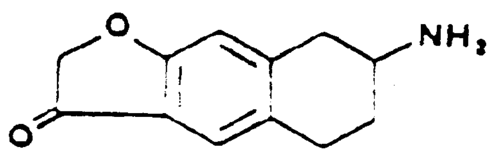

(If)

which may then be alkylated, in accordance with the methods described above for the alkylation of compound Ia, to form the compounds of the general formula $If_1$

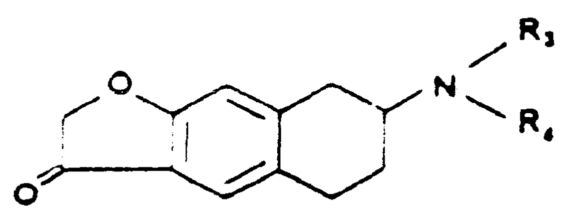

($If_1$)

in which $R_3$ and $R_4$ are as defined for the general formula 1, except that $R_3$ and $R_4$ never simultaneously represent a hydrogen atom,
- or
reduced in an ethanolic solvent at room temperature, with sodium borohydride, to form 7-acetamido-3-hydroxy-5,6,7,8-tetrahydronaphtho[2,3-b]2,3-dihydrofuran of formula XXXIII

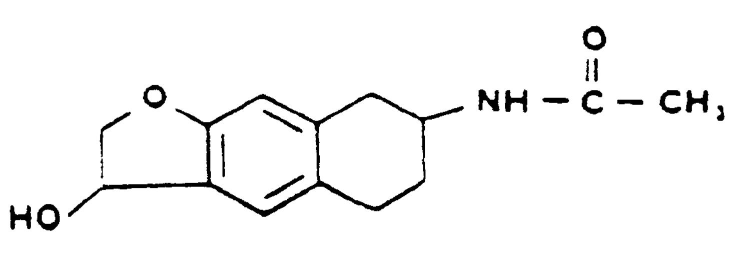

(XXXIII)

which is then subjected

– either

to the action of a strong base to form the compound of formula Ig

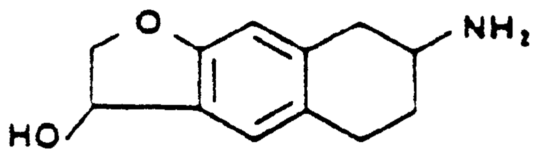

(Ig)

which may then be alkylated, in accordance with the processes described above for compound Ia, to form the compounds of the general formula $Ig_1$

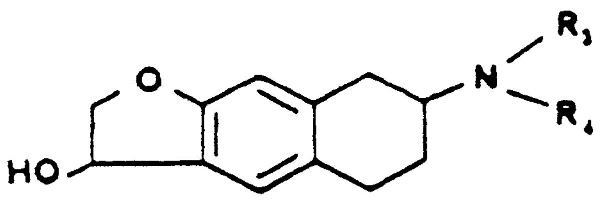

($Ig_1$)

in which $R_3$ and $R_4$ are as defined for the general formula I, except that $R_3$ and $R_4$ never simultaneously represent a hydrogen atom,

– or

to the action of a strong mineral acid, at room temperature, to form 7-acetamido-5,6,7,8-tetrahydronaphtho[2,3-b]furan of formula XXXIV

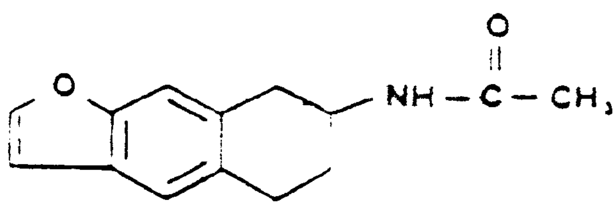

(XXXIV)

which is then subjected to the action of a strong mineral base, in alcoholic medium, to form the compound of formula Ih

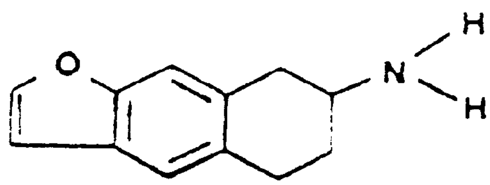

(Ih)

which may then be subjected to alkylation, in accordance with the methods described above for the primary amines, to form the compounds of formula $Ih_1$

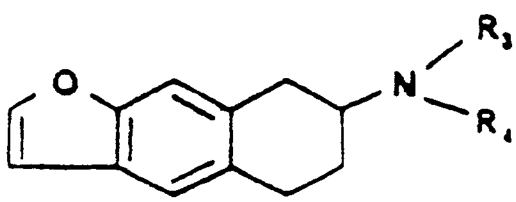

($Ih_1$)

in which $R_3$ and $R_4$ are as defined for the general formula I, except that they never simultaneously represent

a hydrogen atom,

and then, if desired, the compounds of formulae Ia to Ih and $Ia_1$ to $Ih_1$, which together form the totality of the compounds of formula I, are converted into salts with a pharmaceutically acceptable mineral or organic acid, or

are first separated into their optical isomers and then converted into salts.

8. Pharmaceutical composition containing as active ingredient a compound according to any one of claims 1 to 6, in association or admixture with a pharmaceutically acceptable inert non-toxic vehicle or excipient.

9. Pharmaceutical composition according to claim 8, containing the active ingredient in an amount of from 0.5 to 100 mg.

10. Pharmaceutical composition according to claims 8 and 9, containing as active ingredient at least one compound according to claims 1 to 6 for use in the treatment of disorders requiring antidepressive, antiagressive or dopaminergic modulator medication.

11. 5,6,7,8-Tetrahydronaphtho[2,3-b]2,3-dihydrofuran-5-one for use as an intermediate in the preparation of compounds of the general formula I according to claim 1.

## Patentansprüche

1. Verbindungen der allgemeinen Formel I

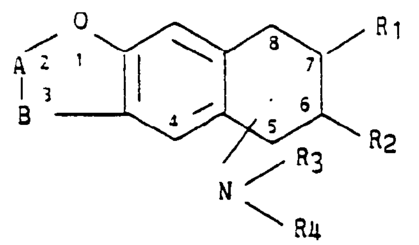

(I)

in der

– $R_1$ ein Wasserstoffatom oder dann, wenn die Aminogruppe in der 7-Stellung steht, eine geradkettige oder verzweigte Alkylgruppe mit 1 bis 4 Kohlenstoffatomen,

– $R_2$ ein Wasserstoffatom oder dann, wenn die Aminogruppe in der 6-Stellung steht, eine geradkettige oder verzweigte Alkylgruppe mit 1 bis 4 Kohlenstoffatomen,

– $R_3$ und $R_4$, die gleichartig oder verschieden sein können, jeweils Wasserstoffatome, Benzylgruppen, Cyclohexylmethylgruppen, geradkettige oder verzweigte Alkylgruppen mit 1 bis 5 Kohlenstoffatomen, geradkettige oder verzweigte Alkylgruppen mit 1 bis 10 Kohlenstoffatomen (die gegebenenfalls durch eine Hydroxylgruppe, eine Carboxygruppe oder eine Alkoxygruppe mit 1 bis 5 Kohlenstoffatomen, eine Alkoxycarbonylgruppe mit 2 bis 6 Kohlenstoffatomen, eine Alkylphenylgruppe mit 7 bis 16 Kohlenstoffatomen oder eine 2-Alkylthienylgruppe mit 12 bis 14 Kohlenstoffatomen substituiert sind), eine halogenierte Alkylgruppe mit 1 bis 5 Kohlenstoffatomen oder gemeinsam mit dem Stickstoffatom, an das sie gebunden sind, eine 2-Oxo-1-pyrrolidinyl-gruppe,

– A-B zusammen mit dem Sauerstoffatom, an das sie gebunden sind, eine Gruppe der Formel

$$-CH_2-CH_2-O-$$

oder dann, wenn die Aminogruppe in der 7-Stellung steht und $R_1$ ein Wasserstoffatom darstellt, eine Gruppe der Formel

$$-CH=CH-O-,$$

eine Gruppe der Formel

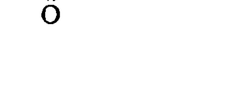

oder eine Gruppe der Formel

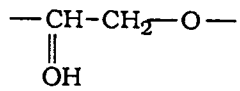

mit der Maßgabe bedeuten, daß, wenn A - B mit dem Sauerstoffatom, an das sie gebunden sind, eine Gruppe der Formel - $CH_2$ - $CH_2$ - O - darstellen, die Aminogruppe in der 7-Stellung steht und $R_1$ und $R_2$ jeweils ein Wasserstoffatom bedeuten und $R_3$ und $R_4$ nicht gemeinsam jeweils Wasserstoffatome bedeuten, in Form des Racemats oder der optischen Isomeren,
und deren Additionssalze mit einer pharmazeutisch annehmbaren anorganischen oder organischen Säure.

2. Verbindungen der allgemeinen Formel I nach Anspruch 1, worin die Aminogruppe in der 7-Stellung steht, und deren optische Isomeren und deren Additionssalze mit einer pharmazeutisch annehmbaren anorganischen oder organischen Säure.

3. Verbindungen der allgemeinen Formel I nach Anspruch 1, worin die Aminogruppe in der 6-Stellung steht, und deren optische Isomeren und deren Additionssalze mit einer pharmazeutisch annehmbaren anorganischen oder organischen Säure.

4. Verbindungen der allgemeinen Formel I nach Anspruch 1, worin $R_3$ und $R_4$ gleichartig sind und jeweils Wasserstoffatome bedeuten, und deren Additionssalze mit einer pharmazeutisch annehmbaren anorganischen oder organischen Säure.

5. 6-Amino-5,6,7,8-tetrahydro-naphtho[2,3b]-2,3-dihydrofuran in Form des Racemats oder der optischen Isomeren und dessen Additionssalze mit einer pharmazeutisch annehmbaren anorganischen oder organischen Säure.

6. 7-Amino-5,6,7,8-tetrahydro-naphtho[2,3b]furan in Form des Racemats oder der optischen Isomeren und dessen Additionssalze mit einer pharmazeutisch annehmbaren anorganischen oder organischen Säure.

7. Verfahren zur Herstellung der Verbindungen der allgemeinen Formel I nach Anspruch 1, **dadurch gekennzeichnet**, daß man
entweder
2,3-Dihydro-benzofuran der Formel II

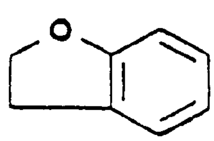

(II)

mit Bernsteinsäureanhydrid in Gegenwart von Aluminiumchlorid in einem chlorierten organischen Lösungsmittel bei einer Temperatur unterhalb 5°C kondensiert zur Bildung der 4-(2,3-Dihydro-benzofuran-5-yl)-4-oxo-butansäure der Formel III

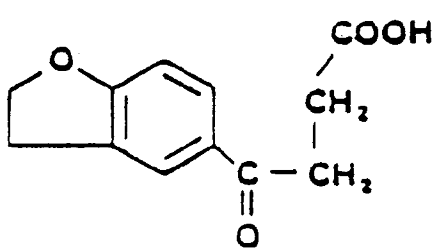

(III)

welche man in saurem Medium in der Wärme und in Gegenwart von Zink und Quecksilber(II)-chlorid reduziert zur Bildung der 4-(2,3 -Dihydro-benzofuran-5-yl) -butansäure der Formel IV

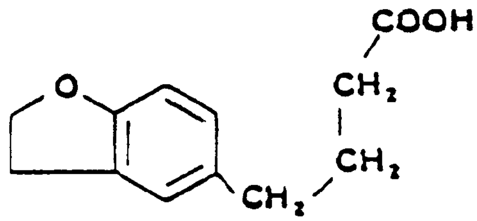

(IV)

welche man anschließend mit Polyphosphorsäure in einem apolaren organischen Lösungsmittel bei einer Temperatur zwischen 80°C und 100°C umsetzt zur Bildung von 5,6,7,8-Tetrahydro-naphtho[2,3b]-2,3-dihydro-furan-8-on der Formel V

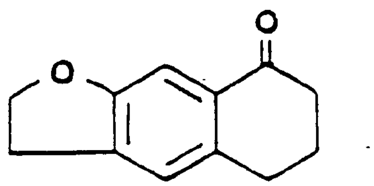

(V)

welches man anschließend

– <u>entweder</u> der Einwirkung eines Hydroxylaminsalzes in der Wärme in einem niedrigmolekularen Alkohol unterwirft zur Bildung von 5,6,7,8-Tetrahydro-naphtho[2,3b]2,3-dihydro-furan-8-on-oxim der Formel VI

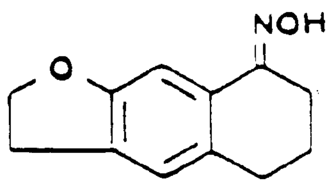

(VI)

welches man anschließend:

– <u>entweder</u>

mit p-Toluolsulfochlorid in einem basischen organischen Medium bei einer Temperatur unterhalb 5°C kondensiert zur Bildung des p-Toluolsulfonats des 5,6,7,8-Tetrahydro-naphtho[2,3b]2,3-dihydro-furan-8-on-oxims der Formel VII

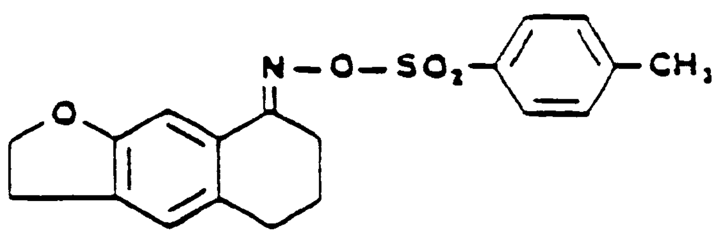

(VII)

welches man in einem apolaren wasserfreien organischen Lösungsmittel bei einer Temperatur von etwa 0°C mit Natriumethanolat umsetzt zur Bildung von 7-Acetylamino-5,6,7,8-tetrahydro-naphtho[2,3b]-2,3-dihydro-furan-8-on der Formel VIII

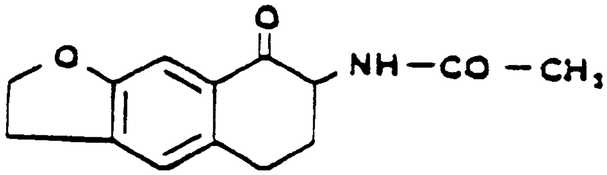

(VIII)

welches man bei Raumtemperatur in Gegenwart einer Säure und von 5 % Palladium-auf-Kohlenstoff hydriert zur Bildung von 7-Acetylamino-5,6,7,8-tetrahydro-naphtho[2,3b]-2,3-dihydro-furan der Formel IX

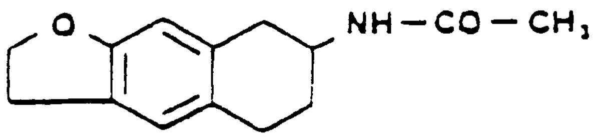

(IX)

welches man anschließend durch saure Hydrolyse in der Wärme in 7-Amino-5,6,7,8-tetrahydro-naphtho[2,3b]-2,3-dihydro-furan der Formel Ia

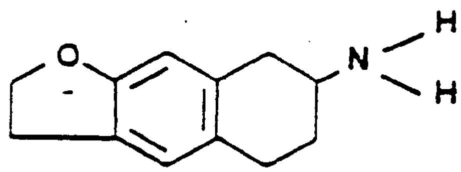

(Ia)

umwandelt,
welches man anschließend alkylieren kann zur Bildung der entsprechenden sekundären oder tertiären Amine,

· oder mit einer Verbindung der allgemeinen Formel X

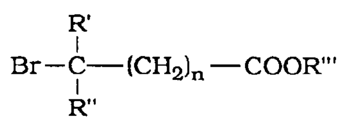

(X)

in der R′ und R″, die gleichartig oder verschieden sein können, jeweils Wasserstoffatome oder niedrigmolekulare Alkylgruppen mit 1 bis 4 Kohlenstoffatomen, R‴ eine niedrigmolekulare Alkylgruppe mit 1 bis 5 Kohlenstoffatomen und n eine ganze Zahl mit einem Wert von 0 bis 9 bedeuten, zu einer Verbindung der allgemeinen Formel $Ia_1$ kondensieren kann

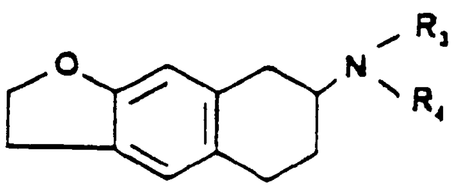

($Ia_1$)

in der $R_4$ ein Wasserstoffatom und $R_3$ eine geradkettige oder verzweigte Alkylgruppe mit 1 bis 10 Kohlenstoffatomen, die durch eine Alkoxycarbonylgruppe mit 2 bis 6 Kohlenstoffatomen substituiert ist, bedeuten,
welche man anschließend der Einwirkung einer starken anorganischen Base unterwerfen kann zur Bildung einer Verbindung der allgemeinen Formel $Ia_1$, in der $R_4$ ein Wasserstoffatom und $R_3$ eine geradkettige oder verzweigte Alkylgruppe mit 1 bis 10 Kohlenstoffatomen, die durch eine Carboxygruppe substituiert ist, bedeuten,
welche man anschließend der Einwirkung eines Doppelmetallhydrids unterwerfen kann zur Bildung einer Verbindung der allgemeinen Formel $Ia_1$ in der $R_4$ ein Wasserstoffatom und $R_3$ eine geradkettige oder verzweigte, durch eine Hydroxygruppe substituierte Alkylgruppe mit 1 bis 10 Kohlenstoffatomen bedeuten,

· oder mit einem Säurechlorid der allgemeinen Formel XI

$$W(CH_2)_nCOCl \quad (XI)$$

in der n eine ganze Zahl mit einem Wert von 0 bis 9 und W eine Phenylgruppe oder eine Thien-2-ylgruppe oder die Gruppe der Formel $W(CH_2)_n$ gemeinsam eine halogenierte Alkylgruppe mit 1 bis 5 Kohlenstoffatomen bedeuten, umsetzen und anschließend die in dieser Weise erhaltene Verbindung mit

einem Doppelmetallhydrid reduzieren kann zur Bildung einer Verbindung der allgemeinen Formel Ia 1, in der $R_3$ eine halogenierte Alkylgruppe mit 1 bis 5 Kohlenstoffatomen oder eine Alkylgruppe mit 1 bis 10 Kohlenstoffatomen, die durch eine Phenylgruppe oder eine Thien-2-yl-gruppe substituiert ist, und $R_4$ ein Wasserstoffatom bedeuten,

· oder mit einer entsprechenden Menge Formaldehyd und Ameisensäure zu Verbindungen der allgemeinen Formel $Ia_1$ umsetzen kann, in der $R_3$ und $R_4$ gleichartig sind und jeweils Methylgruppen bedeuten,

· oder mit 4-Chlor-butyrylchlorid umsetzen kann, um nach der Kondensation mit einem Metallhydrid eine Verbindung der allgemeinen Formel $Ia_1$ zu bilden, in der $R_3$ und $R_4$ gemeinsam mit dem Stickstoffatom, an das sie gebunden sind, eine 2-Oxo-pyrrolidin-1-yl-gruppe bedeuten,

· oder mit einem Alkyliodid- oder -chlorid der allgemeinen Formel XIIa oder XIIb

IR $(XII_a)$

ClR $(XII_b)$

in der R eine geradkettige oder verzweigte Alkylgruppe mit 1 bis 10 Kohlenstoffatomen (die gegebenenfalls durch eine Hydroxylgruppe oder eine Alkoxygruppe mit 1 bis 5 Kohlenstoffatomen substituiert sein kann) oder eine Cyclohexylmethylgruppe bedeutet, in der Wärme in einem organischen Lösungsmittel und in Gegenwart einer anorganischen Base umsetzen kann zur Bildung von Verbindungen der allgemeinen Formel $Ia_1$, in der $R_3$ und $R_4$, die gleichartig sind und die gleichen Bedeutungen besitzen wir R,

· oder mit Benzaldehyd in der Wärme und in Gegenwart eines Alkohols mit niedrigem Molekulargewicht zu dem entsprechenden Benzylimin umsetzen kann, welches anschließend durch katalytische Hydrierung und durch Behandlung mit Ameisensäure oder Formaldehyd zu Verbindungen der allgemeinen Formel $Ia_1$ umgesetzt werden kann, in der $R_3$ eine Methylgruppe und $R_4$ eine Cyclohexylmethylgruppe bedeuten,

· oder zunächst mit Benzyaldehyd in Gegenwart eines inerten apolaren aromatischen Lösungsmittels und anschließend nach Beseitigung des verwendeten Lösungsmittels mit Natriumborhydrid in Gegenwart eines polaren aliphatischen Alkohols mit niedrigem Molekulargewicht zu einer Verbindung der allgemeinen Formel $Ia_1$ umsetzen kann, in der $R_3$ ein Wasserstoffatom und $R_4$ eine Benzylgruppe bedeuten,

welche man anschließend

· entweder mit Formaldehyd und Ameisensäure zu Verbindungen der allgemeinen Formel Ia umsetzen kann, in der $R_3$ eine Methylgruppe und $R_4$ eine Benzylgruppe bedeuten,

· oder mit einem Alkyliodid der allgemeinen Formel XII zu Verbindungen der allgemeinen Formel $Ia_1$ umsetzen kann, in der $R_3$ die gleiche Bedeutung besitzt wie R und $R_4$ eine Benzylgruppe darstellt,

welche man anschließend

einer katalytischen Hydrierung zur Bildung der Verbindungen der allgemeinen Formel $Ia_1$, in der $R_3$ die oben angegebene Bedeutung besitzt und $R_4$ ein Wasserstoffatom darstellen, unterwerfen kann,

welche man anschließend

mit einem Alkyliodid der allgemeinen Formel $XII_a$ umsetzen kann zur Bildung der Verbindungen der allgemeinen Formel $Ia_1$, in der $R_3$ und $R_4$ gleichartig oder verschieden sind, und jeweils Alkylgruppen mit 1 bis 10 Kohlenstoffatomen (die gegebenenfalls mit einer Hydroxygruppe oder einer Alkoxygruppe mit 1 bis 5 Kohlenstoffatomen substituiert sein können) oder Cyclohexylmethylgruppen bedeuten,

– oder

das man in Gegenwart von 5 % Palladium-auf-Kohlenstoff bei Raumtemperatur einer katalytischen Hydrierung unterwirft zur Bildung von 8-Amino-5,6,7,8-tetrahydro-naphtho[2,3b]-2,3-dihydro-furan der Formel $I_b$

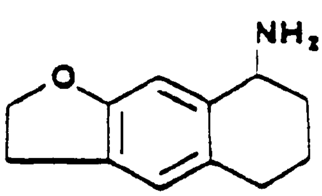

$(I_b)$

welches man anschließend mit den oben für die Verbindungen der allgemeinen Formel Ia beschriebenen Methoden alkylieren kann zur Bildung der entsprechenden sekundären oder tertiären Amine der allgemeinen Formel $Ib_1$

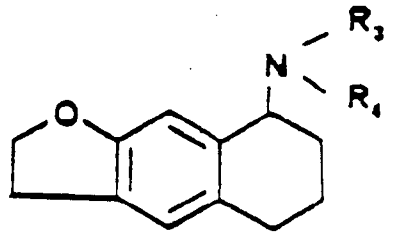

(Ib$_1$)

in der $R_3$ und $R_4$ die oben bezüglich der allgemeinen Formel I angegebenen Bedeutungen besitzen, mit der Maßgabe, daß sie nicht gleichzeitig Wasserstoffatome bedeuten,

– oder

die man der Einwirkung eines Überschusses von perbromiertem Pyridiniumbromid in einem polaren chlorierten organischen Lösungsmittel bei einer Temperatur in der Nähe von 0°C unterzieht zur Bildung von 7, 7-Dibrom-5,6,7,8-tetrahydro-naphtho[2,3b]-2,3-dihydro-furan-8-on der Formel XIII

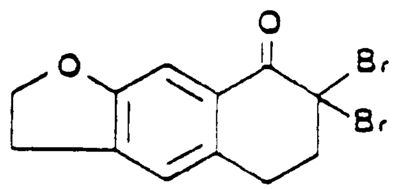

(XIII)

welches man anschließend mit einem Alkylmagnesiumiodid mit geradkettiger oder verzweigter Alkylgruppe mit 1 bis 4 Kohlenstoffatomen in Gegenwart von Kupferbromid in einem wasserfreien organischen Lösungsmittel bei einer Temperatur zwischen -20°C und -40°C kondensiert zur Bildung der Verbindungen der allgemeinen Formel XIV

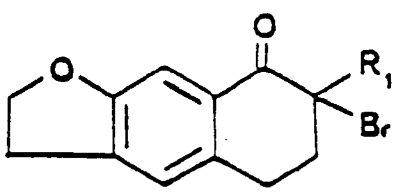

(XIV)

in der $R_1$ die bezüglich der allgemeinen Formel I angegebenen Bedeutungen besitz,
welche man Natriumazid in einem organischen sauren Medium und bei einer Temperatur von etwa 0°C umsetzt zur Bildung eines 7-Azido-7-alkyl-5,6,7,8-tetrahydro-naphtho[2,3b]-2,3-dihydro-furan-8-on-derivats der allgemeinen Formel XV

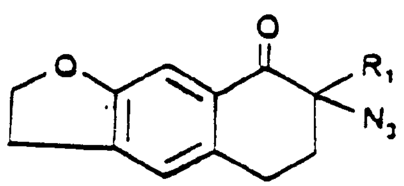

(XV)

in der $R_1$ die oben angegebenen Bedeutungen besitzt, umsetzt, welches man anschließend mit Natriumborhydrid in einem wasserfreien alkoholischen Lösungsmittel reduziert zur Bildung eines 7-Azido-7-alkyl-8-hydroxy-5,6,7,8-tetrahydro-naphtho[2,3b]-2,3-dihydro-furan-derivats der allgemeinen Formel XVI

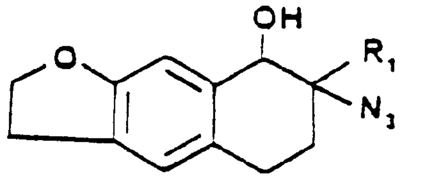

(XVI)

in der $R_1$ die oben angegebenen Bedeutungen besitzt, welches man anschließend in Gegenwart von Trifluoressigsäure unter inerter Atmosphäre mit Triethylsilan umsetzt zur Bildung eines 7-Azido-7-alkyl-5,6,7,8-tetrahydro-naphtho[2,3b]-2,3-dihydro-furan-derivats der allgemeinen Formel XVII

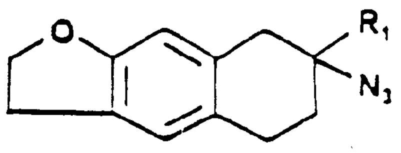

(XVII)

in der $R_1$ die oben angegebenen Bedeutungen besitzt, welches man mit Hydrazin in Gegenwart von Raney-Nickel in wasserfreiem Medium zu den Verbindungen der allgemeinen Formel Ic

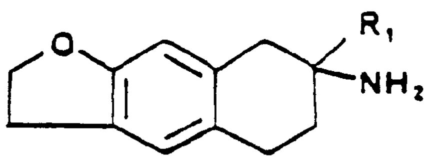

(Ic)

in der $R_1$ die oben angegebenen Bedeutungen besitzt, umsetzt, welche man anschließend nach den oben bezüglich der Alkylierung der Verbindung der Formel Ia beschriebenen Methoden alkylieren kann zur Bildung der entsprechenden sekundären oder tertiären Amine der allgemeinen Formel $Ic_1$

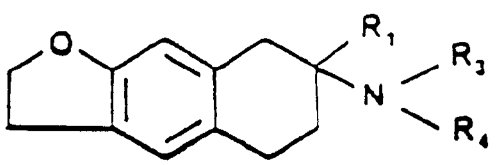

($Ic_1$)

in der $R_1$ die oben angegebenen Bedeutungen besitzt, und $R_3$ und $R_4$ die bezüglich der allgemeinen Formel I angegebenen Bedeutungen besitzen, mit der Maßgabe, daß sie nicht gemeinsam Wasserstoffatome darstellen,

– <u>oder</u>

die man in saurem Medium und in der Wärme mit metallischem Zinkamalgam und Quecksilber(II)-chlorid reduziert zur Bildung von 5,6,7,8-Tetrahydronaphtho[2,3b]-2,3-dihydro-furan der Formel XVIII

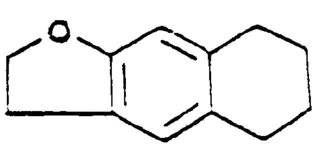

(XVIII)

welches man mit Chromsäureanhydrid in saurem organischem Medium bei einer Temperatur unterhalb 5°C oxidiert zur Bildung von 5,6,7,8-Tetrahydronaphtho[2,3b]-2,3-dihydro-furan-5-on der Formel XIX

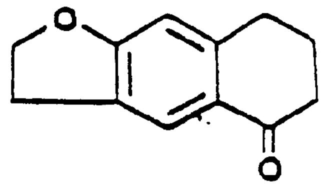

(XIX)

welches man anschließend

– entweder

in einem Alkohol mit niedrigem Molekulargewicht bei einer Temperatur von etwa 100°C mit einem Hydroxylaminsalz kondensiert zur Bildung von 5,6,7,8-Tetrahydro-naphtho[2,3b]-2,3-dihydro-furan-5-on-oxim der allgemeinen Formel XX

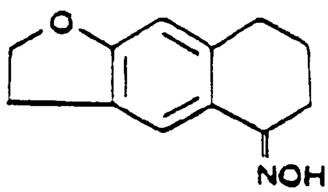

(XX)

welches man anschließend bei Raumtemperatur in Gegenwart von 5 % Palladium-auf-Kohlenstoff katalytisch hydriert zur Bildung von 5-Amino-5,6,7,8-tetrahydro-naphtho[2,3b]-2,3-dihydro-furan der Formel Id

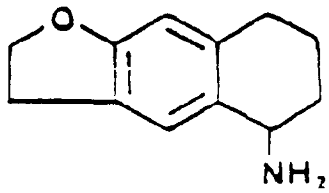

(Id)

welches man anschließend nach den oben bezüglich der Alkylierung der Verbindungen der Formel Ia beschriebenen Methoden alkylieren kann zur Bildung der entsprechenden sekundären oder tertiären Amine der allgemeinen Formel $Id_1$

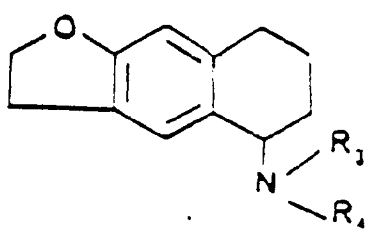

($Id_1$)

in der $R_3$ und $R_4$ die oben bezüglich der Formel I angegebenen Bedeutungen mit der Maßgabe besitzen, daß sie nicht gleichzeitig Wasserstoffatome darstellen,

– oder

die man mit einer geeigneten Menge perbromierten Pyridiniumbromids in einem polaren organischen Lösungsmittel umsetzt zur Bildung einer Verbindung der allgemeinen Formel XXI

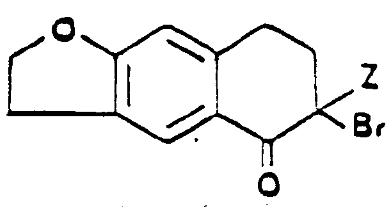

(XXI)

in der Z ein Wasserstoffatom darstellt, wenn die verwendete Menge des perbromierten Pyridiniumbromids

etwa äquimolar ist, oder ein Bromatom bedeutet, wenn die Bromierungsreaktion mit einer mindestens doppelten Menge des Bromierungsreagens durchgeführt worden ist,

welche man anschließend

- dann, wenn sie in der 6-Stellung 2 Bromatome aufweist, zunächst mit einem Alkylmagnesiumiodid mit einer geradkettigen oder verzweigten Alkylgruppe mit 1 bis 4 Kohlenstoffatomen in Gegenwart von Kupferbromid kondensiert und anschließend mit Natriumazid umsetzt, oder
- direkt mit Natriumazid umsetzt, wenn Z ein Wasserstoffatom bedeutet, zur Bildung eines 6-Azido-5,6,7,8-tetrahydro-naphtho[2,3b]-2,3-dihydro-furan-5-on-derivats der allgemeinen Formel XXII

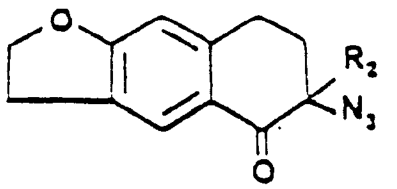

(XXII)

in der $R_2$ ein Wasserstoffatom bedeutet, welches man anschließend mit Natriumborhydrid in Gegenwart eines wasserfreien alkoholischen Lösungsmittels reduziert zur Bildung eines 6-Azido-5-hydroxy-5,6,7,8-tetrahydro-naphtho[2,3b]-2,3-dihydro-furan-derivats der allgemeinen Formel XXIII

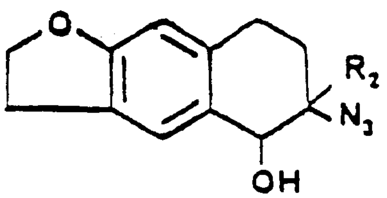

(XXIII)

in der $R_2$ die oben angegebenen Bedeutungen besitzt,
welches man anschließend in Trilluoressigsäure und unter inerter Atmosphäre mit Triethylsilan umsetzt zur Bildung eines 6-Azido-5,6,7,8-tetrahydro-naphtho[2,3b]-2,3-dihydro-furan-derivats der allgemeinen Formel XXIV

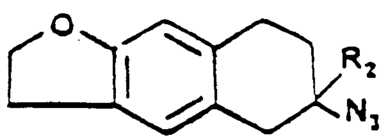

(XXIV)

in der $R_2$ die oben angegebenen Bedeutungen beibehält,
welches man anschließend mit Hydrazin in Gegenwart von Raney-Nickel in wasserfreiem Medium umsetzt zur Bildung eines 6-Amino-5,6,7,8-tetrahydronaphtho[2,3b]-2,3-dihydro-furan-derivats der allgemeinen Formel Ie

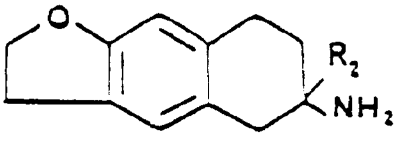

(Ie)

in der $R_2$ die oben angegebenen Bedeutungen besitzt,
welches man anschließend nach den oben bezüglich der Alkylierung der Verbindung der Formel Ia beschriebenen Methoden alkylieren kann zur Bildung der entsprechenden sekundären oder tertiären Amine der allgemeinen Formel $Ie_1$

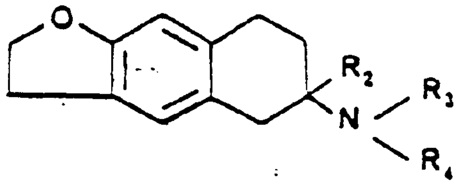

(Ie1)

in der $R_2$, $R_3$ und $R_4$ die bezüglich der allgemeinen Formel I angegebenen Bedeutungen mit der Maßgabe besitzen, daß $R_3$ und $R_4$ nicht gleichzeitig Wasserstoffatome bedeuten,
oder
man 2,7-Dimethoxy-naphthalin der Formel XXV

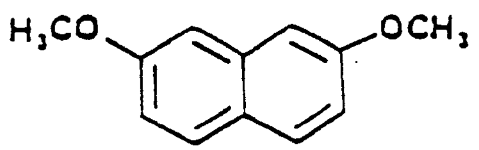

(XXV)

in der Wärme und in Gegenwart eines Wasserfreien Alkohols mit metallischem Natrium umsetzt zur Bildung von 7-Methoxy-1,2,3,4-tetrahydro-naphthalin-2-on der Formel XXVI

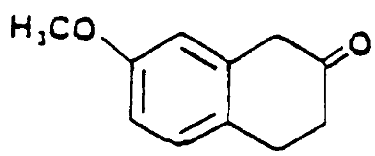

(XXVI)

welches man anschließend in der Wärme in einem alkoholischen Lösungsmittel mit einem Hydroxylaminsalz umsetzt zur Bildung von 7-Methoxy-1,2,3,4-naphthalin-2-on-oxim der Formel XXVIII

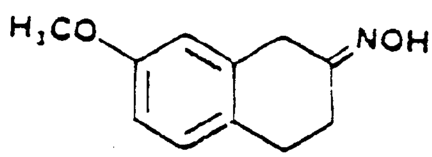

(XXVII)

welches man in Lösung in einem Alkohol in Gegenwart von Raney-Nickel und Ammoniak katalytisch hydriert zur Bildung von 2-Amino-7-methoxy-1,2,3,4-tetrahydro-naphthalin der Formel XXVIII

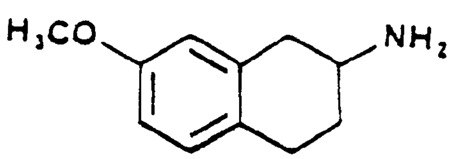

(XXVIII)

welches man in essigsaurem Medium mit Essigsäureanhydrid kondensiert zur Bildung von 2-Acetamido-7-methoxy-1,2,3,4-tetrahydro-naphthalin der Formel XXIX

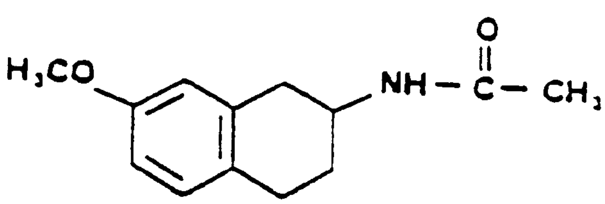

(XXIX)

welches man mit Bortribromid bei Raumtemperatur in einem halogenierten organischen Lösungsmittel umsetzt zur Bildung von 2-Acetamido-7-hydroxy-1,2,3,4-tetrahydro-naphthalin der Formel XXX

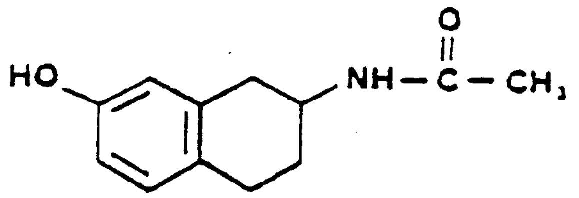

(XXX)

welches man anschließend in Gegenwart von Bortrichlorid und Aluminiumchlorid mit Chloracetonitril umsetzt zur Bildung von 2-Acetamido-6-[(2-chlor-1-oxo)-ethyl]-7-hydroxy-1,2,3,4-naphthalin der Formel XXXI

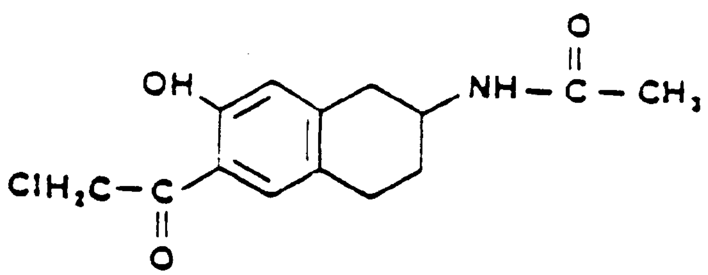

(XXXI)

welches man in der Wärme in einem halogenierten organischen Lösungsmittel der Einwirkung von Triethylamin unterwirft zur Bildung von 7-Acetamido-5,6,7,8-tetrahydro-naphtho[2,3b]-2,3-dihydro-furan-3-on der Formel XXXII

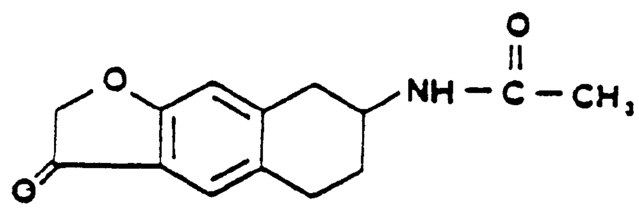

(XXXII)

welches man anschließend
– <u>entweder</u>
der Einwirkung einer anorganischen starken Base unterwirft zur Bildung der Verbindung der Formel If

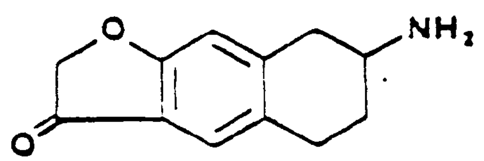

(If)

welche man anschließend nach den oben bezüglich der Alkylierung der Verbindung der Formel Ia beschriebenen Methoden alkylieren kann zur Bildung der Verbindungen der allgemeinen Formel $If_1$

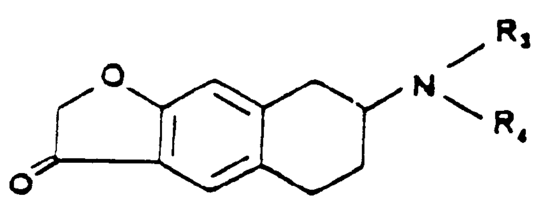

($If_1$)

in der die Definition von $R_3$ und $R_4$ jener für die allgemeine Formel I gegebenen entspricht, mit der Maßgabe, daß $R_3$ und $R_4$ nicht gleichzeitig Wasserstoffatome bedeuten,

– oder
in ethanolischem Lösungsmittel bei Raumtemperatur mit Natriumborhydrid reduziert zur Bildung von 7-Acetamido-3-hydroxy- 5,6,7,8-tetrahydro-naphtho[2,3b]-2,3-dihydro-furan der allgemeinen Formel XXXIII

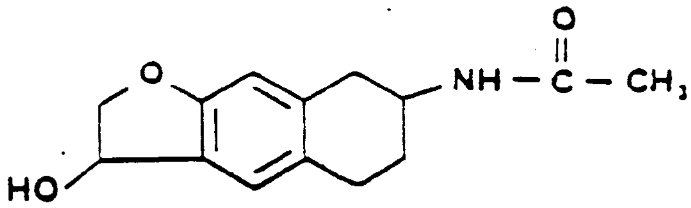

(XXXIII)

welches man anschließend
– entweder
mit einer starken Base umsetzt zur Bildung der Verbindung der Formel Ig

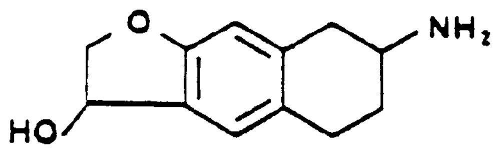

(Ig)

welche man anschließend nach den oben bezüglich der Formel Ia angegebenen Verfahrensweisen alkylieren kann zur Bildung der Verbindungen der allgemeinen Formel $Ig_1$

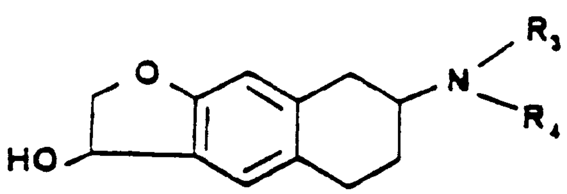

(Ig1)

in der $R_3$ und $R_4$ die bezüglich der allgemeinen Formel I angegebenen Bedeutungen mit der Maßgabe besitzen, daß $R_3$ und $R_4$ nicht gleichzeitig Wasserstoffatome bedeuten,
– oder
mit einer starken anorganischen Säure bei Raumtemperatur umsetzt zur Bildung von 7-Acetamido-5,6,7,8-tetrahydro-naphtho[2,3b]-furan der Formel XXXIV

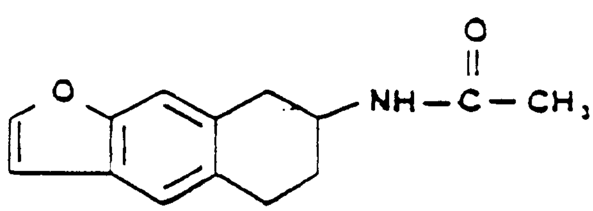

(XXXIV)

welches man anschließend mit einer starken anorganischen Base in alkoholischem Medium umsetzt zur Bildung der Verbindung der Formel Ih

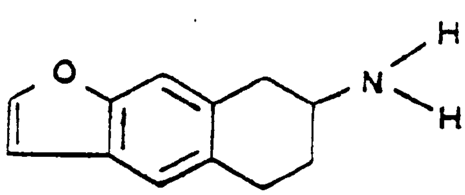

(Ih)

welche man anschließend nach den oben bezüglich der primären Amine beschriebenen Verfahren alkylieren kann zur Bildung der Verbindungen der Formel $Ih_1$

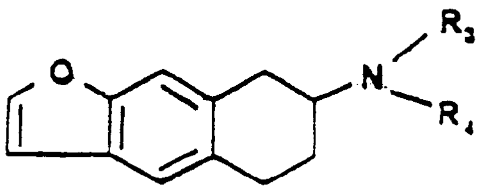

$(Ih_1)$

in der $R_3$ und $R_4$ die bezüglich der allgemeinen Formel I angegebenen Bedeutungen besitzen mit der Maßgabe, daß sie nicht gleichzeitig Wasserstoffatome bedeuten,
und man anschließend gewünschtenfalls die Verbindungen der Formeln Ia bis Ih und $Ia_1$ und $Ih_1$, welche gemeinsam die Verbindungen der allgemeinen Formel I darstellen, mit einer pharmazeutisch annehmbaren anorganischen oder organischen Säure in ein Salz überführt
oder
die man zunächst in ihre optischen Isomeren auftrennt und dann in die Salze umwandelt.

8. Pharmazeutische Zubereitung enthaltend als Wirkstoff eine Verbindung nach einem der Ansprüche 1 bis 6 in Kombination oder in Mischung mit einem inerten, nichttoxischen, pharmazeutisch annehmbaren Trägermaterial und/ oder Bindemittel.

9. Pharmazeutische Zubereitung nach Anspruch 8 enthaltend den Wirkstoff in einer Dosis von 0,5 bis 100 mg.

10. Pharmazeutische Zubereitung nach den Ansprüchen 8 und 9 enthaltend als Wirkstoff mindestens eine Verbindung nach den Ansprüchen 1 bis 6 zur Behandlung von Erkrankungen, welche die Verabreichung von Antidepressiva, Antiaggressiva oder dopaminergen Modulatoren erforderlich machen.

11. 5,6,7,8-Tetrahydro-naphtho[2,3b]-2,3-dihydro-furan-5-on als Zwischenprodukt zur Herstellung der Verbindungen der allgemeinen Formel I nach Anspruch 1.

PLANCHE DE FORMULES N° 1

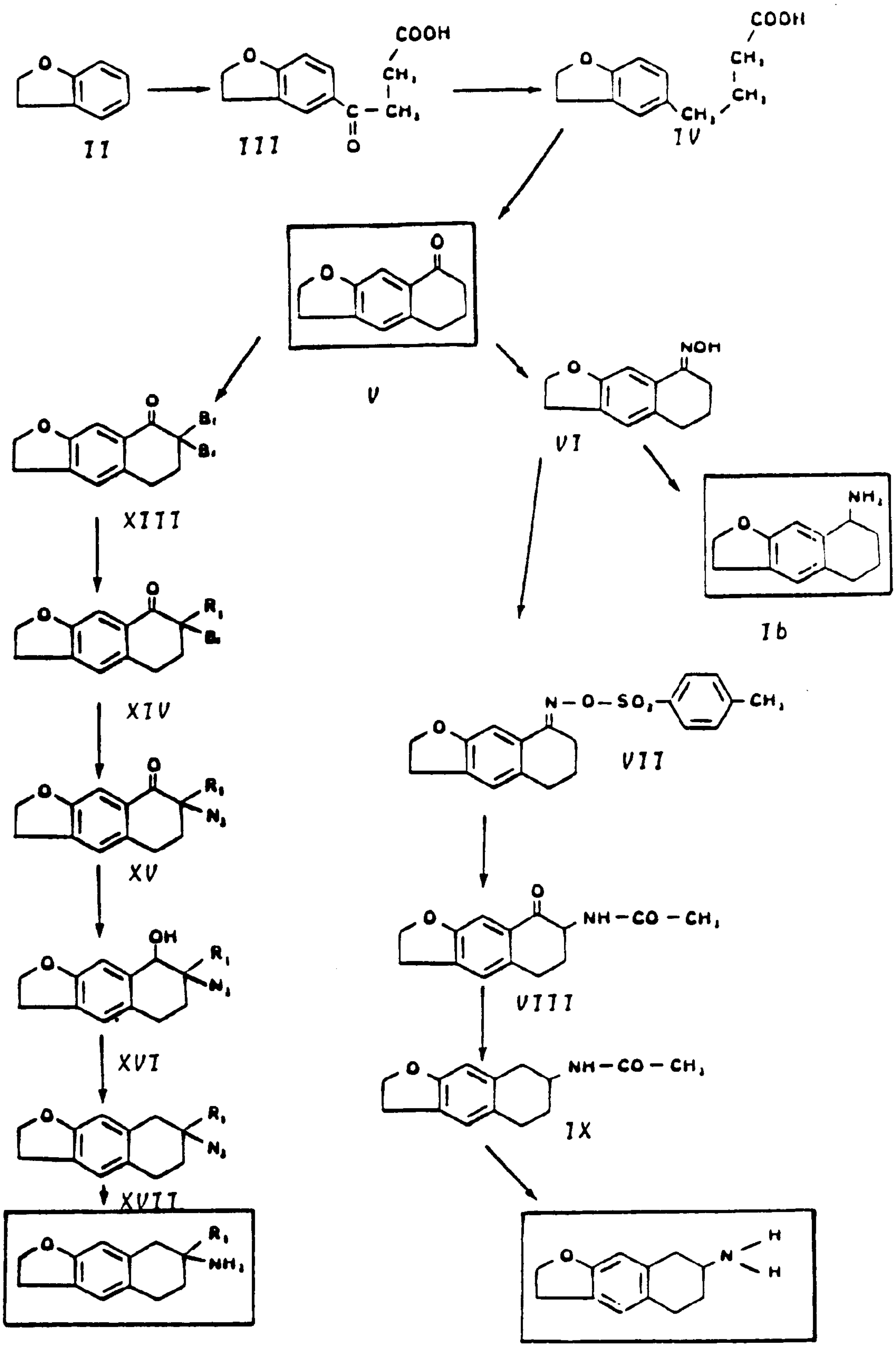

II III IV V VI Ib XIII XIV XV XVI XVII VII VIII IX Ic Ia

PLANCHES DE FORMULES N° 2

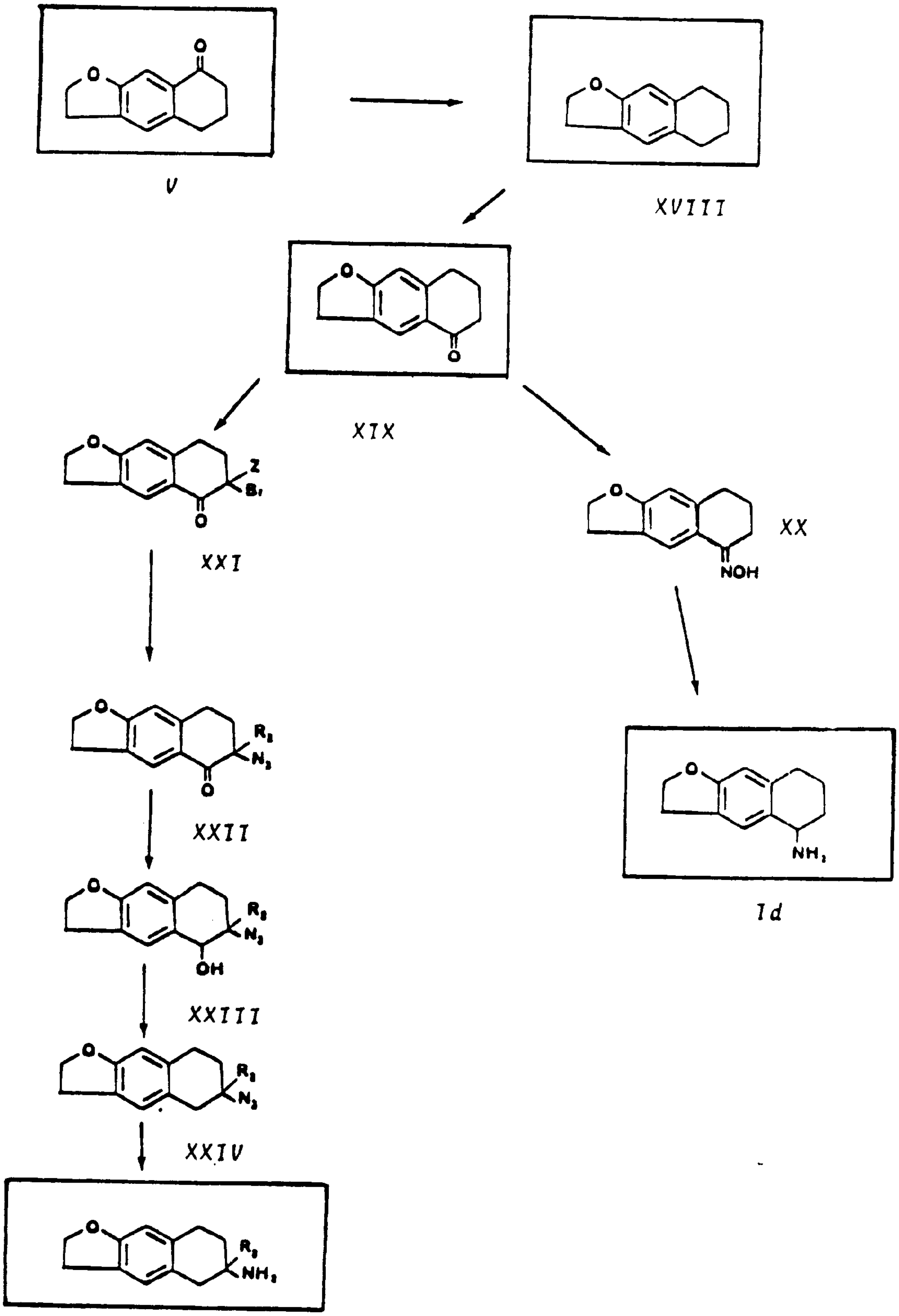

V

XVIII

XIX

XX

XXI

Id

XXII

XXIII

XXIV

Ie

PLANCHE DE FORMULES N° 3

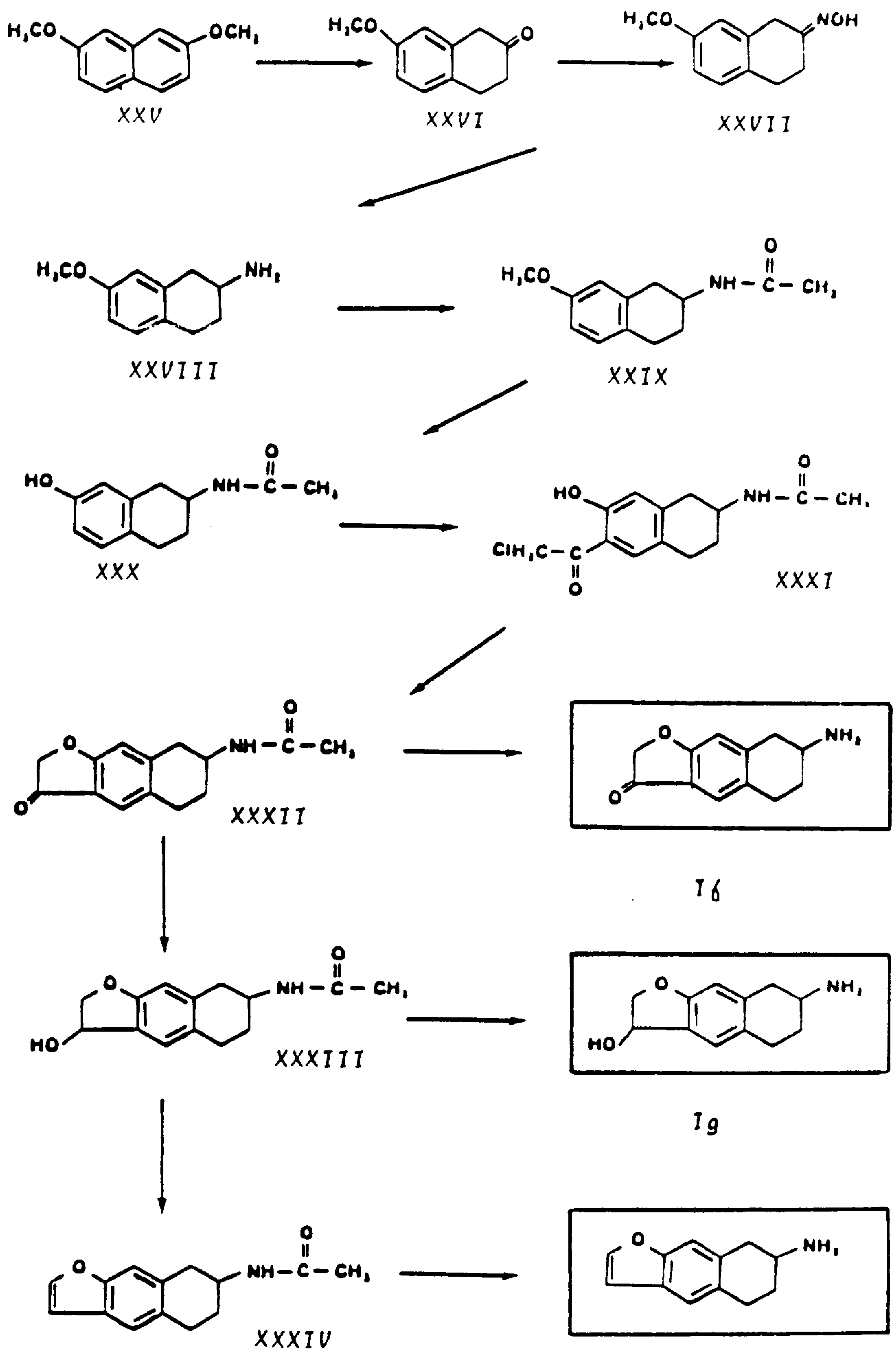

XXV

XXVI

XXVII

XXVIII

XXIX

XXX

XXXI

XXXII

If

XXXIII

Ig

XXXIV

Ih